# EUROPEAN PATENT SPECIFICATION

(11) **EP 4 341 390 B1**
(45) Date of publication and mention of the grant of the patent: **01.07.2026**
(21) Application number: 22808370.5
(22) Date of filing: 12.05.2022
(51) Int. Cl.: C12N 9/12, C12N 9/22, C12N 15/10, C12N 15/11, C12Q 1/6806

(54) **METHODS FOR MAKING LIBRARIES FOR NUCLEIC ACID SEQUENCING**
VERFAHREN ZUR HERSTELLUNG VON BIBLIOTHEKEN ZUR NUKLEINSÄURESEQUENZIERUNG
PROCÉDÉS DE FABRICATION DE BANQUES POUR LE SÉQUENÇAGE D'ACIDES NUCLÉIQUES

(30) Priority: 14.05.2021 US 202163189032 P; 13.09.2021 US 202163243443 P
(43) Date of publication of application: 27.03.2024
(73) Proprietor: Augusta SpinCo Corporation, Franklin Lakes, NJ 07417 (US)
(72) Inventor: GODINEZ, Alvaro, San Jose, CA 95131 (US)
(74) Representative: Carpmaels & Ransford LLP
(86) International application number: PCT/US2022/029057
(87) International publication number: WO 2022/241158

(56) References cited:
- WO-A1-2020/243085
- WO-A1-2021/041922
- WO-A1-2021/058975
- WO-A1-2022/040176
- WO-A1-2022/241135
- WO-A2-2021/252867
- WO-A2-2021/257997
- US-A1- 2020 190 487
- GOSHAYESHI ET AL: "CRISPR/dCas9-mediated transposition with specificity and efficiency of site-directed genomic insertions - Goshayeshi - 2021 - The FASEB Journal - Wiley Online Library", vol. 35, no. 2, 26 January 2021 (2021-01-26), US, XP093222072, ISSN: 0892-6638, Retrieved from the Internet <URL:https://faseb.onlinelibrary.wiley.com/doi/full/10.1096/fj.202001830RR> DOI: 10.1096/fj.202001830RR
- VAN HAASTEREN JOOST ET AL: "Genome-wide integration site detection using Cas9 enriched amplification-free long-range sequencing", vol. 49, no. 3, 22 February 2021 (2021-02-22), GB, pages e16 - e16, XP093033541, ISSN: 0305-1048, Retrieved from the Internet <URL:https://www.ncbi.nlm.nih.gov/pmc/articles/PMC7897500/pdf/gkaa1152.pdf> DOI: 10.1093/nar/gkaa1152

## Description

### REFERENCE TO SEQUENCE LISTING

The present application is being filed along with a Sequence Listing in electronic format. The Sequence Listing is provided as a file entitled 68EB_317326_WO_Sequence_Listing, created May 12, 2022, which is 56.0 kilobytes in size.

### BACKGROUND

### Field

The present disclosure relates generally to the field of molecular biology, for example tagging nucleic acids to generate customized loci-specific sequencing libraries.

### Description of the Related Art

The conventional library preparation methods for sequencing nucleic acids can take hours to make and the process produces libraries that are randomly made. The reason for these libraries being random is because the methods used for fragmenting nucleic acids (including physical, enzymatic, and chemical fragmentation methods) do so in a random manner. As such the output of the DNA sequencing cannot be controlled. Currently, there are two methods that have been used for targeted sequencing. The first one is amplicon sequencing. This method relies on using primers to amplify areas of interest via DNA amplification. This additional amplification step adds further costs, time, and resources to the standard library preparation methods. The second targeted sequencing method is target capture. This method relies on using a probe or a pool of probes so they can hybridize to specific nucleic acid targets. The hybridization of the probes to its target and the isolation of these targets is a time-consuming process that can take days. Furthermore, the probes used in this method are expensive to synthetize. There is a need for compositions, methods, systems, and kits for customized loci-specific library preparation. There is a need for methods, compositions, kits and systems that enable rapid targeted sequencing (and thus fast sequencing-based diagnosis, e.g., less than 2 hours) and theranostics which can provide simultaneous diagnosis and determination of suitable therapeutic approach. WO 2021/041922 A1 describes CRISPR-associated MU transposase systems. WO 2022/241135 A1 describes a multiplexed unbiased nucleic acid amplification method. WO 2021/252867 A2 describes methods of enriching for target nucleic acid molecules. WO 2021/058975 A1 describes methods and systems for preparing a nucleic acid construct for single molecule characterization. Goshayeshi et al. (FASEB Journal, 2021, 35(2):e21539) describes CRISPR/dCas9-mediated transposition with specificity and efficiency of site-directed genomic insertions. WO 2022/040176 A1 describes sequence-specific targeted transposition and selection and sorting of nucleic acids.

### SUMMARY

The present invention is set out in the appended set of claims. The terminologies "embodiment" and "embodiments" are to be construed as embodiment(s) of the invention only in as far as they fall within the scope of the appended claims. Otherwise, they refer to embodiments of the disclosure only, for reference purposes.

Disclosed herein include compositions. In some embodiments, the composition comprises: a plurality of protein complexes. In some embodiments, each of the plurality of protein complexes comprises a transposome and a programmable DNA binding unit capable of specifically binding to a binding site on a target double-stranded DNA (dsDNA). In some embodiments, the transposome comprises a transposase, a first adaptor and a second adaptor. In some embodiments, the binding site for each of the plurality of protein complexes is different from each other.

In some embodiments, at least two of the plurality of protein complexes comprise the same transposome. In some embodiments, all of the plurality of protein complexes comprise the same transposome. In some embodiments, all of the plurality of protein complexes comprise the same transposase. In some embodiments, the first adaptor and the second adaptor in the same transposome are the same. In some embodiments, the first adaptor, the second adaptor, or both, in different transposome are different. In some embodiments, the first adaptor, the second adaptor, or both, is a dsDNA or an RNA/DNA duplex. In some embodiments, the adaptor is about 3-200 base pairs in length. In some embodiments, the first adaptor, the second adaptor, or both, is a sequencing adaptor. In some embodiments, the sequencing adaptor comprises P5 or P7 primer sequences.

In some embodiments, the binding sites of at least two of the plurality of protein complexes are on the same target dsDNA. In some embodiments, the binding sites of at least two of the plurality of protein complexes are about 1-50000 nucleotides apart on the same target dsDNA. In some embodiments, the distance between the binding sites of a pair of the plurality of protein complexes is substantially the same as the distance between the binding sites of another pair of the plurality of protein complexes. In some embodiments, the distance between the binding sites of a pair of the plurality of protein complexes is different as the distance between the binding sites of another pair of the plurality of protein complexes. In some embodiments, the binding sites of at least two of the plurality of protein complexes are on the different strand of a target dsDNA. In some embodiments, at least two of the plurality of protein complexes are capable of specifically binding to different target dsDNA.

In some embodiments, the plurality of protein complex are capable of specifically binding to about 2-5000 target dsDNA. In some embodiments, the transposase is Tn5 transposase, Tn7 transposase, mariner Tc1-like transposase, Himar1C9 transposase, or Sleeping Beauty transposase. In some embodiments, the transposase is a hyperactive transposase. In some embodiments, the programmable DNA binding unit comprises a nuclease-deficient CRISPR associated protein (dCAS protein) and a guide RNA (gRNA) capable of specifically binding to the binding site of the target dsDNA. In some embodiments, the transposome is associated with the programmable DNA binding unit via a linker connecting the transposase and the dCAS protein. In some embodiments, the linker comprises a peptide linker, a chemical linker, or both. In some embodiments, the transposase is present in a fusion protein comprising the dCAS protein. In some embodiments, the dCAS protein is dCAS9, dCAS12, dCAS13, dCAS14, or SpRY dCAS. In some embodiments, the dCAS13 protein is dCAS13a, dCAS13b, dCAS13c, or dCAS13d.

In some embodiments, the programmable DNA binding unit comprises a protein component capable of specifically binding to the binding site on the target dsDNA. In some embodiments, the protein component comprises an endonuclease-deficient zinc finger nuclease (ZFN), a endonuclease-deficient transcription activator-like effector nuclease (TALEN), Argonaute protein, an endonuclease-deficient meganuclease, a recombinase, or a combination thereof. In some embodiments, the transposome is associated with the programmable DNA binding unit via a linker connecting the transposase and the protein component. In some embodiments, the linker comprises a peptide linker, a chemical linker, or both. In some embodiments, the peptide linker comprises multiple glycine, serine, threonine, alanine, lysine, glutamine, or a combination thereof. In some embodiments, the peptide linker comprises a GS linker. In some embodiments, the peptide linker is a XTEN linker. In some embodiments, the protein component is present in a fusion protein comprising the transposase.

Disclosed herein include reaction mixtures. In some embodiments, the reaction mixture comprises: a composition disclosed herein and sample nucleic acids suspected of comprising one or more target dsDNA. The reaction mixture can comprise: a DNA polymerase, dNTPs, or a combination thereof. In some embodiments, the adaptor is covalently attached to the target dsDNA or a fragment thereof. The reaction mixture can comprise: a plurality of dsDNA fragments each comprising the first adaptor and the second adaptor of one of the plurality of protein complexes at each terminus respectively. In some embodiments, the sample nucleic acids comprises eukaryotic DNA, bacterial DNA, viral DNA, fungal DNA, protozoa DNA, or a combination thereof. In some embodiments, the target dsDNA is genomic DNA, mitochondrial DNA, plasmid DNA, or a combination thereof. In some embodiments, the sample nucleic acids are from a biological sample, a clinical sample, an environmental sample, or a combination thereof. In some embodiments, the biological sample comprises stool, sputum, peripheral blood, plasma, serum, lymph nodes, respiratory tissue, exudates, bodily fluid, or a combination thereof.

Disclosed herein include methods for tagging nucleic acids. In some embodiments, the method comprises: contacting the composition disclosed herein with a sample suspected of comprising a plurality of target dsDNA to form a reaction mixture; and incubating the reaction mixture to generate a plurality of dsDNA fragments each comprising the first adaptor and the second adaptor of one of the plurality of protein complexes at each terminus respectively.

Disclosed herein include methods for generating a sequencing library. In some embodiments, the method comprises: contacting the composition disclosed herein with a sample suspected of comprising a plurality of target dsDNA to form a reaction mixture. The method can comprise: incubating the reaction mixture to generate a plurality of dsDNA fragments each comprising the first adaptor and the second adaptor of one of the plurality of protein complexes at each terminus respectively. The method can comprise: amplifying the plurality of dsDNA fragments with primers capable of binding to the adaptors at the termini of the dsDNA fragments to generate a sequencing library.

In some embodiments, each of the primers is about 5-80 nucleotides in length. In some embodiments, amplifying the plurality of dsDNA fragments with the primer is carried out using polymerase chain reaction (PCR). In some embodiments, the PCR is loop-mediated isothermal Amplification (LAMP), helicase-dependent Amplification (HDA), recombinase polymerase amplification (RPA), strand displacement amplification (SDA), nucleic acid sequence-based amplification (NASBA), transcription mediated amplification (TMA), nicking enzyme amplification reaction (NEAR), rolling circle amplification (RCA), multiple displacement amplification (MDA), Ramification (RAM), circular helicase dependent amplification (cHDA), single primer isothermal amplification (SPIA), signal mediated amplification of RNA technology (SMART), self-sustained sequence replication (3SR), genome exponential amplification reaction (GEAR), or isothermal multiple displacement amplification (IMDA). In some embodiments, the PCR is real-time PCR or quantitative real-time PCR (QRT-PCR). In some embodiments, the sample comprises eukaryotic DNA, bacterial DNA, viral DNA, fungal DNA, protozoa DNA, or a combination thereof.

In some embodiments, the plurality of target dsDNA comprises genomic DNA, mitochondrial DNA, plasmid DNA, or a combination thereof. In some embodiments, the sample is, or is derived from, a biological sample, a clinical sample, an environmental sample, or a combination thereof. In some embodiments, the plurality of target dsDNA comprises DNA from at least 2 different organisms. In some embodiments, the plurality of target dsDNA comprises DNA from at least 2 different genes. The method can comprise: generating the plurality of target dsDNA from a plurality of target RNA with a reverse transcriptase. In some embodiments, the plurality of target dsDNA comprises target dsDNA generated from target RNA with a reverse transcriptase.

In some embodiments, the plurality of target dsDNA comprises a genetic signature of interest. In some embodiments, the genetic signature of interest comprises one or more mutations of interest. In some embodiments, the one or more mutations of interest comprise point mutation, inversion, deletion, insertion, translocation, duplication, copy number variation, or a combination thereof. In some embodiments, the one or more mutations of interest comprise nucleotide substitution, deletion, insertion, or a combination thereof. In some embodiments, the genetic signature of interest is indicative of antibiotic resistance or antibiotic susceptibility of the organism from which the target dsDNA is derived. In some embodiments, the genetic signature of interest is indicative of cancer status of the organism from which the target dsDNA is derived. In some embodiments, the genetic signature of interest is indicative of the status of a genetic disease of the organism from which the target dsDNA is derived. In some embodiments, the genetic disease is single-gene disorder. In some embodiments, the genetic disease is cystic fibrosis, Huntington's disease, sickle cell anemia, hemophilia, Duchenne muscular dystrophy, Thalassemia, Fragile X syndrome, familial hypercholesterolemia, polycystic kidney disease, neurofibromatosis type I, hereditary spherocytosis, Marfan syndrome, Tay-Sachs disease, phenylketonuria, mucopolysaccharidoses, lysosomal acid lipase deficiency, glycogen storage diseases, galactosemia, or hemochromatosis.

In some embodiments, contacting the plurality of target dsDNA with the plurality of protein complex pairs is carried out at about 25°C to about 80°C. In some embodiments, incubating the reaction mixture comprises incubating the reaction mixture at about 37°C to about 55°C. In some embodiments, the plurality of protein complex pairs and the plurality of target dsDNA are present in the reaction mixture at a molecular ratio of about 2:1 to about 2,000:1. In some embodiments, the plurality of protein complex pairs and the plurality of target dsDNA are present in the reaction mixture at a molecular ratio of about 2:1 to about 200:1.

The method can comprise: labeling one or both ends of one or more of the plurality of dsDNA fragments. The method can comprise: labeling the two ends of one or more of the plurality of dsDNA fragments differently. In some embodiments, the labeling comprising labeling with anionic labels, cationic labels, neutral labels, electrochemical labels, protein labels, fluorescent labels, magnetic labels, or a combination thereof. The method can comprise: enriching the labeled dsDNA fragments, capturing the labeled dsDNA fragments, isolating the labeled dsDNA fragments, and/or visualizing the labeled dsDNA fragments.

### BRIEF DESCRIPTION OF THE DRAWINGS

FIG. 1 depicts a non-limiting exemplary conventional library preparation method for next generation sequencing. The ligation-based library preparation illustrated is reproduced from www.idtdna.com/pages/technology/next-generation-sequencing/library-preparation/ligation-based-library-prep.
FIG. 2 depicts a non-limiting exemplary conventional sequencing library preparation by tagmentation process - reproduced from Nextera XT Library Prep: Tips and Troubleshooting (2015) from Illumina.
FIG. 3 depicts a non-limiting exemplary schematic of Customized Loci-specific Library Preparation (CLLP) disclosed herein.
FIG. 4 depicts a non-limiting exemplary embodiment of targeted sequencing using genome editing tool (Cas9 and guide RNA).
FIG. 5A-FIG. 5F depict a non-limiting exemplary embodiment of Customized Loci-specific Library Preparation (CLLP) disclosed herein.
FIG. 6 depicts a non-limiting exemplary embodiment showing ONT Rapid Sequencing Kit, tagmentation based. Reproduced from workflow described for Rapid Sequencing Kit from Nanopore.
FIG. 7A-FIG. 7H depict a non-limiting exemplary embodiment of Genome Editing Tagmentation (GET) for generating sequencing library to use in existing sequencing platform such as that from Oxford Nanopore.
FIG. 8 depicts a non-limiting exemplary schematic of a plasmid construct (3XFlag-Cas9-Fl26-Tn5; SEQ ID NO: 1) for use in generating protein complexes provided herein.
FIG. 9 depicts a non-limiting exemplary schematic of a plasmid construct (3XFlag-Cas9-xTen-Tn5; SEQ ID NO: 2) for use in generating protein complexes provided herein.
FIG. 10 depicts a non-limiting exemplary schematic of a plasmid construct (pET-Tn5-xTen-dCas9; SEQ ID NO: 3) for use in generating protein complexes provided herein.
FIG. 11 depicts the relative binding sites of exemplary sgRNAs for *S. enterica* InvA gene.
FIG. 12 depicts the relative binding sites of exemplary sgRNAs for *S. enterica* FliC gene.
FIG. 13 shows a graph of exemplary bioanalyzer data showing that cuts in the genomic DNA were specific to the expected size, demonstrating that the guide RNAs for Salmonella enterica are functional. Also, *See,* Table 2.
FIG. 14 depicts a graph of tape station analysis showing amplification of Tn5-generated fragments using Adaptor A as a primer. This indicates that the adaptor was added to the 5' and 3' end of the cut molecules.
FIG. 15 depicts a graph of tape station analysis showing amplification of Tn5-generated fragments using Adaptor B as primer. This indicates that the adaptor was added to the 5' and 3' end of the cut molecules.
FIG. 16 depicts an exemplary SDS-PAGE gel analysis of recombinantly expressed and purified dCas9-Fl26-Tn5 fusion protein. Arrow points to fusion protein band.
FIG. 17 depicts bioanalyzer analysis of an exemplary electrophoresis gel of recombinantly expressed and purified dCas9-Fl26-Tn5 fusion protein.
FIG. 18 depicts an exemplary SDS-PAGE gel analysis of recombinantly expressed and purified dCas9-xTen-Tn5 fusion protein. Arrow points to fusion protein band.
FIG. 19 depicts bioanalyzer data from exemplary electrophoresis analysis of recombinantly expressed and purified dCas9-xTen-Tn5 fusion protein.
FIG. 20 depicts an exemplary SDS-PAGE gel analysis of recombinantly expressed and purified Tn5-Fl26-dCas9 fusion protein. Arrow points to fusion protein band.
FIG. 21 depicts an exemplary SDS-PAGE gel analysis of recombinantly expressed and purified Tn5-xTen-dCas9 fusion protein. Arrow points to fusion protein band.
FIG. 22 depicts tape station analysis of amplification reactions using catalytically active Cas9 only (no fusion protein). No amplification is observed, suggesting that Cas9 itself cannot add adaptors to the 5' and 3' ends of digested fragments. The visible signal is from sample incubated with Cas9, but not subjected to PCR. The lower peak is a 100 bp size marker, and the upper peak is genomic DNA.
FIG. 23 depicts tape station analysis of amplification reactions following tagmentation reactions with dCas9-Fl26-Tn5. Arrow indicates signal from a reaction subjected to PCR conditions following incubation with Cas9-Tn5 fusion protein. No gRNAs were included in this reaction, resulting in a broad peak indicative of random tagmentation. The lower peak is a 100 bp size marker, and the upper peak is genomic DNA.
FIG. 24 depicts exemplary tape station analysis of amplification reactions following tagmentation reactions with dCas9-xTen-Tn5. Arrow indicates signal from a reaction subjected to PCR conditions following incubation with Cas9-Tn5 fusion protein. No gRNAs were included in this reaction, resulting in a broad peak indicative of random tagmentation. The lower peak is a 100 bp size marker, and the upper peak is genomic DNA.
FIG. 25 depicts tape station analysis of amplification reactions following tagmentation with 100 nM dCas9-Fl26-Tn5 fusion protein. Arrow indicates signal from reactions subjected to PCR conditions following incubation with Cas9-Tn5 fusion protein. The lower peak is a 100 bp size marker, and the upper peak is genomic DNA.
FIG. 26 depicts tape station analysis of amplification reactions following tagmentation reactions with 1 nM dCas9-Fl26-Tn5 fusion protein. Arrow indicates signal from reactions subjected to PCR conditions following incubation with Cas9-Tn5 fusion protein. The lower peak is a 100 bp size marker, and the upper peak is genomic DNA.
FIG. 27 depicts tape station analysis of amplification reactions following tagmentation reactions with 100 pM dCas9-Fl26-Tn5 fusion protein. Arrow indicates signal from reactions subjected to PCR conditions following incubation with Cas9-Tn5 fusion protein. The lower peak is a 100 bp size marker, and the upper peak is genomic DNA.
FIG. 28 depicts tape station analysis of amplification reactions following tagmentation reactions with 100 pM dCas9-Fl26-Tn5 fusion protein from FIG. 27 zoomed in.
FIG. 29 depicts tape station analysis of amplification reactions following tagmentation reactions with 100 pM dCas9-xTen-Tn5 fusion protein. Lower, lower 100 bp marker.
FIG. 30 depicts tape station analysis of amplification reactions following tagmentation reactions with 10 pM dCas9-xTen-Tn5 fusion protein. Lower, lower 100 bp marker.
FIG. 31 depicts tape station analysis of amplification reactions following tagmentation reactions with 1 pM dCas9-xTen-Tn5 fusion protein.
FIG. 32 depicts bioanalyzer analysis of amplification from libraries prepared by Tn5 only tagmentation, loaded with only one adaptor (Adaptor B).
FIG. 33 depicts bioanalyzer analysis of amplification from libraries prepared by dCas9-Fl26-Tn5 guided tagmentation, loaded with only one adaptor (Adaptor B). In this experiment, the shorter incubation protocol was used.
FIG. 34 depicts bioanalyzer analysis of amplification from libraries prepared by dCas9-Fl26-Tn5 guided tagmentation, loaded with only one adaptor (Adaptor B). In this experiment, the longer incubation protocol was used.
FIG. 35 depicts exemplary bioanalyzer analysis of amplification from libraries prepared by dCas9-Fl26-Tn5 guided tagmentation, loaded with both Adaptors A and B. In this experiment, the longer incubation protocol was used.
FIG. 36 depicts exemplary bioanalyzer analysis of amplification from libraries prepared by dCas9-Fl26-Tn5 guided tagmentation, loaded with both Adaptors A and B. In this experiment, the shorter incubation protocol was used.
FIG. 37 depicts an exemplary embodiment of DNA fragments labeled with NGS sequences adaptors using the CasTn-NEBNext Ligation based Library methods disclosed herein.
FIG. 38 depicts exemplary tape station analysis of PCR amplification from S. enterica genomic DNA samples incubated with dCas9-xTen-Tn5 loaded with S. enterica sgRNAs. Lower, lower 100 bp marker.
FIG. 39 depicts exemplary tape station analysis of PCR amplification from S. *enterica* samples incubated with dCas9-xTen-Tn5 without sgRNA. Lower, lower 100 bp marker.
FIG. 40 shows an illustration of a dCas9-Tn5 generated fragment using a single adaptor (e.g., Adaptor B).
FIG. 41 depicts an illustration of a dCas9-Tn5 generated fragment from a reaction in which the Tn5 was loaded with two different adaptors (e.g., Adaptor A and Adaptor B).
FIG. 42A-FIG. 42B depict an illustration of NEBNext Ligation-based library preparation for next generation sequencing. Symbols as shown in the key label portions of adaptor and primer sequences. Fragments generated by dCas9-Tn5 tagmentation with NEBNext library preparation are shown in FIG. 37.
FIG. 43 depicts an illustration of tagmentation based Nextera library preparation for next generation sequencing.
FIG. 44 depicts tagmentation based library preparation using dCas9-Tn5 guided tagmentation.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

Disclosed herein include compositions. In some embodiments, the composition comprises: a plurality of protein complexes. In some embodiments, each of the plurality of protein complexes comprises a transposome and a programmable DNA binding unit capable of specifically binding to a binding site on a target double-stranded DNA (dsDNA). In some embodiments, the transposome comprises a transposase, a first adaptor and a second adaptor. In some embodiments, the binding site for each of the plurality of protein complexes is different from each other.

Disclosed herein include reaction mixtures. In some embodiments, the reaction mixture comprises: a composition disclosed herein and sample nucleic acids suspected of comprising one or more target dsDNA. The reaction mixture can comprise: a DNA polymerase, dNTPs, or a combination thereof. In some embodiments, the adaptor is covalently attached to the target dsDNA or a fragment thereof. The reaction mixture can comprise: a plurality of dsDNA fragments each comprising the first adaptor and the second adaptor of one of the plurality of protein complexes at each terminus respectively. In some embodiments, the sample nucleic acids comprises eukaryotic DNA, bacterial DNA, viral DNA, fungal DNA, protozoa DNA, or a combination thereof. In some embodiments, the target dsDNA is genomic DNA, mitochondria DNA, plasmid DNA, or a combination thereof. In some embodiments, the sample nucleic acids are from a biological sample, a clinical sample, an environmental sample, or a combination thereof. In some embodiments, the biological sample comprises stool, sputum, peripheral blood, plasma, serum, lymph nodes, respiratory tissue, exudates, bodily fluid, or a combination thereof.

Disclosed herein include methods for tagging nucleic acids. In some embodiments, the method comprises: contacting the composition disclosed herein with a sample suspected of comprising a plurality of target dsDNA to form a reaction mixture; and incubating the reaction mixture to generate a plurality of dsDNA fragments each comprising the first adaptor and the second adaptor of one of the plurality of protein complexes at each terminus respectively.

Disclosed herein include methods for generating a sequencing library. In some embodiments, the method comprises: contacting the composition disclosed herein with a sample suspected of comprising a plurality of target double-stranded DNA (dsDNA) to form a reaction mixture. The method can comprise: incubating the reaction mixture to generate a plurality of dsDNA fragments each comprising the first adaptor and the second adaptor of one of the plurality of protein complexes at each terminus respectively. The method can comprise: amplifying the plurality of dsDNA fragments with primers capable of binding to the adaptors at the termini of the dsDNA fragments to generate a sequencing library.

### Definitions

Unless defined otherwise, technical and scientific terms used herein have the same meaning as commonly understood by one of ordinary skill in the art to which the present disclosure belongs. *See, e.g.* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence capable of facilitating amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adaptors can be linear. The adaptors can be pre-adenylated adaptors. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adaptor can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adaptors can comprise identical and/or universal nucleic acid sequences and the 3' adaptors can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach adaptors (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, a "complementary" sequence can refer to a "complement" or a "reverse complement" of a sequence. It is understood from the disclosure that if a molecule can hybridize to another molecule it may be complementary, or partially complementary, to the molecule that is hybridizing.

As used herein, the term "label" or "labels" can refer to nucleic acid codes associated with a target within a sample. A label can be, for example, a nucleic acid label. A label can be an entirely or partially amplifiable label. A label can be entirely or partially sequenceable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein n is 1 or 2. LNA and LNA analogs can display very high duplex thermal stabilities with complementary nucleic acid (Tm=+3 to +10°C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used here, the term "target" can refer to a nucleic acid of interest (e.g., target dsDNA). In some embodiments, targets can be associated with an adaptor and/or a barcode. Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

As used herein, the term "isolate nucleic acids" can refer to the purification of nucleic acids from one or more cellular components. One of skill in the art will appreciate that samples processed to "isolate nucleic acids" therefrom can include components and impurities other than nucleic acids. Samples that comprise isolated nucleic acids can be prepared from specimens using any acceptable method known in the art. For example, cells can be lysed using known lysis agents, and nucleic acids can be purified or partially purified from other cellular components. Suitable reagents and protocols for DNA and RNA extractions can be found in, for example, U.S. Patent Application Publication Nos. US 2010-0009351, and US 2009-0131650, respectively.

As used herein, "template" can refer to all or part of a polynucleotide containing at least one target nucleotide sequence.

As used herein, a "primer" can refer to a polynucleotide that can serve to initiate a nucleic acid chain extension reaction. The length of a primer can vary, for example, from about 5 to about 100 nucleotides, from about 10 to about 50 nucleotides, from about 15 to about 40 nucleotides, or from about 20 to about 30 nucleotides. The length of a primer can be about 10 nucleotides, about 20 nucleotides, about 25 nucleotides, about 30 nucleotides, about 35 nucleotides, about 40 nucleotides, about 50 nucleotides, about 75 nucleotides, about 100 nucleotides, or a range between any two of these values. In some embodiments, the primer has a length of 10 to about 50 nucleotides, *i.e.,* 10, 11, 12, 13, 14, 15, 16, 17, 18, 19, 20, 21, 22, 23, 24, 25 26, 27, 28, 29, 30, 31, 32, 33, 34, 35, 36, 37, 38, 39, 40, 41, 42, 43, 44, 45, 46, 47, 48, 49, 50, or more nucleotides. In some embodiments, the primer has a length of 18 to 32 nucleotides.

As used herein, a "probe" can refer to a polynucleotide that can hybridizes (e.g., specifically) to a target sequence in a nucleic acid, under conditions that allow hybridization, thereby allowing detection of the target sequence or amplified nucleic acid. A probe's "target" generally refers to a sequence within or a subset of an amplified nucleic acid sequence which hybridizes specifically to at least a portion of a probe oligomer by standard hydrogen bonding (i.e., base pairing). A probe may comprise target-specific sequences and other sequences that contribute to three-dimensional conformation of the probe. Sequences are "sufficiently complementary" if they allow stable hybridization in appropriate hybridization conditions of a probe oligomer to a target sequence that is not completely complementary to the probe's target-specific sequence. The length of a probe can vary, for example, from about 5 to about 100 nucleotides, from about 10 to about 50 nucleotides, from about 15 to about 40 nucleotides, or from about 20 to about 30 nucleotides. The length of a probe can be about 10 nucleotides, about 20 nucleotides, about 25 nucleotides, about 30 nucleotides, about 35 nucleotides, about 40 nucleotides, about 50 nucleotides, about 100 nucleotides, or a range between any two of these values. In some embodiments, the probe has a length of 10 to about 50 nucleotides. For example, the primers and or probes can be at least 10, 11, 12, 13, 14, 15, 16, 17, 18, 19, 20, 21, 22, 23, 24, 25 26, 27, 28, 29, 30, 31, 32, 33, 34, 35, 36, 37, 38, 39, 40, 41, 42, 43, 44, 45, 46, 47, 48, 49, 50, or more nucleotides. In some embodiments, the probe can be non-sequence specific.

Preferably, the primers and/or probes can be between 8 and 45 nucleotides in length. For example, the primers and or probes can be at least 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or more nucleotides in length. The primer and probe can be modified to contain additional nucleotides at the 5' or the 3' terminus, or both. One of skill in the art will appreciate that additional bases to the 3' terminus of amplification primers (not necessarily probes) are generally complementary to the template sequence. The primer and probe sequences can also be modified to remove nucleotides at the 5' or the 3' terminus. One of skill in the art will appreciate that in order to function for amplification, the primers or probes will be of a minimum length and annealing temperature as disclosed herein.

Primers and probes can bind to their targets at an annealing temperature, which is a temperature less than the melting temperature (Tₘ). As used herein, "Tₘ" and "melting temperature" are interchangeable terms which refer to the temperature at which 50% of a population of double-stranded polynucleotide molecules becomes dissociated into single strands. The formulae for calculating the Tₘ of polynucleotides are well known in the art. For example, the Tₘ may be calculated by the following equation: Tₘ = 69.3+0.41 × (G+C)%-6- 50/L, wherein L is the length of the probe in nucleotides. The Tₘ of a hybrid polynucleotide may also be estimated using a formula adopted from hybridization assays in 1 M salt, and commonly used for calculating Tₘ for PCR primers: [(number of A+T) × 2°C + (number of G+C) x 4°C]. *See, e.g.,* C. R. Newton et al. PCR, 2nd ed., Springer-Verlag (New York: 1997), p.24. Other more sophisticated computations exist in the art, which take structural as well as sequence characteristics into account for the calculation of Tₘ. The melting temperature of an oligonucleotide can depend on complementarity between the oligonucleotide primer or probe and the binding sequence, and on salt conditions. In some embodiments, an oligonucleotide primer or probe provided herein has a Tₘ of less than about 90°C in 50 mM KCl, 10 mM Tris-HCl buffer, for example about 89°C, 88, 87, 86, 85, 84, 83, 82, 81, 80 79, 78, 77, 76, 75, 74, 73, 72, 71, 70, 69, 68, 67, 66, 65, 64, 63, 62, 61, 60, 59, 58, 57, 56, 55, 54, 53, 52, 50, 49, 48, 47, 46, 45, 44, 43, 42, 41, 40, 39°C, or less, including ranges between any two of the listed values.

In some embodiments, the primers disclosed herein*, e.g*., amplification primers, can be provided as an amplification primer pair, *e.g.*, comprising a forward primer and a reverse primer (first amplification primer and second amplification primer). Preferably, the forward and reverse primers have Tₘ's that do not differ by more than 10°C, *e.g.,* that differ by less than 10°C, less than 9°C, less than 8°C, less than 7°C, less than 6°C, less than 5°C, less than 4°C, less than 3°C, less than 2°C, or less than 1°C.

The primer and probe sequences may be modified by having nucleotide substitutions (relative to the target sequence) within the oligonucleotide sequence, provided that the oligonucleotide contains enough complementarity to hybridize specifically to the target nucleic acid sequence. In this manner, at least 1, 2, 3, 4, or up to about 5 nucleotides can be substituted. As used herein, the term "complementary" can refer to sequence complementarity between regions of two polynucleotide strands or between two regions of the same polynucleotide strand. A first region of a polynucleotide is complementary to a second region of the same or a different polynucleotide if, when the two regions are arranged in an antiparallel fashion, at least one nucleotide of the first region is capable of base pairing with a base of the second region. Therefore, it is not required for two complementary polynucleotides to base pair at every nucleotide position. "Fully complementary" can refer to a first polynucleotide that is 100% or "fully" complementary to a second polynucleotide and thus forms a base pair at every nucleotide position. "Partially complementary" also can refer to a first polynucleotide that is not 100% complementary (e.g., 90%, or 80% or 70% complementary) and contains mismatched nucleotides at one or more nucleotide positions. In some embodiments, an oligonucleotide includes a universal base.

As used herein, the term "sufficiently complementary" can refer to a contiguous nucleic acid base sequence that is capable of hybridizing to another base sequence by hydrogen bonding between a series of complementary bases. Complementary base sequences can be complementary at each position in the oligomer sequence by using standard base pairing (e.g., G:C, A:T or A:U) or can contain one or more residues that are not complementary (including abasic positions), but in which the entire complementary base sequence is capable of specifically hybridizing with another base sequence in appropriate hybridization conditions. Contiguous bases can be at least about 80%, at least about 85%, at least about 90%, at least about 95%, at least about 99%, or 100% complementary to a sequence to which an oligomer is intended to hybridize. Substantially complementary sequences can refer to sequences ranging in percent identity from 100, 99, 98, 97, 96, 95, 94, 93, 92, 91, 90, 89, 88, 87, 86, 85, 84, 83, 82, 81, 80, 75, 70 or less, or any number in between, compared to the reference sequence. A skilled artisan can readily choose appropriate hybridization conditions which can be predicted based on base sequence composition, or be determined by using routine testing (*see e.g.,* Green and Sambrook, Molecular Cloning, A Laboratory Manual, 4th ed. (Cold Spring Harbor Laboratory Press, Cold Spring Harbor, N.Y., 2012)).

As used herein, the term "multiplex PCR" refers to a type of PCR where more than one set of primers is included in a reaction allowing one single target, or two or more different targets, to be amplified in a single reaction vessel (e.g., tube). The multiplex PCR can be, for example, a real-time PCR.

Disclosed herein include methods, compositions, kits and systems that enable rapid targeted sequencing (and thus fast sequencing-based diagnosis, e.g., less than 2 hours) and theranostics which requires simultaneous diagnose and determination of suitable therapy approach. In some embodiments, applications of a rapid targeted sequencing approach can comprise: fast pathogen diagnostics, fast Cancer diagnostics, rare disease diagnostics (e.g., for cystic fibrosis).

Disclosed herein include methods for making customized loci-specific libraries for DNA and RNA sequencing using genome editing tools (e.g., Cas proteins, Zinc Finger Nucleases (ZFN), Transcription activator-like effector nucleases (TALEN), and Argonaute proteins) to guide an enzyme (e.g., a transposase) to cut nucleic acid at user-defined loci. The enzyme (e.g., transposase) can add to these sites adaptors for sequencing, for example sequencing by next-generation or third generation sequencing technologies (including but not limited to sequencing technologies by Illumina, PacBio, Roche, Thermo Fisher, and Oxford Nanopore).

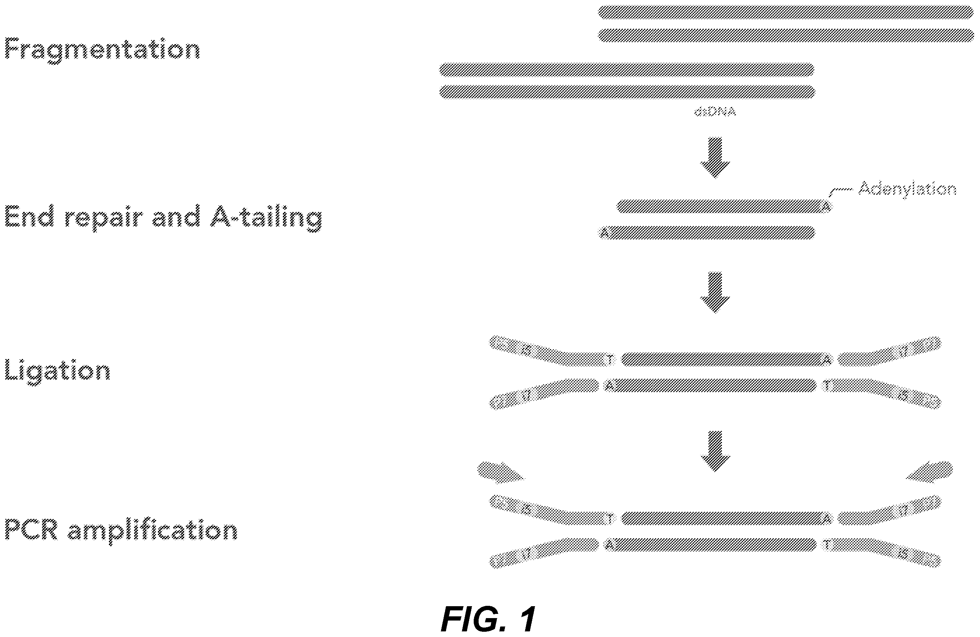

The conventional library preparation methods for sequencing nucleic acids can take hours to make and the process produces libraries that are randomly made (FIG. 1). The reason for these libraries being random is because the methods used for fragmenting nucleic acids (including physical, enzymatic, and chemical fragmentation methods) do so in a random manner, so that the output of the DNA sequencing cannot be controlled.

When there is an interest to study specific loci in a genome, millions of bases would have to be sequenced in the hopes that there will be enough sequence information on these loci. Once all this data are acquired, bioinformatical methods must be used to extract the information on the loci of interest. This process can be both bioinformatically and computationally intensive since most of the DNA that has been prepared and sequenced is of no relevance to these areas of interest. In addition, because of the randomness of the library preparation process there is a risk that there will not be enough information (coverage) in these areas. In this case another library would have to be made and sequenced again in the hopes of getting enough coverage of these areas, wasting both time and resources.

The rapid targeted library preparation method disclosed herein for sequencing using the Customized Loci-specific Library Preparation (CLLP) method is a fast process that takes only minutes to make instead of the hours required to make a library using the conventional library preparation methods. In addition, the libraries made by the CLLP method disclosed herein are not random. In some embodiments, only the loci that have been selected are sequenced and everything else can be ignored, which provides cost effectiveness, time and resource savings, and precision. Furthermore, by only sequencing the areas of interest, the bioinformatic resources and the analysis needed will be minimal in comparison to standard methods. The Customized Loci-specific Library Preparation (CLLP) method disclosed herein enables the use of DNA sequencing as a fast and affordable method for diagnostic and/or theranostics.

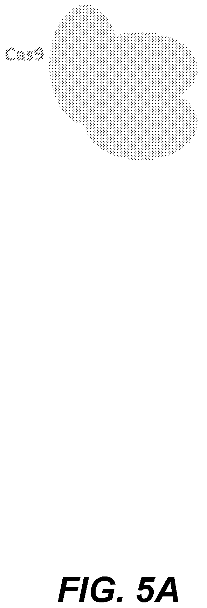

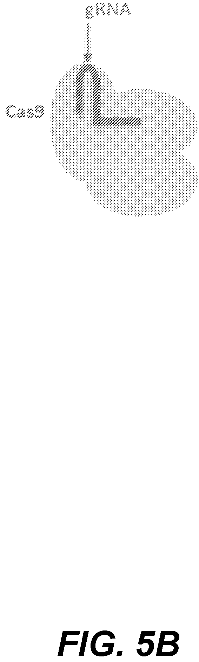

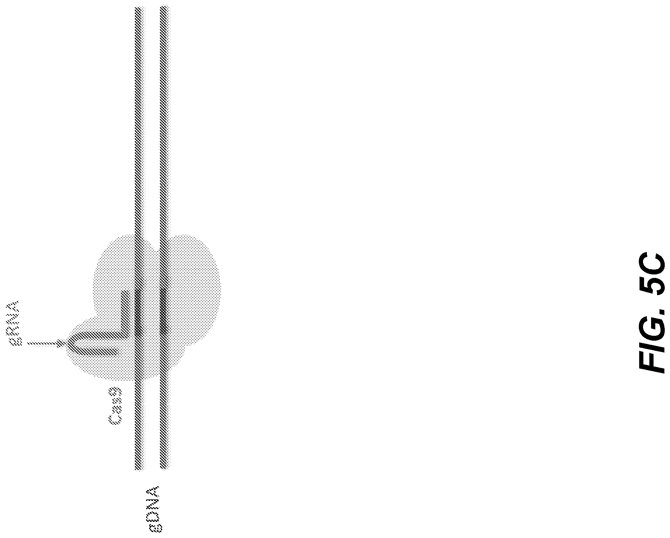

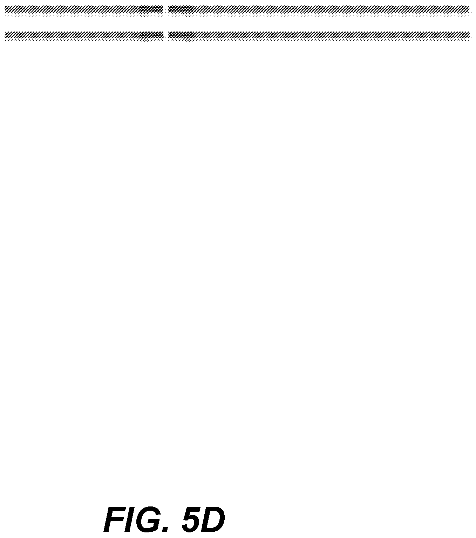

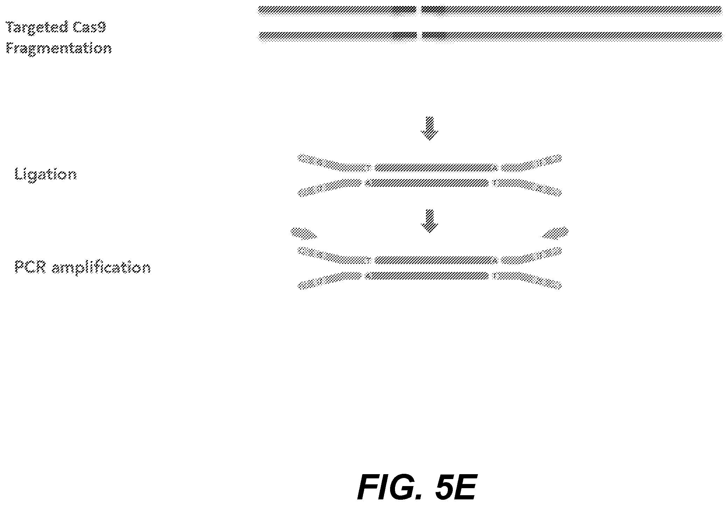

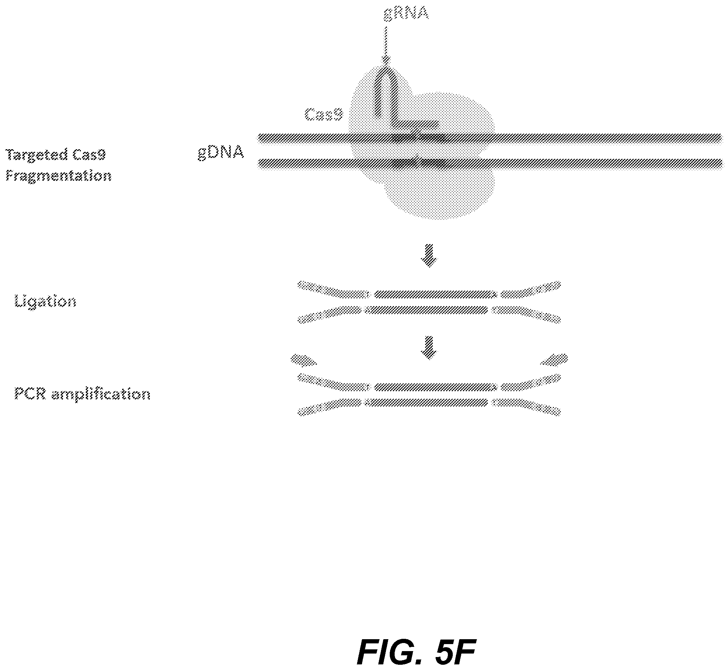

In some embodiments of CLLP, genome editing tools and a transposase (e.g., a hyperactive transposase) are used to achieve targeted fragmentation. Any genome editing tools that can make a user-defined double-strand breaks in DNA or a single-strand breaks in RNA can be used. These tools include, but are not limited to, CAS proteins, ZFN, TALEN, Argonaute proteins, or any combination thereof. In some embodiments, the genome editing tools are used to control and direct the fragmentation of the nucleic acid to specific areas of the genome which can be precisely selected. The cuts made by the genome editing tools can be used as priming sites for sequencing adaptors. This in turn will heavily bias the areas of the genome that will be sequenced. The programmable fragmentation process disclosed herein can lead to targeted sequencing. Furthermore, this method can be used with any sequencing technology (including, but not limited to, the sequencing technologies of Illumina, PacBio, Oxford Nanopore, Roche and Thermo Fisher). FIG. 5A-FIG. 5F depict a non-limiting exemplary embodiment of Customized Loci-specific Library Preparation (CLLP) disclosed herein.

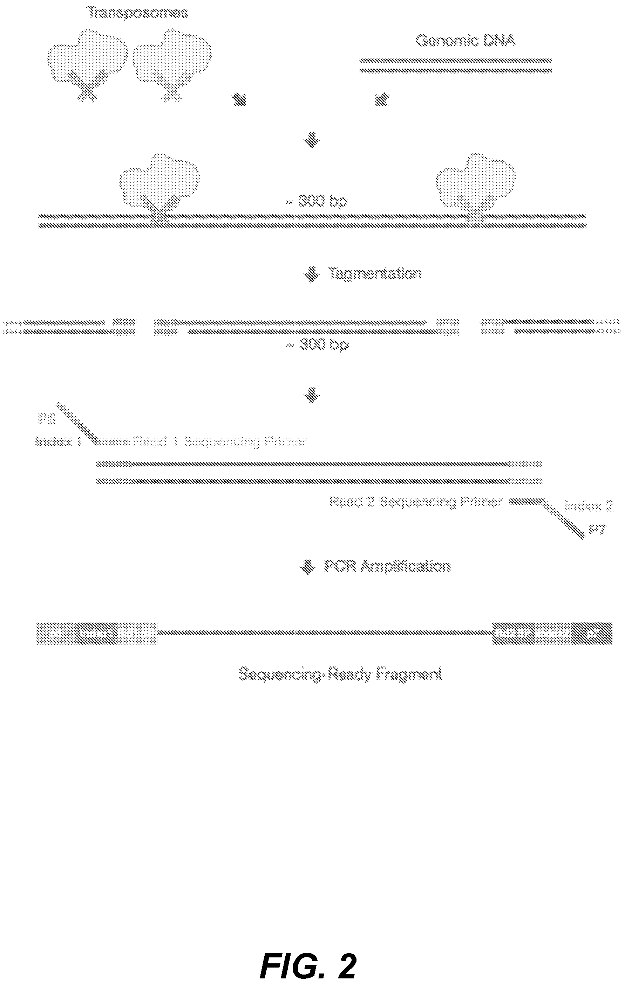

In some embodiments, tagmentation comprises making libraries for DNA sequencing that utilizes a hyperactive transposase. Tagmentation uses a transposon that cleaves double-stranded DNA and pastes DNA adaptors at the cut site (FIG. 2). Tagmentation is a very fast process that make libraries in considerably less time than the standard library preparation methods. However, transposons cut genomes in a random unbiased manner.

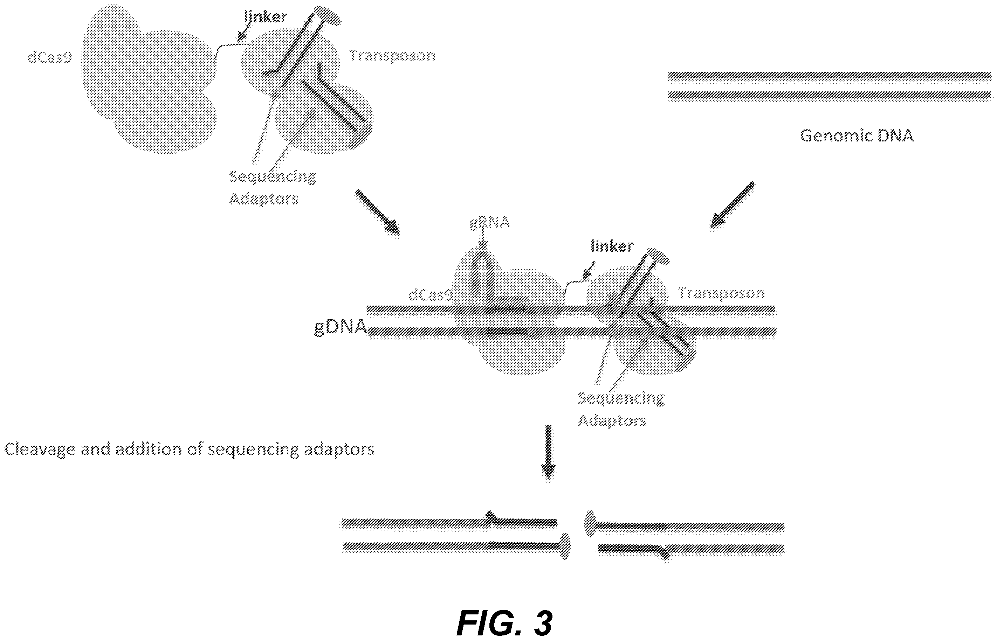

In some embodiments, to improve the speed of library preparation, the method disclosed herein uses a transposon linked to a genome editing tool. For example, a dCAS9 protein can be used as the genome editing tool. The dCAS9 protein, can be bound to a guide RNA which is programmable to a specific area in the genome. The dCAS9, is a type of CAS9 protein that is mutated so the nuclease activity of the CAS9 protein is lost but it retains the target specificity. Once the dCAS9 binds its target, the transposase, which is attached to the CAS9 protein, will cut the DNA and attach to that cut site adaptors for sequencing. The end results are targeted DNA fragments which are ready for sequencing, shortening the targeted library preparation process to minutes instead of hours (FIG. 3).

Non-limiting advantages that the methods, compositions, kits and systems of present disclosure enable include: faster time to results; use of less laboratory, bioinformatic, and computational resources than prior art; allows the rapid detection and quantification of rare and low frequency variants; capable of being able to analyze more samples than whole genome sequencing; can be used as a rapid diagnostic tool capable of simultaneously detect multiple customizable number of targets; simpler and straightforward data analysis; and any combination thereof.

Currently, there are two methods that have been used for targeted sequencing. The first one is amplicon sequencing. This method relies on using primers to amplify areas of interest via DNA amplification. This additional amplification step adds further costs, time, and resources to the standard library preparation methods. The second targeted sequencing method is target capture. This method relies on using a probe or a pool of probes so they can hybridize to specific nucleic acid targets. The hybridization of the probes to its target and the isolation of these targets is a time-consuming process that can take days. Furthermore, the probes used in this method are expensive to synthetize.

In some embodiments, the hyperactive transposase Tn5 linked to a dCAS9 protein can be used. The dCAS9, is a catalytic dead version of the CAS9 protein that is mutated so the nuclease activity of the CAS9 protein is lost but it retains the programmable DNA binding activity. The N-terminus of the dCAS9 protein is attached to the C-terminus of the Tn5 transposase via a linker (e.g., X-TEN), a SNAP-tag or CLIP-tag. Although many different methods can be employed to attach both proteins. The TN5 transposase will be loaded with sequencing adapters that are specific to a sequencing technology platform. The dCas9 protein will be attached with a guide RNA (sgRNA) that is specific to a user-defined locus. Multiple sgRNA each bound to a dCAS9 protein and each targeting different loci be used to select multiple loci.

Once the dCAS9, which is attached to an sgRNA finds the molecule that is complementary to the sgRNA sequence, the attached Tn5 transposase can cut the DNA at a specified site and attach to that cut site adaptors for sequencing. The end results are targeted DNA fragments which are ready for sequencing, shortening the targeted library preparation process to minutes instead of hours (FIG. 3).

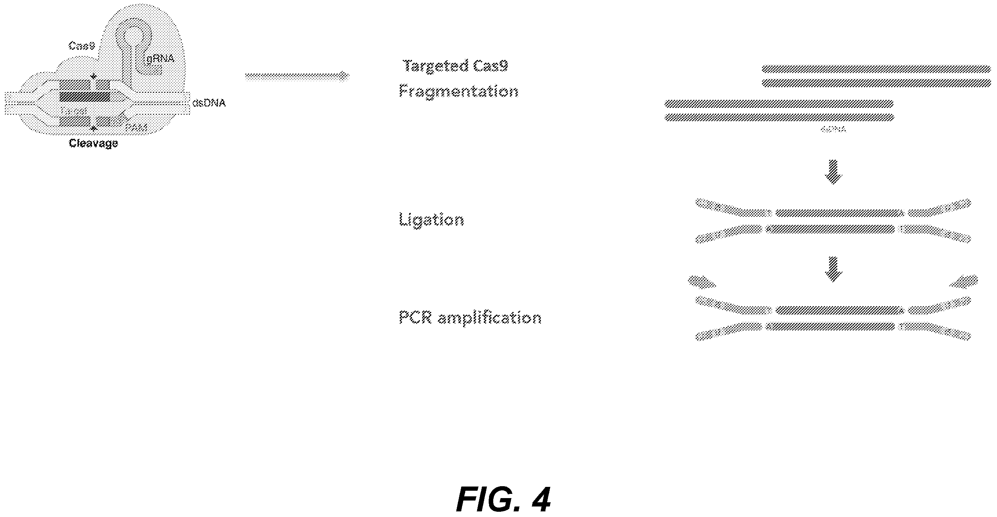

In some embodiments, a hyperactive transposase that is not Tn5 transposase can be mariner Tc1-like transposon, Himar1C9 transposase, Sleeping Beauty transposase, the Tn7 transposon, or a combination thereof. In some embodiments, alternatives to the dCas9 protein can be used for the programmable DNA binding activity. For example, the usage of Zinc Fingers that are not bound to the FOK1 nuclease can be used. Similarly, a TALEN molecule without the FOK1 nuclease can be use. In some embodiments, the use of a recombinase in conjunction with by a sequence specific primer could be used as a programmable DNA binding molecule. In some embodiments, an alternative method for making loci-specific libraries can be done by using only the genome editing tools (e.g., Cas proteins, Zinc Finger Nucleases (ZFN), Transcription activator-like effector nucleases (TALEN), Argonaute proteins) without the assistance of the transposase. This will result in a programmable fragmentation method of nucleic acids (FIG. 4) that can further be used for making a loci-specific sequencing library.

The use of a genome editing tool as a programmable tool to target specific areas of a genome and the use of a transposase to cut and paste adaptors needed for creating a sequencing library are disclosed herein.

Some embodiments provide a disease panel (e.g., a sepsis panel) configured to identify pathogens/cause of disease (gene mutation(s)) and simultaneously identify susceptibility to antibiotics. In some embodiments, a cancer panel can comprise identification of multiple mutations in cancer cells. In some embodiments, a rare disease panel can comprise sequencing of specific loci that have associated with mutations that can lead to genetic diseases (e.g., cystic fibrosis).

Disclosed herein include compositions. In some embodiments, the composition comprises: a plurality of protein complexes. In some embodiments, each of the plurality of protein complexes comprises a transposome and a programmable DNA binding unit capable of specifically binding to a binding site on a target dsDNA. In some embodiments, the transposome comprises a transposase, a first adaptor and a second adaptor. In some embodiments, the binding site for each of the plurality of protein complexes is different from each other.

Disclosed herein include reaction mixtures. In some embodiments, the reaction mixture comprises: a composition disclosed herein and sample nucleic acids suspected of comprising one or more target dsDNA. The reaction mixture can comprise: a DNA polymerase, dNTPs, or a combination thereof. The adaptor can be covalently attached to the target dsDNA or a fragment thereof. The reaction mixture can comprise: a plurality of dsDNA fragments each comprising the first adaptor and the second adaptor of one of the plurality of protein complexes at each terminus respectively.

Disclosed herein include methods for tagging nucleic acids. In some embodiments, the method comprises: contacting the composition disclosed herein with a sample suspected of comprising a plurality of target dsDNA to form a reaction mixture; and incubating the reaction mixture to generate a plurality of dsDNA fragments each comprising the first adaptor and the second adaptor of one of the plurality of protein complexes at each terminus respectively.

Disclosed herein include methods for generating a sequencing library. In some embodiments, the method comprises: contacting the composition disclosed herein with a sample suspected of comprising a plurality of target dsDNA to form a reaction mixture. The method can comprise: incubating the reaction mixture to generate a plurality of dsDNA fragments each comprising the first adaptor and the second adaptor of one of the plurality of protein complexes at each terminus respectively. Contacting the plurality of target dsDNA with the plurality of protein complex pairs can be carried out at about 25°C to about 85°C (e.g., about 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 45°C, 50°C, 55°C, 60°C, 65°C, 70°C, 75°C, 80°C, 85°C, or a number or a range between any two of these values). Incubating the reaction mixture can comprise incubating the reaction mixture at about 37°C to about 55°C (e.g., about 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, or a number or a range between any two of these values).

The plurality of protein complex pairs and the plurality of target dsDNA can be present in the reaction mixture at a molecular ratio of about 2:1 to about 2000:1 (e.g., 2:1, 2.5:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, 200:1, 300:1, 400:1, 500:1, 600:1, 700:1, 800:1, 900:1, 1000:1, 2000:1, or a number or a range between any two of these values). In some embodiments, the plurality of protein complex pairs and the plurality of target dsDNA are present in the reaction mixture at a molecular ratio of about 2:1 to about 200:1 (e.g., 2:1, 2.5:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, 200:1, or a number or a range between any two of these values).

The binding sites of at least two of the plurality of protein complexes can be on the same target dsDNA. The binding sites of at least two of the plurality of protein complexes can be about 1-50000 nucleotides apart on the same target dsDNA. In some embodiments, the binding sites of at least two of the plurality of protein complexes can be, or can be about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, or a number or a range between any two of these values, nucleotides apart on the same target dsDNA. In some embodiments, the binding sites of at least two of the plurality of protein complexes can be at least, or can be at most, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, or 100000 nucleotides apart on the same target dsDNA. The distance between the binding sites of a pair of the plurality of protein complexes can be substantially the same as the distance between the binding sites of another pair of the plurality of protein complexes. The distance between the binding sites of a pair of the plurality of protein complexes can be different as the distance between the binding sites of another pair of the plurality of protein complexes. The binding sites of at least two of the plurality of protein complexes can be on the different strand of a target dsDNA. At least two of the plurality of protein complexes can be capable of specifically binding to different target dsDNA. The plurality of protein complex can be capable of specifically binding to about 2-5000 target dsDNA. In some embodiments, plurality of protein complexes is capable of specifically binding to about, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 110, 120, 128, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1200, 1300, 1400, 1500, 1600, 1700, 1800, 1900, 2000, 2100, 2200, 2300, 2400, 2500, 2600, 2700, 2800, 2900, 3000, 3250, 3500, 3750, 4000, 4250, 4500, 4750, 5000, 5500, 6000, 6500, 7000, 7500, 8000, 8500, 9000, 9500, 10000, or a number or a range between any two of these values, target dsDNA.

### Transposomes

In some embodiments, the transposome comprises a transposase, a first adaptor and a second adaptor. At least two of the plurality of protein complexes can comprise the same transposome. All of the plurality of protein complexes can comprise the same transposome. All of the plurality of protein complexes can comprise the same transposase. The transposase can be Tn5 transposase, Tn7 transposase, mariner Tc1-like transposase, Himar1C9 transposase, or Sleeping Beauty transposase. The transposase can be a hyperactive transposase.

The transposase can be a Tn5, Tn7, MuA, or *Vibrio harveyi* transposase, or an active mutant thereof. In some embodiments, the transposase is a Tn5 transposase or a mutant thereof. In some embodiments, the Tn5 transposase is a hyperactive Tn5 transposase, or an active mutant thereof. In some embodiments, the Tn5 transposase is a Tn5 transposase as described in WO2015/160895. In some embodiments, the Tn5 transposase is a hyperactive Tn5 with mutations at positions 54, 56, 372, 212, 214, 251, and 338 relative to wild-type Tn5 transposase. In some embodiments, the Tn5 transposase is a hyperactive Tn5 with the following mutations relative to wild-type Tn5 transposase: E54K, M56A, L372P, K212R, P214R, G251R, and A338V. In some embodiments, the Tn5 transposase is a fusion protein. In some embodiments, the Tn5 transposase fusion protein comprises a fused elongation factor Ts (Tsf) tag. In some embodiments, the Tn5 transposase is a hyperactive Tn5 transposase comprising mutations at amino acids 54, 56, and 372 relative to the wild type sequence. In some embodiments, the hyperactive Tn5 transposase is a fusion protein. In some embodiments, the recognition site is a Tn5-type transposase recognition site.

The transposase may comprise a single protein or comprise multiple protein subunits. A transposase may be an enzyme capable of forming a functional complex with a transposon end or transposon end sequences. In some embodiments, the transposase complex comprises a transposase (e.g., a Tn5 transposase) dimer comprising a first and a second monomer. In some embodiments, the transposome complex comprises a dimer of two molecules of a transposase.

The transposase and/or transposome can vary depending on the embodiment. The transposase can comprise a Tn5 transposase. The transposase can be a Tn transposase (e.g., Tn3, Tn5, Tn7, Tn10, Tn552, Tn903), a MuA transposase, a Vibhar transposase (e.g., from *Vibrio harveyi*)*,* Ac-Ds, Ascot-1, Bs1, Cin4, Copia, En/Spm, F element, hobo, Hsmar1, Hsmar2, IN (HIV), IS1, IS2, IS3, IS4, IS5, IS6, IS10, IS21, IS30, IS50, IS51, IS150, IS256, IS407, IS427, IS630, IS903, IS911, IS982, IS1031, ISL2, L1, Mariner, P element, Tam3, Tc1, Tc3, Tel, THE-1, Tn/O, TnA, Tn3, Tn5, Tn7, Tn10, Tn552, Tn903, Tol1, Tol2, Tn10, Ty1, any prokaryotic transposase, or any transposase related to and/or derived from those listed above. In some embodiments, a transposase related to and/or derived from a parent transposase can comprise a peptide fragment with at least about 50%, about 55%, about 60%, about 65%, about 70%, about 75%, about 80%, about 85%, about 90%, about 91%, about 92%, about 93%, about 94%, about 95%, about 96%, about 97%, about 98%, or about 99% amino acid sequence homology to a corresponding peptide fragment of the parent transposase. The peptide fragment can be at least about 10, about 15, about 20, about 25, about 30, about 35, about 40, about 45, about 50, about 60, about 70, about 80, about 90, about 100, about 150, about 200, about 250, about 300, about 400, or about 500 amino acids in length. For example, a transposase derived from Tn5 can comprise a peptide fragment that is 50 amino acids in length and about 80% homologous to a corresponding fragment in a parent Tn5 transposase. In some cases, the insertion can be facilitated and/or triggered by addition of one or more cations. The cations can be divalent cations such as, for example, Ca²⁺, Mg²⁺ and Mn²⁺.

### Adaptors

The first adaptor and the second adaptor in the same transposome can be the same. The first adaptor, the second adaptor, or both, in different transposome can be different. The first adaptor, the second adaptor, or both, can be a dsDNA or an RNA/DNA duplex. The adaptor can be about 3-200 (e.g., about 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, or a number or a range between any two of these values, nucleotides in length) base pairs in length. In some embodiments, the adaptors can be 3-500 (e.g., about 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 300, 400, 500 or a number or a range between any two of these values, nucleotides in length) base pairs in length. The first adaptor, the second adaptor, or both, can be a sequencing adaptor. Sequencing adaptors can comprise one or more components employed in a given sequencing protocol, e.g., sequencing platform adaptor constructs, indexing domains, clustering domains, etc. The sequencing adaptor can comprise P5 or P7 primer sequences. In some embodiments, the first adaptor and/or the second adaptor comprise a barcode (e.g., a stochastic barcode). In some embodiments, the first adaptor and/or the second adaptor comprises a universal sequence. In some embodiments, the first adaptor and/or the second adaptor comprises a single-stranded portion and/or a double-stranded portion. In some embodiments, an adaptor comprises a transposon end sequence that binds to the transposase. The transposon end sequence can be double-stranded. In some embodiments, the transposon end sequence is a mosaic end (ME) sequence. In particular embodiments, the transposon ends are mosaic ends, or hyperactive versions of transposon ends. An adaptor sequence can be attached to one of the two transposon end sequences. Thus, in some embodiments, the first adaptor transposon end sequence is an ME sequence and the second adaptor end sequence is an ME' sequence.

The first adaptor and/or the second adaptor can comprise one or more nucleotides (or analogs thereof) that are modified or otherwise non-naturally occurring. For example, the first adaptor and/or the second adaptor can include one or more nucleotide analogs (e.g., LNA, FANA, 2'-O-Me RNA, 2'-fluoro RNA, or the like), linkage modifications (e.g., phosphorothioates, 3'-3' and 5'-5' reversed linkages), 5' and/or 3' end modifications (e.g., 5' and/or 3' amino, biotin, DIG, phosphate, thiol, dyes, quenchers, etc.), one or more fluorescently labeled nucleotides, or any other feature that provides a desired functionality.

The first adaptor and/or the second adaptor can comprise all or a component of a sequencing platform adapter construct. By "sequencing platform adapter construct" is meant a nucleic acid construct that includes at least a portion of a nucleic acid domain (e.g., a sequencing platform adapter nucleic acid sequence) utilized by a sequencing platform of interest, such as a sequencing platform provided by Illumina^{®} (e.g., the HiSeq^{™}, MiSeq^{™} and/or Genome Analyzer^{™} sequencing systems); Ion Torrent^{™} (e.g., the Ion PGM^{™} and/or Ion Proton^{™} sequencing systems); Pacific Biosciences (e.g., the PACBIO RS II sequencing system); Life Technologies^{™} (e.g., a SOLiD sequencing system); Roche (e.g., the 454 GS FLX+ and/or GS Junior sequencing systems); or any other sequencing platform of interest. The first adaptor and/or the second adaptor can comprise one or more nucleic acid domains selected from: a domain (e.g., a "capture site" or "capture sequence") that specifically binds to a surface-attached sequencing platform oligonucleotide (e.g., the P5 or P7 oligonucleotides attached to the surface of a flow cell in an Illumina^{®} sequencing system); a sequencing primer binding domain (e.g., a domain to which the Read 1 or Read 2 primers of the Illumina^{®} platform may bind); a barcode domain (e.g., a domain that uniquely identifies the sample source of the nucleic acid being sequenced to enable sample multiplexing by marking every molecule from a given sample with a specific barcode or "tag"); a barcode sequencing primer binding domain (a domain to which a primer used for sequencing a barcode binds); a molecular identification domain (e.g., a molecular index tag, such as a randomized tag of 4, 6, or other number of nucleotides) for uniquely marking molecules of interest to determine expression levels based on the number of instances a unique tag is sequenced; or any combination of such domains. In some embodiments, a barcode domain (e.g., sample index tag) and a molecular identification domain (e.g., a molecular index tag) may be included in the same nucleic acid.

A sequencing platform adapter domain, when present in a first adaptor and/or a second adaptor, may include one or more nucleic acid domains of any length and sequence suitable for the sequencing platform of interest. The nucleic acid domains may have a length and sequence that enables a polynucleotide (e.g., an oligonucleotide) employed by the sequencing platform of interest to specifically bind to the nucleic acid domain, e.g., for solid phase amplification and/or sequencing by synthesis of the cDNA insert flanked by the nucleic acid domains. Example nucleic acid domains include the P5 , P7, Read 1 primer and Read 2 primer domains employed on the Illumina^{®}-based sequencing platforms. Other example nucleic acid domains include the A adapter and P1 adapter domains employed on the Ion Torrent^{™}-based sequencing platforms.

The nucleotide sequences of nucleic acid domains useful for sequencing on a sequencing platform of interest may vary and/or change over time. Adapter sequences are typically provided by the manufacturer of the sequencing platform (e.g., in technical documents provided with the sequencing system and/or available on the manufacturer's website). Based on such information, the sequence of the adaptors provided herein may be designed to include all or a portion of one or more nucleic acid domains in a configuration that enables sequencing the target dsDNA on the platform of interest.

The first adaptor and/or the second adaptor can comprise a component of an Ion Torrent^{™} sequencing platform (e.g., the Ion PGM^{™} and/or Ion Proton^{™} sequencing systems). The first adaptor and/or the second adaptor can comprise a P1 adapter, A adapter, an Ion Xpress^{™} barcode adapter, Ion P1 Adapter, and/or an Ion Xpress^{™} Barcode X adapter.

The first adaptor and/or the second adaptor can comprise a hairpin. The first adaptor and/or the second adaptor can be configured to generate a SMRTbell^{™} technology library. The methods provided herein can result in hairpin adaptors ligated onto the ends of the double stranded fragments to produce circular template molecules having a central double-stranded portion and single-stranded hairpin loops at the ends (see SMRTbell^{™} from Pacific Biosciences^{®}). Methods for the preparation and use of cyclic templates such as SMRTBELL^{®} templates are described for example in U.S. Pat. No. 8,003,330, entitled "Error-free amplification of DNA for clonal sequencing", and US2009/0280538 entitled "Methods and compositions for nucleic acid sample preparation".

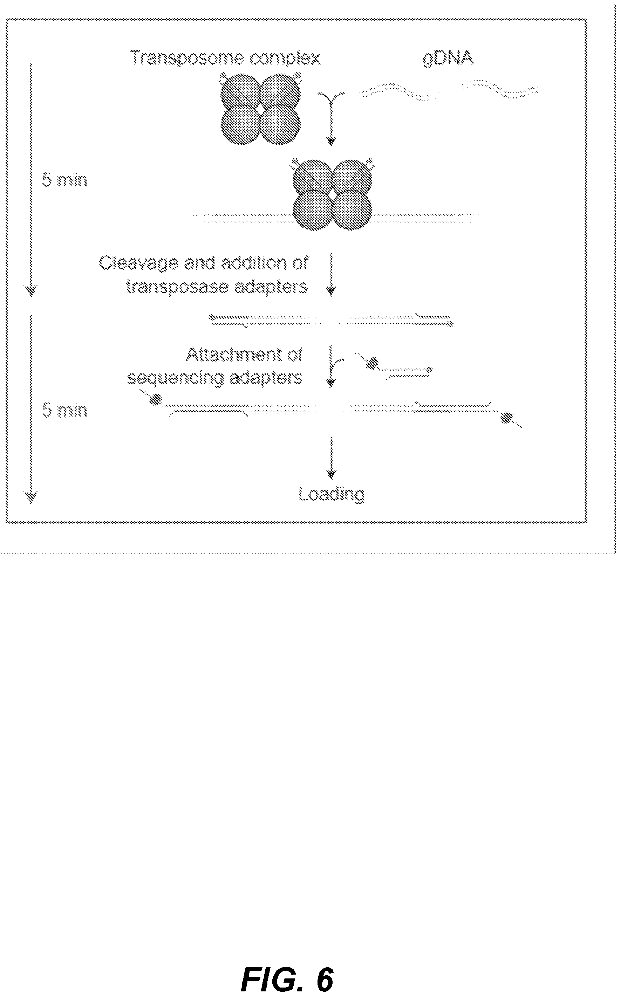

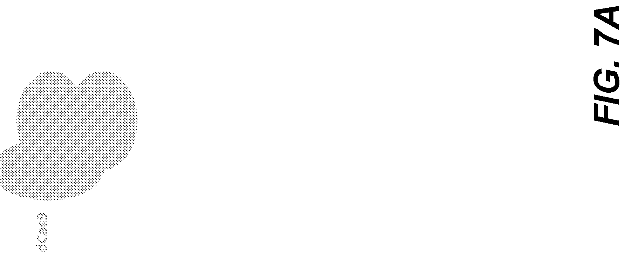

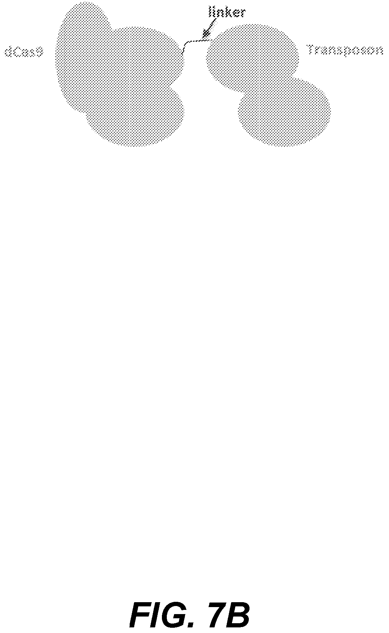

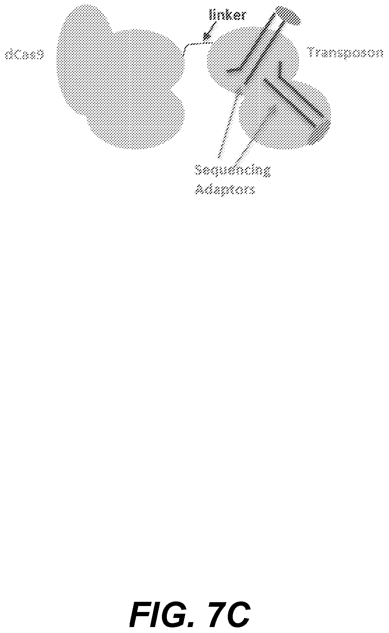

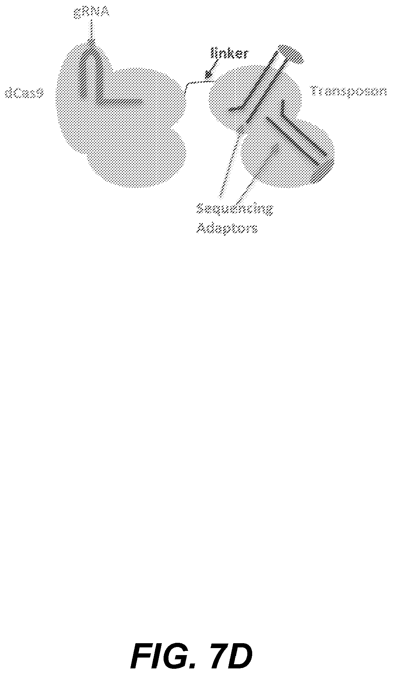

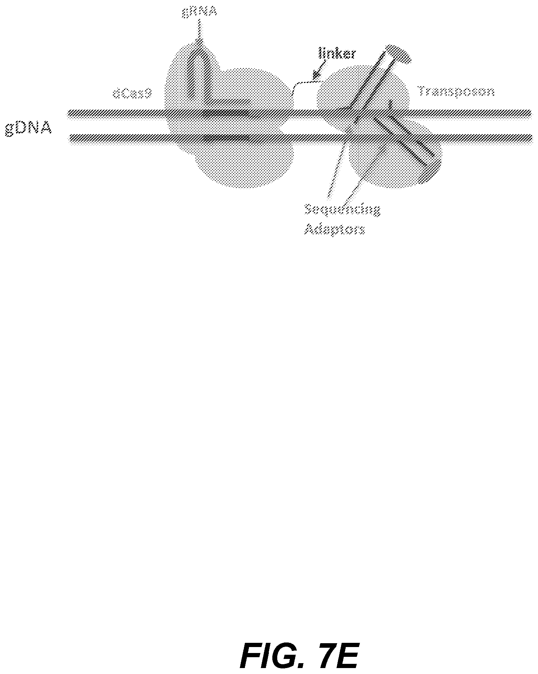

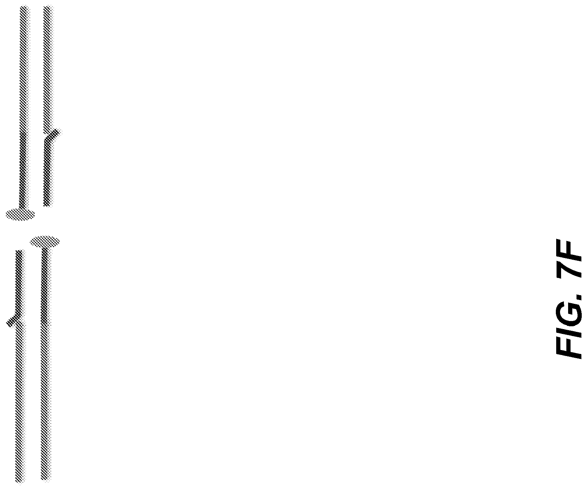

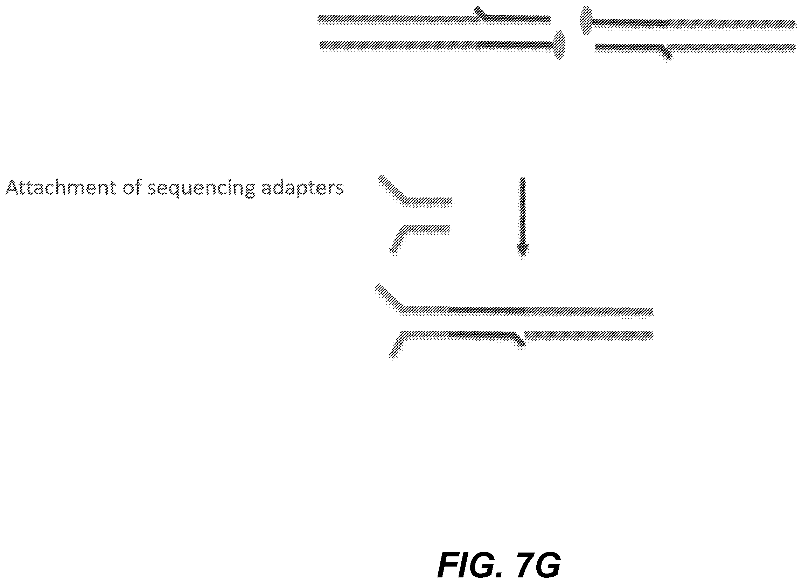

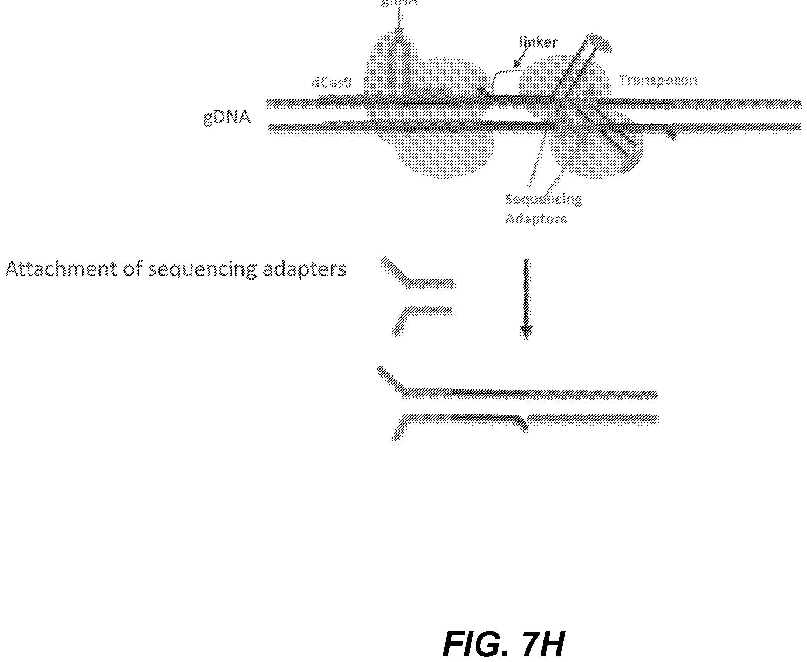

The first adaptor and/or the second adaptor can be configured for downstream use of the tagged nucleic acids on ONT instruments (eg: SmidgION, MinION, GridION, PromethION). FIG. 6 depicts a non-limiting exemplary embodiment showing ONT Rapid Sequencing Kit, tagmentation based-Rapid Sequencing Kit. The first adaptor and/or the second adaptor can comprise (i) a spacer; (ii) a motor protein stalled on the spacer, wherein the active site of the motor protein is occupied by the spacer; and/or (iii) a blocking moiety bound to the adapter, wherein the blocking moiety prevents the motor protein from moving off the spacer. The first adaptor and/or the second adaptor can comprise a hairpin loop adapter. A hairpin loop adapter can be an adapter comprising a single polynucleotide strand, wherein the ends of the polynucleotide strand are capable of hybridizing to each other, or are hybridized to each other, and wherein the middle section of the polynucleotide forms a loop. Suitable hairpin loop adapters can be designed using methods known in the art. The first adaptor and/or the second adaptor can comprise a linear adapter. The first adaptor and/or the second adaptor can be a Y adapter. A Y adapter is typically a polynucleotide adapter. A Y adapter is typically double stranded and comprises (a) at one end, a region where the two strands are hybridized together and (b), at the other end, a region where the two strands are not complementary. The non-complementary parts of the strands typically form overhangs. The presence of a non-complementary region in the Y adapter gives the adapter its Y shape since the two strands typically do not hybridize to each other unlike the double stranded portion. The two single-stranded portions of the Y adapter may be the same length, or may be different lengths. A motor protein may bind to an overhang of an adapter such as a Y adapter. In some embodiments, a motor protein may bind to the double stranded region. In some embodiments, a motor protein may bind to a single-stranded and/or a double-stranded region of the adapter. In some embodiments, a first motor protein may bind to the single-stranded region of such an adapter and a second motor protein may bind to the double-stranded region of the adapter. The first adaptor and/or the second adaptor can comprise additional bound components that facilitate nanopore sequencing reactions, e.g., bound enzymes (e.g., helicases, polymerases, or other motor proteins), membrane binding moieties (e.g., cholesterol), and the like. Typically, the motor protein is a helicase, a polymerase, an exonuclease, a topoisomerase, or a variant thereof. In some embodiments, the motor protein on the spacer of the polynucleotide adapter is modified to prevent the motor protein disengaging from the spacer (other than by passing off the end of the spacer). The motor protein can be adapted in any suitable way. FIG. 7A-FIG. 7H depict a non-limiting exemplary embodiment of Genome Editing Tagmentation (GET) for generating sequencing library to use in existing sequencing platform such as that from Oxford Nanopore.

Adaptors provided herein (e.g., the first adaptor and/or the second adaptor) can comprise a barcode, for example a stochastic barcode, and can comprise one or more labels. Barcoding, such as stochastic barcoding, has been described in, for example, Fu et al., Proc Natl Acad Sci U.S.A., 2011 May 31,108(22):9026-31; US2011/0160078; Fan et al., Science, 2015, 347(6222):1258367; US2015/0299784; and WO2015/031691. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

An adaptor and/or barcode can comprise one or more universal labels. In some embodiments, the one or more universal labels can be the same for all barcodes and/or adaptors. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length.

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. In some embodiments, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprising seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

### CRISPR-associated Proteins

The programmable DNA binding unit can comprise a nuclease-deficient CRISPR associated protein (dCAS protein) and a guide RNA (gRNA) capable of specifically binding to the binding site of the target dsDNA. The dCAS protein can be dCAS9, dCAS12, dCAS13, dCAS14, or SpRY dCAS. The dCAS13 protein can be dCAS13a, dCAS13b, dCAS13c, or dCAS13d.

In some embodiments, a Cas9 protein has an inactive (e.g., an inactivated) DNA cleavage domain. A nuclease-inactivated Cas9 protein can interchangeably be referred to as a "dCas9" protein (for nuclease-dead Cas9). Methods for generating a Cas9 protein (or a fragment thereof) having an inactive DNA cleavage domain are known (See, e.g., Jinek et al., Science.337:816-821(2012); Qi et al., (2013) Cell.28; 152(5): 1173-83). For example, the DNA cleavage domain of Cas9 is known to include two subdomains, the HNH nuclease subdomain and the RuvC1 subdomain. The HNH subdomain cleaves the strand complementary to the gRNA, whereas the RuvCl subdomain cleaves the non-complementary strand. Mutations within these subdomains can silence the nuclease activity of Cas9. For example, the mutations D10A and H840A completely inactivate the nuclease activity of S. pyogenes Cas9 (Jinek et al., and Qi et al.).

The programmable DNA binding unit can comprise a suitable nuclease-deficient Cas Protein that can still bind a guide RNA. The programmable DNA binding unit can comprise a Class 2 Type II Cas protein. The Class 2 Type II Cas protein may be a mutated Cas protein as compared to a wildtype counterpart. The mutated Cas protein can be nuclease-deficient. The mutated Cas protein may be mutated Cas9. The mutated Cas9 may be Cas9D10A. Other examples of mutations in Cas9 include H820A, D839A, H840A, N863A, or any combination thereof, e.g., D10A/H820A, D10A, D10A/D839A/H840A, and D10A/D839A/H840A/N863A. The mutations described here are with reference to SpCas9 and also include an analogous mutation in a CRISPR protein other than SpCas9. The programmable DNA binding unit can comprise *Streptococcus pyogenes* Cas9 (SpCas9), *Staphylococcus aureus* Cas9 (SaCas9), Cas1, Cas1B, Cas2, Cas3, Cas4, Cas5, Cas6, Cas7, Cas8, Cas9, Cas100, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, C2c1, C2c3, Cas12a, Cas12b, Cas12c, Cas12d, Cas12e, Cas13a, Cas13b, Cas13c, derivatives thereof, or any combination thereof. Cas9 molecules of a variety of species can be used in the methods and compositions described herein. While S. pyogenes and S. aureus Cas9 molecules are the subject of much of the disclosure herein, Cas9 molecules of, derived from, or based on the Cas9 proteins of other species listed herein can be used as well. These include, for example, Cas9 molecules from Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp., or Verminephrobacter eiseniae. Catalytically inactivating mutations and means of assessing the nuclease activity of said mutants are known to those of skill in the art.

The programmable DNA binding unit can comprise a guide molecule. A guide RNA molecule (sgRNA) can be composed of two separate molecules; a crRNA which is target specific and tracrRNA, which binds to Cas molecule. In some embodiments, the crRNA and tracrRNA are provided as separate molecules and one has to anneal it to make into a functional sgRNA. As used herein, the term "guide sequence" and "guide molecule" in the context of a CRISPR-Cas system, comprises any polynucleotide sequence having sufficient complementarity with a selected binding site to hybridize with the selected binding site and direct sequence-specific binding of a programmable DNA binding unit to the selected binding site. A gRNA molecule can refer to a nucleic acid that promotes the specific targeting or homing of a gRNA molecule/Cas9 molecule complex to a target binding site. gRNA molecules can be unimolecular (having a single RNA molecule) (e.g., chimeric) or modular (comprising more than one, and typically two, separate RNA molecules). The guide sequences made using the methods disclosed herein may be a full-length guide sequence, a truncated guide sequence, a full-length sgRNA sequence, a truncated sgRNA sequence, or an E+F sgRNA sequence. In some embodiments, the degree of complementarity of the guide sequence to a given binding site, when optimally aligned using a suitable alignment algorithm, is about or more than about 50%, 60%, 75%, 80%, 85%, 90%, 95%, 97.5%, 99%, or more. In certain example embodiments, the guide molecule comprises a guide sequence that may be designed to have at least one mismatch with the binding site, such that an RNA duplex formed between the guide sequence and the binding site. Accordingly, the degree of complementarity is preferably less than 99%. For instance, where the guide sequence consists of 24 nucleotides, the degree of complementarity is more particularly about 96% or less. In particular embodiments, the guide sequence is designed to have a stretch of two or more adjacent mismatching nucleotides, such that the degree of complementarity over the entire guide sequence is further reduced. For instance, where the guide sequence consists of 24 nucleotides, the degree of complementarity is more particularly about 96% or less, more particularly, about 92% or less, more particularly about 88% or less, more particularly about 84% or less, more particularly about 80% or less, more particularly about 76% or less, more particularly about 72% or less, depending on whether the stretch of two or more mismatching nucleotides encompasses 2, 3, 4, 5, 6 or 7 nucleotides, etc. In some embodiments, aside from the stretch of one or more mismatching nucleotides, the degree of complementarity, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows- Wheeler Transform (e.g., the Burrows Wheeler Aligner), Clustal W, Clustal X, Clustal Omega, BLAT, Novoalign (Novocraft Technologies; available at www.novocraft.com), ELAND (Illumina, San Diego, CA), SOAP (available at soap.genomics.org.cn), and Maq (available at maq.sourceforge.net). The ability of a guide sequence (within a nucleic acid-targeting guide RNA) to direct sequence-specific binding of a programmable DNA binding unit to a selected binding site may be assessed by any suitable assay. In some embodiments, the guide sequence is an RNA sequence of between 10 to 50 nt in length, but more particularly of about 20-30 nt advantageously about 20 nt, 23-25 nt or 24 nt. The guide sequence can be selected so as to ensure that it hybridizes to the selected binding site.

### Dead Guide Sequences

The programmable DNA binding unit can comprise a CRISPR associated protein (CAS protein) and a guide RNA (gRNA) capable of specifically binding to the binding site of the target dsDNA. In some embodiments, guide sequences are modified in a manner which allows for formation of the CRISPR Cas complex and successful binding to the binding site, while at the same time, not allowing for successful nuclease activity. Such modified guide sequences are referred to as "dead guides" or "dead guide sequences". These dead guides or dead guide sequences can be thought of as catalytically inactive or conformationally inactive with regard to nuclease activity. The programmable DNA binding unit can comprise a functional Cas protein and guide RNA (gRNA) or crRNA wherein the gRNA or crRNA comprises a dead guide sequence whereby the gRNA is capable of hybridizing to a selected binding site such that the Cas protein is directed to a selected binding site without detectable cleavage activity of a non-mutant Cas protein. The ability of a dead guide sequence to direct sequence-specific binding of a CRISPR complex to an binding site may be assessed by any suitable assay. Dead guide sequences can be typically shorter than respective guide sequences which result in active cleavage. In particular embodiments, dead guides are 5%, 10%, 20%, 30%, 40%, 50%, shorter than respective guides directed to the same.

### Protein Components

The programmable DNA binding unit can comprise a protein component capable of specifically binding to the binding site on the target dsDNA. The protein component can comprise an endonuclease-deficient zinc finger nuclease (ZFN), an endonuclease-deficient transcription activator-like effector nuclease (TALEN), Argonaute protein, an endonuclease-deficient meganuclease, a recombinase, or a combination thereof. In some embodiments, the programmable DNA binding unit does not have a nuclease domain. In some embodiments, the programmable DNA binding unit has a nuclease domain that has been rendered catalytically inactive via one or more mutations. Catalytically inactivating mutations and means of assessing the nuclease activity of said mutants are known to those of skill in the art.

### Transcription Activator-like Effectors (TALEs)

The programmable DNA binding unit can comprise an endonuclease-deficient transcription activator-like effector nuclease (TALEN), a functional fragment thereof, or a variant thereof. Transcription activator-like effectors (TALEs) can be engineered to bind practically any desired DNA sequence. Exemplary methods of targeting using the TALEN systems can be found for example in Cermak T. Doyle EL. Christian M. Wang L. Zhang Y. Schmidt C, et al. Efficient design and assembly of custom TALEN and other TAL effector-based constructs for DNA targeting. Nucleic Acids Res. 2011;39:e82; Zhang F. Cong L. Lodato S. Kosuri S. Church GM. Arlotta P Efficient construction of sequence-specific TAL effectors for modulating mammalian transcription. Nat Biotechnol. 2011;29:149-153 and US Patent Nos. 8,450,471, 8,440,431 and 8,440,432.

The programmable DNA binding unit can comprise a TALE polypeptide. TALEs are transcription factors from the plant pathogen Xanthomonas that may be readily engineered to bind new DNA targets. In some embodiments provided herein, TALEs are not linked to the catalytic domain of an endonuclease (e.g., Fokl). In some embodiments provided herein, the programmable DNA binding unit can comprises a TALEN wherein the endonuclease domain is catalytically inactive. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. As used herein, the term "polypeptide monomers", or "TALE monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. The TALE monomers have a nucleotide binding affinity that is determined by the identity of the amino acids in its RVD. For example, polypeptide monomers with an RVD of NI preferentially bind to adenine (A), polypeptide monomers with an RVD of NG preferentially bind to thymine (T), polypeptide monomers with an RVD of HD preferentially bind to cytosine (C) and polypeptide monomers with an RVD of NN preferentially bind to both adenine (A) and guanine (G). In some embodiments, polypeptide monomers with an RVD of IG preferentially bind to T. Thus, the number and order of the polypeptide monomer repeats in the nucleic acid binding domain of a TALE determines its nucleic acid target specificity. In still further embodiments provided herein, polypeptide monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29: 149-153 (2011). The programmable DNA binding unit can comprise polypeptide monomer repeats that are designed to target specific nucleic acid sequences.

As described in Zhang et al., Nature Biotechnology 29:149-153 (2011), TALE polypeptide binding efficiency may be increased by including amino acid sequences from the "capping regions" that are directly N-terminal or C-terminal of the DNA binding region of naturally occurring TALEs into the engineered TALEs at positions N-terminal or C-terminal of the engineered TALE DNA binding region. Thus, in certain embodiments, the TALE polypeptides described herein further comprise an N-terminal capping region and/or a C- terminal capping region.

As used herein the predetermined "N-terminus" to "C terminus" orientation of the N-terminal capping region, the DNA binding domain comprising the repeat TALE monomers and the C-terminal capping region provide structural basis for the organization of different domains in the TALEs or polypeptides provided herein.

The entire N-terminal and/or C-terminal capping regions are not necessary to enhance the binding activity of the DNA binding region. Therefore, in certain embodiments, fragments of the N-terminal and/or C-terminal capping regions are included in the TALE polypeptides described herein.

In certain embodiments, the TALE polypeptides described herein contain a N-terminal capping region fragment that included at least 10, 20, 30, 40, 50, 54, 60, 70, 80, 87, 90, 94, 100, 102, 110, 117, 120, 130, 140, 147, 150, 160, 170, 180, 190, 200, 210, 220, 230, 240, 250, 260 or 270 amino acids of an N-terminal capping region. In certain embodiments, the N-terminal capping region fragment amino acids are of the C-terminus (the DNA-binding region proximal end) of an N-terminal capping region. As described in Zhang et al., Nature Biotechnology 29:149-153 (2011), N-terminal capping region fragments that include the C- terminal 240 amino acids enhance binding activity equal to the full length capping region, while fragments that include the C-terminal 147 amino acids retain greater than 80% of the efficacy of the full length capping region, and fragments that include the C-terminal 117 amino acids retain greater than 50% of the activity of the full-length capping region.

In some embodiments, the TALE polypeptides described herein contain a C-terminal capping region fragment that included at least 6, 10, 20, 30, 37, 40, 50, 60, 68, 70, 80, 90, 100, 110, 120, 127, 130, 140, 150, 155, 160, 170, 180 amino acids of a C-terminal capping region. In certain embodiments, the C-terminal capping region fragment amino acids are of the N-terminus (the DNA-binding region proximal end) of a C-terminal capping region. As described in Zhang et al., Nature Biotechnology 29: 149-153 (2011), C-terminal capping region fragments that include the C-terminal 68 amino acids enhance binding activity equal to the full length capping region, while fragments that include the C-terminal 20 amino acids retain greater than 50% of the efficacy of the full length capping region.

### Zinc-finger (ZF) Proteins

The programmable DNA binding unit can comprise a Zn-finger (ZF) nuclease, a functional fragment thereof, or a variant thereof. The programmable DNA binding unit can comprise an endonuclease-deficient ZF nuclease, a functional fragment thereof, or a variant thereof, wherein the domain of an endonuclease (e.g., Fokl) is catalytically inactive or absent. The programmable DNA binding unit can comprise a ZF protein (ZFP). The ZFP can engineered to bind to a target site of choice. See, for example, Beerli et al. (2002) Nature Biotechnol. 20: 135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct Biol. 10:411-416; U.S. Pat. Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061. ZFPs can comprise arrays of ZF modules to target desired DNA-binding sites. Each finger module in a ZF array can target three DNA bases. A customized array of individual zinc finger domains can be assembled into a ZFP.

### Meganucleases

The programmable DNA binding unit can be an endonuclease-deficient meganuclease, a functional fragment thereof, or a variant thereof. The DNA-binding domain of meganucleases may have a double-stranded DNA target sequence of 12 to 45 bp. In some embodiments, the meganuclease is either a dimeric enzyme, wherein each meganuclease domain is on a monomer, or a monomeric enzyme comprising the two domains on a single polypeptide. Not only wild-type meganucleases but also various meganuclease variants have been generated by protein engineering to cover a myriad of unique sequence combinations. In some embodiments, chimeric meganucleases with a recognition site composed of a half-site of meganuclease A and a half-site of protein B can also be used. Specific examples of such chimeric meganucleases comprise the protein domains of I-DmoI and I-CreI. Examples of meganucleases include homing endonucleases from the LAGLIDADG family. The "LAGLIDADG meganuclease" refers to a homing endonuclease from the LAGLIDADG family, as defined in Stoddard et al (Stoddard, 2005), or an engineered variant comprising a polypeptide sharing at least 80%, 85%, 90%, 95%, 97.5%, 99% or more identity or similarity with said natural homing endonuclease. Such engineered LAGLIDADG meganucleases can be derived from monomeric or dimeric meganucleases. When derived from dimeric meganucleases, such engineered LAGLIDADG meganucleases can be single-chain or dimeric endonucleases. Meganucleases may be targeted to specific sequences by modifying their recognition sequence using techniques well known to those skilled in the art. See, for example, Epinat et al., 2003, Nuc. Acid Res., 31(11):2952-62 and Stoddard, 2005, Quarterly Review of Biophysics, pp. 1-47.

The LAGLIDADG meganuclease can be I-SceI, I-ChuI, I-CreI, I-CsmI, PI-SceI, PI-TliI, PI-MtuI, I-CeuI, I-SceII, I-SceIII, HO, PI-CivI, PI-CtrI, PI-AaeI, PI-BsuI, PI-DhaI, PI-DraI, PI-MavI, PI-MchI, PI-MfuI, PI-MflI, PI-MgaI, PI-MgoI, PI-MinI, PI-MkaI, PI-MleI, PI-MmaI, PI-MshI, PI-MsmI, PI-MthI, PI-MtuI, PI-MxeI, PI-NpuI, PI-PfuI, PI-RmaI, PI-SpbI, PI-SspI, PI-FacI, PI-MjaI, PI-PhoI, PI-TagI, PI-Thyl, PI-TkoI, PI-TspI, or I-MsoI; or can be a functional mutant or variant thereof, whether homodimeric, heterodimeric or monomeric. In some embodiments, the LAGLIDADG meganuclease is a I-CreI derivative. In some embodiments, the LAGLIDADG meganuclease shares at least 80% similarity with the natural I-CreI LAGLIDADG meganuclease. In some embodiments, the LAGLIDADG meganuclease shares at least 80% similarity with residues 1-152 of the natural I-CreI LAGLIDADG meganuclease. In some embodiments, the LAGLIDADG meganuclease may consists of two monomers sharing at least 80% similarity with residues 1-152 of the natural I-CreI LAGLIDADG meganuclease linked together, with or without a linker peptide.

### Argonaute proteins

In some embodiments, the programmable DNA binding unit comprises a nuclease inactive Argonaute. In some embodiments, the programmable DNA binding unit comprises an Argonaute protein from Natronobacterium gregoryi (NgAgo), a functional fragment thereof, or a variant thereof. NgAgo is a ssDNA-guided endonuclease. NgAgo binds 5' phosphorylated ssDNA of roughly 24 nucleotides (gDNA) to guide it to its target site and will make DNA double-strand breaks at gDNA site. In some embodiments, the programmable DNA binding unit comprises a nuclease inactive NgAgo (dNgAgo). The characterization and use of NgAgo have been described in Gao et al, Nat Biotechnol. Epub 2016 May 2. PubMed PMID: 27136078; Swarts et al, Nature. 507(7491) (2014):258-61; and Swarts et al, Nucleic Acids Res. 43(10) (2015):5120-9. A NgAgo-based programmable DNA binding unit can comprise at least one guide DNA element or a nucleic acid comprising a nucleic acid sequence(s) encoding the guide DNA element, and achieves specific targeting or recognition of a binding site(s) via base-pairs directly with the DNA of the binding site(s). Prokaryotic homologs of Argonaute proteins are known and have been described, for example, in Makarova K., et al., "Prokaryotic homologs of Argonaute proteins are predicted to function as key components of a novel system of defense against mobile genetic elements", Biol. Direct. 2009 Aug. 25; 4:29. doi: 10.1186/1745-6150-4-29. In some embodiments, the programmable DNA binding unit is a *Marinitoga piezophila* Argunaute (MpAgo) protein, a functional fragment thereof, or a variant thereof.

### Recombinases

In some embodiments, the programmable DNA binding unit comprises a recombinase configured to bind binding site(s) on target dsDNA. Site-specific recombinases are well known in the art, and may be generally referred to as invertases, resolvases, or integrases. Non-limiting examples of site-specific recombinases include, but are not limited to: lambda integrase, Cre, Int, IHF, Xis, Flp, Fis, Hin, Gin, phiC31, Cin, Tn3 resolvase, TndX, XerC, XerD, TnpX, Hjc, Gin, SpCCEl, and ParA.

### Linkers

The transposome can be associated with the programmable DNA binding unit via a linker connecting the transposase and the dCAS protein. The linker can comprise a peptide linker, a chemical linker, or both. The transposase can be present in a fusion protein comprising the dCAS protein. The transposome can be associated with the programmable DNA binding unit via a linker connecting the transposase and the protein component. The peptide linker can comprise multiple glycine, serine, threonine, alanine, lysine, glutamine, or a combination thereof. The peptide linker can comprise a GS linker. The peptide linker can be a XTEN linker. The protein component can be present in a fusion protein comprising the transposase. The term "linker", as used herein, refers to a molecule which facilitates an interaction between molecules or parts of molecules. In some embodiments, a linker is a polypeptide linker. In some embodiments, a linker is a chemical linker. The term "peptide linker" or "polypeptide linker" as used herein means a peptide or polypeptide comprising two or more amino acids residues joined by peptide bonds. Such peptide or polypeptide linkers are well known in the art. Linkers can comprise naturally occurring and/or non-naturally occurring peptides or polypeptides. The linker can be associated with the C-terminus and/or the N-terminus of a transposase and/or a programmable DNA binding unit.

The linker may be a chemical linker or a peptide linker. Thus, embodiments relate to polypeptides conjugated to other molecules through a peptide bond and polypeptides conjugated to other molecules through chemical conjugation.

Peptide linkers with a degree of flexibility can be used. The peptide linkers may have virtually any amino acid sequence, bearing in mind that suitable peptide linkers will have a sequence that results in a generally flexible peptide. The use of small amino acids, such as glycine and alanine, are of use in creating a flexible peptide. The creation of such sequences is routine to those of skill in the art.

Suitable linkers can be readily selected and can be of any suitable length, such as from 1 amino acid (e.g., Gly) to 50 amino acids, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a number or a range between any two of these values, amino acids.

Preferred peptide linker sequences adopt a flexible extended conformation and do not exhibit a propensity for developing an ordered secondary structure. In certain embodiments, the linker can be a chemical moiety which can be monomeric, dimeric, multimeric or polymeric. Preferably, the linker comprises amino acids. Typical amino acids in flexible linkers include Gly, Asn and Ser. Accordingly, in particular embodiments, the linker comprises a combination of one or more of Gly, Asn and Ser amino acids. Other near neutral amino acids, such as Thr and Ala, also may be used in the linker sequence. Example flexible linkers include glycine polymers (G)n (SEQ ID NO: 32), glycine- serine polymers (including, for example, (GS)n (SEQ ID NO: 33), (GSGGS)n (SEQ ID NO: 34), (G4S)n (SEQ ID NO: 35) and (GGGS)n (SEQ ID NO: 36), where n is an integer of at least one. In some embodiments, n is at least, at most, or exactly 1, 2, 3, 4, 5, 6, 7, 8, 9, or 10 (or any derivable range therein). Glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains. Exemplary spacers can comprise amino acid sequences including, but not limited to, GGSG (SEQ ID NO: 37), GGSGG (SEQ ID NO: 38), GSGSG (SEQ ID NO: 39), GSGGG (SEQ ID NO:40), GGGSG (SEQ ID NO: 41), GSSSG (SEQ ID NO: 42), and the like. Other near neutral amino acids, such as Thr and Ala, may also be used in the linker sequence. The length of the linker sequence may vary without significantly affecting the function or activity of the fusion protein (see, e.g., U.S. Pat. No. 6,087,329). In some embodiments, the linker may be at least, at most, or exactly 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, or 100 amino acid residues (or any range derivable therein).

In some embodiments, the polypeptide linker is an XTEN linker. In some embodiments, the linker is an XTEN linker or a variation of an XTEN linker such as SGSETPGTSESA (SEQ ID NO: 43), SGSETPGTSESATPES (SEQ ID NO: 44), or SGSETPGTSESATPEGGSGGS (SEQ ID NO: 45). XTEN linkers are described in, for example Schellenberger et al. (2009), Nature Biotechnology 27: 1186-1190.

Suitable linkers for use in the methods provided herein are well known to those of skill in the art and include, but are not limited to, straight or branched-chain carbon linkers, heterocyclic carbon linkers, or peptide linkers. However, as used herein the linker may also be a covalent bond (carbon-carbon bond or carbon-heteroatom bond). In particular embodiments, the linker is used to separate the transposome and the programmable DNA binding unit by a distance sufficient to ensure that each protein retains its required functional property.

Linkers may be used to fuse two protein partners to form a fusion protein. A "linker" can be a chemical group or a molecule linking two molecules or moieties, e.g., two domains of a fusion protein. Typically, the linker is positioned between (flanked by) two groups, molecules, domains, or other moieties and connected to each one via a covalent bond, thus connecting the two. In some embodiments, the linker is an amino acid or a plurality of amino acids (e.g., a peptide or protein). In some embodiments, the linker is an organic molecule, group, polymer (e.g. a non-natural polymer, non-peptidic polymer), or chemical moiety. In some embodiments, linkers comprise a direct bond or an atom such as, e.g., an oxygen (O) or sulfur (S), a unit such as -NR- wherein R is hydrogen or alkyl, -C(O)-, -C(O)O-, -C(O)NH-, SO, SO₂, -SO₂NH- or a chain of atoms, such as substituted or unsubstituted alkyl, substituted or unsubstituted alkenyl, substituted or unsubstituted alkynyl, arylalkyl, heteroarylalkyl. In some embodiments, one or more methylenes in the chain of atoms can be replaced with one or more of O, S, S(O), SO₂, -SO₂NH-, -NR-, -NR₂, -C(O)-, - C(O)O-, -C(O)NH-, a cleavable linking group, substituted or unsubstituted aryl, substituted or unsubstituted heteroaryl, and substituted or unsubstituted heterocyclic. Examples of linkers may also include chemical moieties and conjugating agents, such as sulfo-succinimidyl derivatives (sulfo-SMCC, sulfo-SMPB), disuccinimidyl suberate (DSS), disuccinimidyl glutarate (DSG) and disuccinimidyl tartrate (DST). Examples of linkers further comprise a linear carbon chain, such as CN (where N=1-100 carbon atoms). In some embodiments, the linker can be a dipeptide linker, such as a valine-citrulline (val-cit), a phenylalanine-lysine (phe-lys) linker, or maleimidocapronic-valine-citruline-p-aminobenzyloxy carbonyl (vc) linker. In some embodiments, the linker is sulfosuccinimidyl-4-[N-maleimidomethyl]cyclohexane-1-carboxylate (smcc). Sulfo-smcc conjugation occurs via a maleimide group which reacts with sulfhydryls (thiols, -SH), while its sulfo-NHS ester is reactive toward primary amines (as found in lysine and the protein or peptide N-terminus). Further, the linker may be maleimidocaproyl (me). In some embodiments, the covalent linkage may be achieved through the use of Traut's reagent.

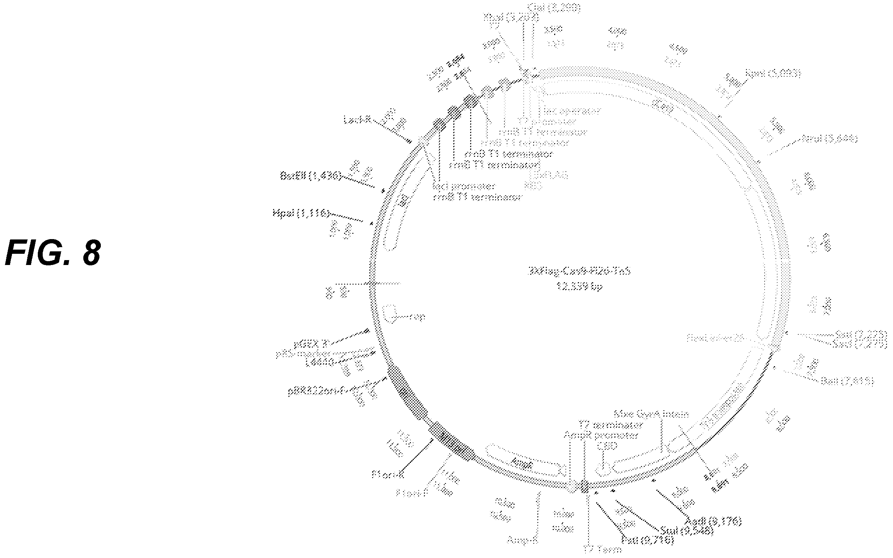

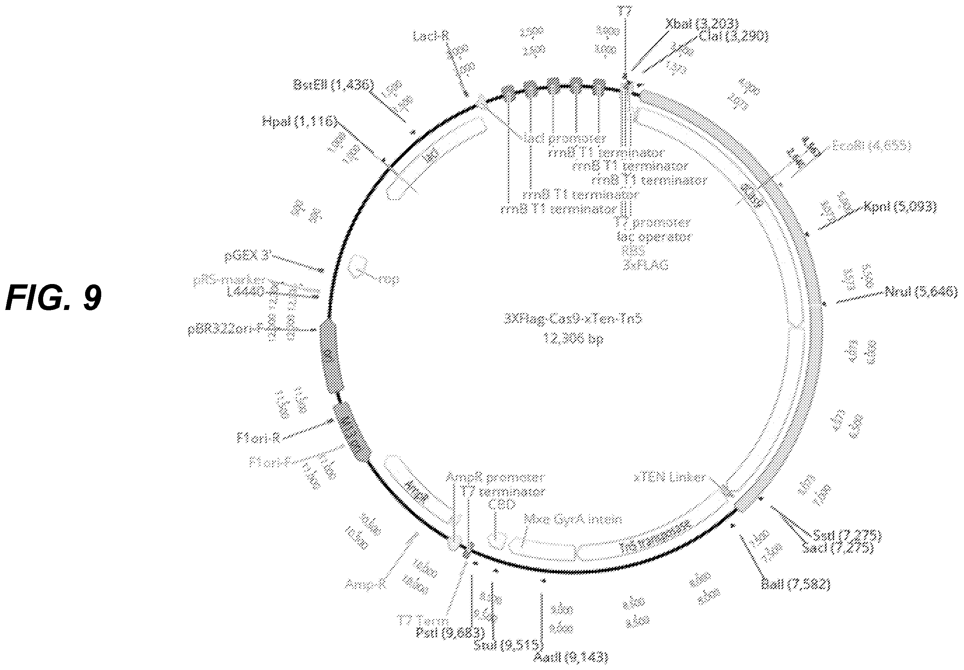

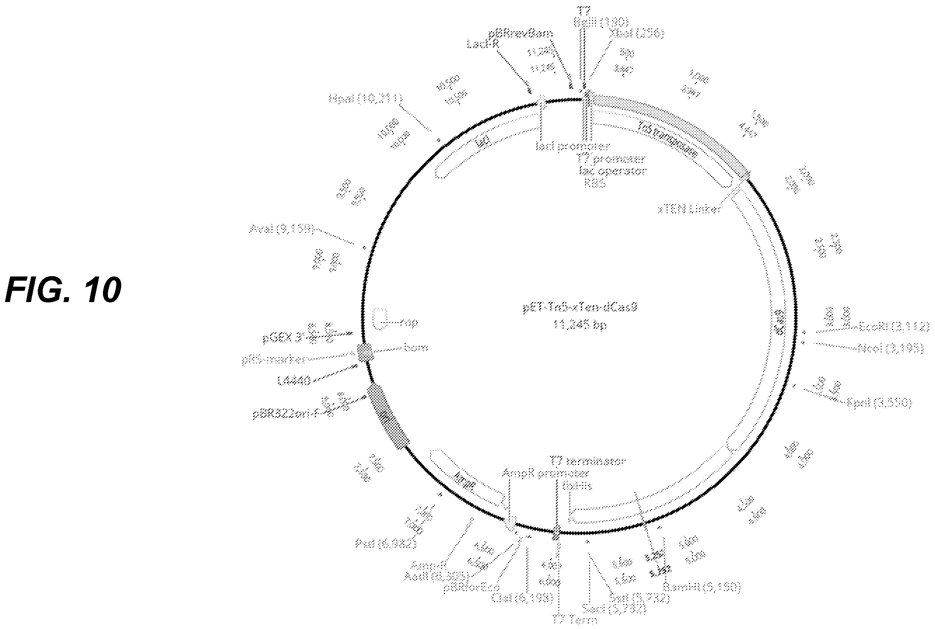

FIGS. 8-10 depict non-limiting exemplary schematics of plasmid constructs 3XFlag-Cas9-Fl26-Tn5 (SEQ ID NO: 1), 3XFlag-Cas9-xTen-Tn5 (SEQ ID NO: 2), and pET-Tn5-xTen-dCas9 (SEQ ID NO: 3), respectively, for use in generating protein complexes provided herein. A protein complex, linker, programmable DNA binding unit, and/or transposase disclosed herein can be encoded by a nucleotide sequence at least about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between any two of these values, identical to the protein complex, linker, programmable DNA binding unit, and/or transposase encoded in SEQ ID NOS: 1-3.

### Amplification

The method can comprise: amplifying the plurality of dsDNA fragments with primers capable of binding to the adaptors at the termini of the dsDNA fragments. The amplification can generate nucleic acid amplification products. The nucleic acid amplification products can comprise a library (e.g., a sequencing library). Each of the primers can be about 5-80 (e.g., about 5, 6, 7, 8, 9, 10, 11, 12, 13, 14, 15, 16, 17, 18, 19, 20, 25, 30, 35, 40, 45, 50, 60, 70, 80, or a number or a range between any two of these values, nucleotides in length) nucleotides in length. Amplifying the plurality of dsDNA fragments with the primer can be carried out using polymerase chain reaction (PCR). The PCR can be loop-mediated isothermal Amplification (LAMP), helicase-dependent Amplification (HDA), recombinase polymerase amplification (RPA), strand displacement amplification (SDA), nucleic acid sequence-based amplification (NASBA), transcription mediated amplification (TMA), nicking enzyme amplification reaction (NEAR), rolling circle amplification (RCA), multiple displacement amplification (MDA), Ramification (RAM), circular helicase dependent amplification (cHDA), single primer isothermal amplification (SPIA), signal mediated amplification of RNA technology (SMART), self-sustained sequence replication (3SR), genome exponential amplification reaction (GEAR), or isothermal multiple displacement amplification (IMDA). The PCR can be real-time PCR or quantitative real-time PCR (QRT-PCR).

As used herein, nucleic acid amplification can refer to any known procedure for obtaining multiple copies of a target nucleic acid sequence or its complement or fragments thereof, using sequence-specific methods. Examples of known amplification methods include, but are not limited to, polymerase chain reaction (PCR), ligase chain reaction (LCR), loop-mediated isothermal amplification (LAMP), strand displacement amplification (SDA) (e.g., multiple displacement amplification (MDA)), replicase-mediated amplification, immuno-amplification, nucleic acid sequence based amplification (NASBA), self-sustained sequence replication (3SR), rolling circle amplification, and transcription-mediated amplification (TMA). In some embodiments, two or more of the aforementioned nucleic acid amplification methods can be performed, for example sequentially.

For example, LCR amplification uses at least four separate oligonucleotides to amplify a target and its complementary strand by using multiple cycles of hybridization, ligation, and denaturation. SDA amplifies by using a primer that contains a recognition site for a restriction endonuclease which nicks one strand of a hemimodified DNA duplex that includes the target sequence, followed by amplification in a series of primer extension and strand displacement steps.

PCR is a method well-known in the art for amplification of nucleic acids. PCR involves amplification of a target sequence using two or more extendable sequence-specific oligonucleotide primers that flank the target sequence. The nucleic acid containing the target sequence of interest is subjected to a program of multiple rounds of thermal cycling (denaturation, annealing and extension) in the presence of the primers, a thermostable DNA polymerase (e.g., Taq polymerase) and various dNTPs, resulting in amplification of the target sequence. PCR uses multiple rounds of primer extension reactions in which complementary strands of a defined region of a DNA molecule are simultaneously synthesized by a thermostable DNA polymerase. At the end of each cycle, each newly synthesized DNA molecule acts as a template for the next cycle. During repeated rounds of these reactions, the number of newly synthesized DNA strands increases exponentially such that after 20 to 30 reaction cycles, the initial template DNA will have been replicated several thousand-fold or million-fold.

PCR can generate double-stranded amplification products suitable for post-amplification processing. If desired, amplification products can be detected by visualization with agarose gel electrophoresis, by an enzyme immunoassay format using probe-based colorimetric detection, by fluorescence emission technology, or by other detection means known to one of skill in the art.

Examples of PCR methods include, but are not limited to, Real-Time PCR, End-Point PCR, Amplified fragment length polymorphism PCR (AFLP-PCR), Alu-PCR, Asymmetric PCR, Colony PCR DD-PCR, Degenerate PCR, Hot-start PCR, In situ PCR, Inverse PCR Long-PCR, Multiplex PCR, Nested PCR, PCR-ELISA, PCR-RFLP, PCR-single strand conformation polymorphism (PCR-SSCP), quantitative competitive PCR (QC-PCR), rapid amplification of cDNA ends-PCR (RACE-PCR), Random Amplification of Polymorphic DNA-PCR (RAPD-PCR), Real-Time PCR, Repetitive extragenic palindromic-PCR (Rep-PCR), reverse transcriptase PCR (RT-PCR), TAIL-PCR, Touchdown PCR and Vectorette PCR.

Real-time PCR, also called quantitative real time polymerase chain reaction (QRT-PCR), can be used to simultaneously quantify and amplify a specific part of a given nucleic acid molecule. It can be used to determine whether a specific sequence is present in the sample; and if it is present, the number of copies of the sequence that are present. The term "real-time" can refer to periodic monitoring during PCR. Certain systems such as the ABI 7700 and 7900HT Sequence Detection Systems (Applied Biosystems, Foster City, CA) conduct monitoring during each thermal cycle at a pre-determined or user-defined point. Real-time analysis of PCR with fluorescence resonance energy transfer (FRET) probes measures fluorescent dye signal changes from cycle-to-cycle, preferably minus any internal control signals. The real-time procedure follows the general pattern of PCR, but the nucleic acid is quantified after each round of amplification. Two examples of method of quantification are the use of fluorescent dyes (e.g., SYBRGreen) that intercalate into double-stranded DNA, and modified DNA oligonucleotide probes that fluoresce when hybridized with a complementary DNA. Intercalating agents have a relatively low fluorescence when unbound, and a relatively high fluorescence upon binding to double-stranded nucleic acids. As such, intercalating agents can be used to monitor the accumulation of double strained nucleic acids during a nucleic acid amplification reaction. Examples of such non-specific dyes useful in the embodiments disclosed herein include intercalating agents such as SYBR Green I (Molecular Probes), propidium iodide, ethidium bromide, and the like.

### Labels

Method described herein can comprise: labeling one or both ends of one or more of the plurality of dsDNA fragments (e.g., with a detectable label). The method can comprise: labeling the two ends of one or more of the plurality of dsDNA fragments differently. The labeling can comprise labeling with detectable labels (e.g., anionic labels, cationic labels, neutral labels, electrochemical labels, protein labels, fluorescent labels, magnetic labels, or a combination thereof). The method can comprise: enriching the labeled dsDNA fragments, capturing the labeled dsDNA fragments, isolating the labeled dsDNA fragments, and/or visualizing the labeled dsDNA fragments. The method can comprise monitoring (e.g., chemical monitoring) of the detectable label(s).

In some embodiments, the detectable moiety (e.g., detectable label) comprises an optical moiety, a luminescent moiety, an electrochemically active moiety, a nanoparticle, or a combination thereof. In some embodiments, the luminescent moiety comprises a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. In some embodiments, the nanoparticle comprises a quantum dot. In some embodiments, the methods comprise performing a reaction to convert the detectable moiety precursor into the detectable moiety. In some embodiments, performing a reaction to convert the detectable moiety precursor into the detectable moiety comprises contacting the detectable moiety precursor with a substrate. In some such embodiments, contacting the detectable moiety precursor with a substrate yields a detectable byproduct of a reaction between the two molecules.

### Detection and Quantification of Amplification Products

Some of the methods provided herein comprise amplifying the plurality of dsDNA fragments to generate nucleic acid amplification products. The methods described herein may further comprise detecting and/or quantifying a nucleic acid amplification product, or a product thereof. An amplification product, or a product thereof, may be detected and/or quantified by any suitable detection and/or quantification method including, for example, any detection method or quantification method described herein. Non-limiting examples of detection and/or quantification methods include molecular beacon (e.g., real-time, endpoint), lateral flow, fluorescence resonance energy transfer (FRET), fluorescence polarization (FP), surface capture, 5' to 3' exonuclease hydrolysis probes (e.g., TAQMAN), intercalating/binding dyes, absorbance methods (e.g., colorimetric, turbidity), electrophoresis (e.g., gel electrophoresis, capillary electrophoresis), mass spectrometry, nucleic acid sequencing, digital amplification, a primer extension method (e.g., iPLEX^{™}), Molecular Inversion Probe (MIP) technology from Affymetrix, restriction fragment length polymorphism (RFLP analysis), allele specific oligonucleotide (ASO) analysis, methylation-specific PCR (MSPCR), pyrosequencing analysis, acycloprime analysis, Reverse dot blot, GeneChip microarrays, Dynamic allele-specific hybridization (DASH), Peptide nucleic acid (PNA) and locked nucleic acids (LNA) probes, AlphaScreen, SNPstream, genetic bit analysis (GBA), Multiplex minisequencing, SNaPshot, GOOD assay, Microarray miniseq, arrayed primer extension (APEX), Microarray primer extension, Tag arrays, Coded microspheres, Template-directed incorporation (TDI), colorimetric oligonucleotide ligation assay (OLA), sequence-coded OLA, microarray ligation, ligase chain reaction, padlock probes, invader assay, hybridization using at least one probe, hybridization using at least one fluorescently labeled probe, cloning and sequencing, the use of hybridization probes and quantitative real time polymerase chain reaction (QRT-PCR), nanopore sequencing, chips and combinations thereof. Detecting a nucleic acid amplification product can comprise use of a real-time detection method (i.e., product is detected and/or continuously monitored during an amplification process), use of an endpoint detection method (i.e., product is detected after completing or stopping an amplification process), or both. Nucleic acid detection methods may also employ the use of labeled nucleotides incorporated directly into a target sequence or into probes containing complementary sequences to a target. Such labels may be radioactive and/or fluorescent in nature and can be resolved in any of the manners discussed herein. In some embodiments, quantification of a nucleic acid amplification product is achieved using one or more detection methods described below. The detection method can be used in conjunction with a measurement of signal intensity, and/or generation of (or reference to) a standard curve and/or look-up table for quantification of a nucleic acid amplification product.

Detecting a nucleic acid amplification product can comprise use of molecular beacon technology. The term molecular beacon generally refers to a detectable molecule, where the detectable property of the molecule is detectable under certain conditions, thereby enabling the molecule to function as a specific and informative signal. Non-limiting examples of detectable properties include optical properties (e.g., fluorescence), electrical properties, magnetic properties, chemical properties and time or speed through an opening of known size. Molecular beacons for detecting nucleic acid molecules may be, for example, hair-pin shaped oligonucleotides containing a fluorophore on one end and a quenching dye on the opposite end. The loop of the hair-pin may contain a probe sequence that is complementary to a target sequence and the stem is formed by annealing of complementary arm sequences located on either side of the probe sequence. A fluorophore and a quenching molecule can be covalently linked at opposite ends of each arm. Under conditions that prevent the oligonucleotides from hybridizing to its complementary target or when the molecular beacon is free in solution, the fluorescent and quenching molecules are proximal to one another preventing FRET. When the molecular beacon encounters a target molecule (e.g., a nucleic acid amplification product), hybridization can occur, and the loop structure is converted to a stable more rigid conformation causing separation of the fluorophore and quencher molecules leading to fluorescence. Due to the specificity of the probe, the generation of fluorescence generally is exclusively due to the synthesis of the intended amplified product. In some embodiments, a molecular beacon probe sequence hybridizes to a sequence in an amplification product that is identical to or complementary to a sequence in a target nucleic acid. In some embodiments, a molecular beacon probe sequence hybridizes to a sequence in an amplification product that is not identical to or complementary to a sequence in a target nucleic acid (e.g., hybridizes to a sequence added to an amplification product by way of a tailed amplification primer or ligation). Molecular beacons can be synthesized with different colored fluorophores and different target sequences, enabling simultaneous detection of several products in the same reaction (e.g., in a multiplex reaction). For quantitative amplification processes, molecular beacons can specifically bind to the amplified target following each cycle of amplification, and because non-hybridized molecular beacons are dark, it is not necessary to isolate the probe-target hybrids to quantitatively determine the amount of amplified product. The resulting signal is proportional to the amount of amplified product. Detection using molecular beacons can be done in real time or as an end-point detection method.

Detecting a nucleic acid amplification product can comprise use of lateral flow, Lateral flow device generally include a solid phase fluid permeable flow path through which fluid flows through by capillary force. Example devices include, but are not limited to, dipstick assays and thin layer chromatographic plates with various appropriate coatings. Immobilized on the flow path are various binding reagents for the sample, binding partners or conjugates involving binding partners for the sample and signal producing systems. Detection can be achieved in several manners including, for example, enzymatic detection, nanoparticle detection, colorimetric detection, and fluorescence detection.

Detecting a nucleic acid amplification product can comprise use of FRET which is an energy transfer mechanism between two chromophores: a donor and an acceptor molecule. Briefly, a donor fluorophore molecule is excited at a specific excitation wavelength. The subsequent emission from the donor molecule as it returns to its ground state may transfer excitation energy to the acceptor molecule through a long range dipole-dipole interaction. The emission intensity of the acceptor molecule can be monitored and is a function of the distance between the donor and the acceptor, the overlap of the donor emission spectrum and the acceptor absorption spectrum and the orientation of the donor emission dipole moment and the acceptor absorption dipole moment. FRET can be useful for quantifying molecular dynamics, for example, in DNA-DNA interactions as described for molecular beacons. For monitoring the production of a specific product, a probe can be labeled with a donor molecule on one end and an acceptor molecule on the other. Probe-target hybridization brings a change in the distance or orientation of the donor and acceptor and FRET change is observed.

Detecting a nucleic acid amplification product comprises use of FP which is generally based on the principle that a fluorescently labeled compound when excited by linearly polarized light will emit fluorescence having a degree of polarization inversely related to its rate of rotation. Therefore, when a molecule such as a tracer-nucleic acid conjugate, for example, having a fluorescent label is excited with linearly polarized light, the emitted light remains highly polarized because the fluorophore is constrained from rotating between the time light is absorbed and emitted. When a free tracer compound (i.e., unbound to a nucleic acid) is excited by linearly polarized light, its rotation is much faster than the corresponding tracer-nucleic acid conjugate and the molecules are more randomly oriented, therefore, the emitted light is depolarized. Thus, fluorescence polarization provides a quantitative means for measuring the amount of tracer-nucleic acid conjugate produced in an amplification reaction.

Detecting a nucleic acid amplification product comprises use of surface capture which can be accomplished by the immobilization of specific oligonucleotides to a surface producing a biosensor that is both highly sensitive and selective.

Detecting a nucleic acid amplification product can comprise use of 5' to 3' exonuclease hydrolysis probes (e.g., TAQMAN). TAQMAN probes, for example, are hydrolysis probes that can increase the specificity of a quantitative amplification method (e.g., quantitative PCR). The TAQMAN probe principle relies on 1) the 5' to 3' exonuclease activity of Taq polymerase to cleave a dual-labeled probe during hybridization to a complementary target sequence and 2) fluorophore-based detection. A resulting fluorescence signal permits quantitative measurements of the accumulation of amplification product during the exponential stages of amplification.

Detecting a nucleic acid amplification product comprises use of intercalating and/or binding dyes, for example dyes capable of specifically staining nucleic acid. For example, intercalating dyes exhibit enhanced fluorescence upon binding to DNA or RNA. Non-limiting examples of dyes include SYTO^{®} 82, acridine orange, ethidium bromide, Hoechst dyes, PicoGreen^{®}, propidium iodide, SYBR^{®} I (an asymmetrical cyanine dye), SYBR^{®} II, TOTO (a thiaxole orange dimer) and YOYO (an oxazole yellow dimer).

Detecting a nucleic acid amplification product comprises use of absorbance methods (e.g., colorimetric, turbidity). Detection and/or quantitation of nucleic acid can be achieved by directly converting absorbance (e.g., UV absorbance measurements at 260 nm) to concentration, for example. Direct measurement of nucleic acid can be converted to concentration using the Beer Lambert law which relates absorbance to concentration using the path length of the measurement and an extinction coefficient.

Detecting a nucleic acid amplification product can comprise use of electrophoresis (e.g., gel electrophoresis, capillary electrophoresis), mass spectrometry, nucleic acid sequencing, digital amplification (e.g., digital PCR), or any combination thereof.

### Genetic Signatures of Interest

The plurality of target dsDNA can comprise a genetic signature of interest (e.g., biomarker signature). The genetic signature of interest can comprise one or more mutations of interest (e.g., biomarkers). The one or more mutations of interest can comprise point mutation, inversion, deletion, insertion, translocation, duplication, copy number variation, or a combination thereof. The one or more mutations of interest can comprise nucleotide substitution, deletion, insertion, or a combination thereof. The genetic signature of interest can be indicative of antibiotic resistance or antibiotic susceptibility of the organism from which the target dsDNA is derived. The genetic signature of interest can be indicative of cancer status of the organism from which the target dsDNA is derived. The genetic signature of interest can be indicative of the status of a genetic disease of the organism from which the target dsDNA is derived. The genetic disease can be single-gene disorder. The genetic disease can be cystic fibrosis, Huntington's disease, sickle cell anemia, hemophilia, Duchenne muscular dystrophy, Thalassemia, Fragile X syndrome, familial hypercholesterolemia, polycystic kidney disease, neurofibromatosis type I, hereditary spherocytosis, Marfan syndrome, Tay-Sachs disease, phenylketonuria, mucopolysaccharidoses, lysosomal acid lipase deficiency, glycogen storage diseases, galactosemia, or hemochromatosis. Genetic signatures of interest (e.g., biomarker signatures) can be detected with the methods and compositions provided herein. Diagnostic assessments can be performed using the methods and compositions provided herein.

Diagnostic assessments, as described herein, are made, either alone or in combination with other evaluations or factors, based on a biomarker signature (e.g., a genetic signature of interest). Provided herein are compositions and methods for assessing the risk of developing a disease or condition, prognosing said disease, diagnosing said disease or condition, monitoring said disease or condition progression or regression, assessing the efficacy of a treatment, or identifying a compound capable of ameliorating or treating said disease or condition, based on a biomarker signature (e.g., a genetic signature of interest).

### Diseases and Conditions

The methods provided herein can be applied to various diseases or conditions based on a biomarker signature (e.g., a genetic signature of interest) associated therewith. Exemplary diseases or conditions with a genetic signature of interest subject to the disclosed compositions and methods include a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition.

### Samples

The sample can comprise eukaryotic DNA, bacterial DNA, viral DNA, fungal DNA, protozoa DNA, or a combination thereof. The plurality of target dsDNA can comprise genomic DNA, mitochondrial DNA, plasmid DNA, or a combination thereof. The sample can be, or can be derived from, a biological sample, a clinical sample, an environmental sample, or a combination thereof. The plurality of target dsDNA can comprise DNA from at least 2 different organisms. The plurality of target dsDNA can comprise DNA from at least 2 different genes. The method can comprise: generating the plurality of target dsDNA from a plurality of target RNA with a reverse transcriptase. The plurality of target dsDNA can comprise target dsDNA generated from target RNA with a reverse transcriptase. The sample nucleic acids can comprise eukaryotic DNA, bacterial DNA, viral DNA, fungal DNA, protozoa DNA, or a combination thereof. The target dsDNA can be genomic DNA, mitochondria DNA, plasmid DNA, or a combination thereof. The sample nucleic acids can be from a biological sample, a clinical sample, an environmental sample, or a combination thereof. The biological sample can comprise stool, sputum, peripheral blood, plasma, serum, lymph nodes, respiratory tissue, exudates, bodily fluid, or a combination thereof.

Nucleic acid utilized in methods described herein may be obtained from any suitable biological specimen or sample, and often is isolated from a sample obtained from a subject. A subject can be any living or non-living organism, including but not limited to a human, a non-human animal, a plant, a bacterium, a fungus, a virus, or a protist. Any human or non-human animal can be selected, including but not limited to mammal, reptile, avian, amphibian, fish, ungulate, ruminant, bovine (e.g., cattle), equine (e.g., horse), caprine and ovine (e.g., sheep, goat), swine (e.g., pig), camelid (e.g., camel, llama, alpaca), monkey, ape (e.g., gorilla, chimpanzee), ursid (e.g., bear), poultry, dog, cat, mouse, rat, fish, dolphin, whale and shark. A subject may be a male or female, and a subject may be any age (e.g., an embryo, a fetus, infant, child, adult).

A sample or test sample can be any specimen that is isolated or obtained from a subject or part thereof. Non-limiting examples of specimens include fluid or tissue from a subject, including, without limitation, blood or a blood product (e.g., serum, plasma, or the like), umbilical cord blood, bone marrow, chorionic villi, amniotic fluid, cerebrospinal fluid, spinal fluid, lavage fluid (e.g., bronchoalveolar, gastric, peritoneal, ductal, ear, arthroscopic), biopsy sample, celocentesis sample, cells (e.g., blood cells) or parts thereof (e.g., mitochondrial, nucleus, extracts, or the like), washings of female reproductive tract, urine, feces, sputum, saliva, nasal mucous, prostate fluid, lavage, semen, lymphatic fluid, bile, tears, sweat, breast milk, breast fluid, hard tissues (e.g., liver, spleen, kidney, lung, or ovary), the like or combinations thereof. The term blood encompasses whole blood, blood product or any fraction of blood, such as serum, plasma, buffy coat, or the like as conventionally defined. Blood plasma refers to the fraction of whole blood resulting from centrifugation of blood treated with anticoagulants. Blood serum refers to the watery portion of fluid remaining after a blood sample has coagulated. Fluid or tissue samples often are collected in accordance with standard protocols hospitals or clinics generally follow. For blood, an appropriate amount of peripheral blood (e.g., between 3-40 milliliters) often is collected and can be stored according to standard procedures prior to or after preparation.

A sample or test sample can include samples containing spores, viruses, cells, nucleic acid from prokaryotes or eukaryotes, or any free nucleic acid. For example, a method described herein may be used for detecting nucleic acid on the outside of spores (e.g., without the need for lysis). A sample can be isolated from any material suspected of containing a target sequence, such as from a subject described above. In some embodiments, a target sequence is present in air, plant, soil, or other materials suspected of containing biological organisms.

Nucleic acid may be derived (e.g., isolated, extracted, purified) from one or more sources by methods known in the art. Any suitable method can be used for isolating, extracting and/or purifying nucleic acid from a biological sample, non-limiting examples of which include methods of DNA preparation in the art, and various commercially available reagents or kits, such as Qiagen's QIAamp Circulating Nucleic Acid Kit, QiaAmp DNA Mini Kit or QiaAmp DNA Blood Mini Kit (Qiagen, Hilden, Germany), GenomicPrep^{™} Blood DNA Isolation Kit (Promega, Madison, Wis.), GFX^{™} Genomic Blood DNA Purification Kit (Amersham, Piscataway, N.J.), and the like or combinations thereof.

In some embodiments, a cell lysis procedure is performed. Cell lysis may be performed prior to initiation of a reaction provided herein. Cell lysis procedures and reagents are known in the art and may generally be performed by chemical (e.g., detergent, hypotonic solutions, enzymatic procedures, and the like, or combination thereof), physical (e.g., French press, sonication, and the like), or electrolytic lysis methods. Any suitable lysis procedure can be utilized. For example, chemical methods generally employ lysing agents to disrupt cells and extract nucleic acids from the cells, followed by treatment with chaotropic salts. In some embodiments, cell lysis comprises use of detergents (e.g., ionic, nonionic, anionic, zwitterionic). In some embodiments, cell lysis comprises use of ionic detergents (e.g., sodium dodecyl sulfate (SDS), sodium lauryl sulfate (SLS), deoxycholate, cholate, sarkosyl). Physical methods such as freeze/thaw followed by grinding, the use of cell presses and the like also may be useful. High salt lysis procedures also may be used. For example, an alkaline lysis procedure may be utilized. The latter procedure traditionally incorporates the use of phenol-chloroform solutions, and an alternative phenol-chloroform-free procedure involving three solutions may be utilized. In the latter procedures, one solution can contain 15 mM Tris, pH 8.0; 10 mM EDTA and 100 µg/ml RNase A; a second solution can contain 0.2 N NaOH and 1% SDS; and a third solution can contain 3M KOAc, pH 5.5, for example. In some embodiments, a cell lysis buffer is used in conjunction with the methods and components described herein.

Nucleic acid can be provided for conducting methods described herein without processing of the sample(s) containing the nucleic acid. For example, in some embodiments, nucleic acid is provided for conducting amplification methods described herein without prior nucleic acid purification. In some embodiments, a target sequence is amplified directly from a sample (e.g., without performing any nucleic acid extraction, isolation, purification and/or partial purification steps). In some embodiments, nucleic acid is provided for conducting methods described herein after processing of the sample(s) containing the nucleic acid. For example, a nucleic acid can be extracted, isolated, purified, or partially purified from the sample(s). The term "isolated" generally refers to nucleic acid removed from its original environment (e.g., the natural environment if it is naturally occurring, or a host cell if expressed exogenously), and thus is altered by human intervention (e.g., "by the hand of man") from its original environment. The term "isolated nucleic acid" can refer to a nucleic acid removed from a subject (e.g., a human subject). An isolated nucleic acid can be provided with fewer non-nucleic acid components (e.g., protein, lipid, carbohydrate) than the amount of components present in a source sample. A composition comprising isolated nucleic acid can be about 50% to greater than 99% free of non-nucleic acid components. A composition comprising isolated nucleic acid can be about 90%, 91%, 92%, 93%, 94%, 95%, 96%, 97%, 98%, 99% or greater than 99% free of non-nucleic acid components. The term "purified" generally refers to a nucleic acid provided that contains fewer non-nucleic acid components (e.g., protein, lipid, carbohydrate) than the amount of non-nucleic acid components present prior to subjecting the nucleic acid to a purification procedure. A composition comprising purified nucleic acid may be about 80%, 81%, 82%, 83%, 84%, 85%, 86%, 87%, 88%, 89%, 90%, 91%, 92%, 93%, 94%, 95%, 96%, 97%, 98%, 99% or greater than 99% free of other non-nucleic acid components.

Nucleic acid may be provided for conducting methods described herein without modifying the nucleic acid. Modifications may include, for example, denaturation, digestion, nicking, unwinding, incorporation and/or ligation of heterogeneous sequences, addition of epigenetic modifications, addition of labels (e.g., radiolabels such as ³²P, ³³P, ¹²⁵I, or ³⁵S; enzyme labels such as alkaline phosphatase; fluorescent labels such as fluorescein isothiocyanate (FITC); or other labels such as biotin, avidin, digoxigenin, antigens, haptens, fluorochromes), and the like. Accordingly, in some embodiments, an unmodified nucleic acid is amplified.

A method of the present disclosure for detecting a target nucleic acid sequence (single-stranded or double-stranded DNA and/or RNA) in a sample can detect a target nucleic acid sequence (e.g., DNA or RNA) with a high degree of sensitivity. In some embodiments, a method of the present disclosure can be used to detect a target RNA/DNA present in a sample comprising a plurality of RNAs/DNAs (including the target RNA/DNA and a plurality of non-target RNAs/DNAs), where the target RNA/DNA is present at one or more copies per 10⁷ non-target RNAs/DNAs (e.g., one or more copies per 10⁶ non-target RNAs/DNAs, one or more copies per 10⁵ non-target RNAs/DNAs, one or more copies per 10⁴ non-target RNAs/DNAs, one or more copies per 10³ non-target RNAs/DNAs, one or more copies per 10² non-target RNAs/DNAs, one or more copies per 50 non-target RNAs/DNAs, one or more copies per 20 non-target RNAs/DNAs, one or more copies per 10 non-target RNAs/DNAs, or one or more copies per 5 non-target RNAs/DNAs). In some embodiments, a method of the present disclosure can be used to detect a target RNA/DNA present in a sample comprising a plurality of RNAs/DNAs (including the target RNA/DNA and a plurality of non-target RNAs/DNAs), where the target RNA/DNA is present at one or more copies per 10¹⁸ non-target RNAs/DNAs (e.g., one or more copies per 10¹⁵ non-target RNAs/DNAs, one or more copies per 10¹² non-target RNAs/DNAs, one or more copies per 10⁹ non-target RNAs/DNAs, one or more copies per 10⁶ non-target RNAs/DNAs, one or more copies per 10⁵ non-target RNAs/DNAs, one or more copies per 10⁴ non-target RNAs/DNAs, one or more copies per 10³ non-target RNAs/DNAs, one or more copies per 10² non-target RNAs/DNAs, one or more copies per 50 non-target RNAs/DNAs, one or more copies per 20 non-target RNAs/DNAs, one or more copies per 10 non-target RNAs/DNAs, or one or more copies per 5 non-target RNAs/DNAs). As used herein, the terms "RNA/DNA" and "RNAs/DNAs" shall be given their ordinary meaning, and shall also refer to DNA, or RNA, or a combination of DNA and RNA.

In some embodiments, a method of the present disclosure can detect a target RNA/DNA present in a sample, where the target RNA/DNA is present at from one copy per 10⁷ non-target RNAs/DNAs to one copy per 10 non-target RNAs/DNAs (e.g., from 1 copy per 10⁷ non-target RNAs/DNAs to 1 copy per 10² non-target RNAs/DNAs, from 1 copy per 10⁷ non-target RNAs/DNAs to 1 copy per 10³ non-target RNAs/DNAs, from 1 copy per 10⁷ non-target RNAs/DNAs to 1 copy per 10⁴ non-target RNAs/DNAs, from 1 copy per 10⁷ non-target RNAs/DNAs to 1 copy per 10⁵ non-target RNAs/DNAs, from 1 copy per 10⁷ non-target RNAs/DNAs to 1 copy per 10⁶ non-target RNAs/DNAs, from 1 copy per 10⁶ non-target RNAs/DNAs to 1 copy per 10 non-target RNAs/DNAs, from 1 copy per 10⁶ non-target RNAs/DNAs to 1 copy per 10² non-target RNAs/DNAs, from 1 copy per 10⁶ non-target RNAs/DNAs to 1 copy per 10³ non-target RNAs/DNAs, from 1 copy per 10⁶ non-target RNAs/DNAs to 1 copy per 10⁴ non-target RNAs/DNAs, from 1 copy per 10⁶ non-target RNAs/DNAs to 1 copy per 10⁵ non-target RNAs/DNAs, from 1 copy per 10⁵ non-target RNAs/DNAs to 1 copy per 10 non-target RNAs/DNAs, from 1 copy per 10⁵ non-target RNAs/DNAs to 1 copy per 10² non-target RNAs/DNAs, from 1 copy per 10⁵ non-target RNAs/DNAs to 1 copy per 10³ non-target RNAs/DNAs, or from 1 copy per 10⁵ non-target RNAs/DNAs to 1 copy per 10⁴ non-target RNAs/DNAs).

In some embodiments, a method of the present disclosure can detect a target RNA/DNA present in a sample, where the target RNA/DNA is present at from one copy per 10¹⁸ non-target RNAs/DNAs to one copy per 10 non-target RNAs/DNAs (e.g., from 1 copy per 10¹⁸ non-target RNAs/DNAs to 1 copy per 10² non-target RNAs/DNAs, from 1 copy per 10¹⁵ non-target RNAs/DNAs to 1 copy per 10² non-target RNAs/DNAs, from 1 copy per 10¹² non-target RNAs/DNAs to 1 copy per 10² non-target RNAs/DNAs, from 1 copy per 10⁹ non-target RNAs/DNAs to 1 copy per 10² non-target RNAs/DNAs, from 1 copy per 10⁷ non-target RNAs/DNAs to 1 copy per 10² non-target RNAs/DNAs, from 1 copy per 10⁷ non-target RNAs/DNAs to 1 copy per 10³ non-target RNAs/DNAs, from 1 copy per 10⁷ non-target RNAs/DNAs to 1 copy per 10⁴ non-target RNAs/DNAs, from 1 copy per 10⁷ non-target RNAs/DNAs to 1 copy per 10⁵ non-target RNAs/DNAs, from 1 copy per 10⁷ non-target RNAs/DNAs to 1 copy per 10⁶ non-target RNAs/DNAs, from 1 copy per 10⁶ non-target RNAs/DNAs to 1 copy per 10 non-target RNAs/DNAs, from 1 copy per 10⁶ non-target RNAs/DNAs to 1 copy per 10² non-target RNAs/DNAs, from 1 copy per 10⁶ non-target RNAs/DNAs to 1 copy per 10³ non-target RNAs/DNAs, from 1 copy per 10⁶ non-target RNAs/DNAs to 1 copy per 10⁴ non-target RNAs/DNAs, from 1 copy per 10⁶ non-target RNAs/DNAs to 1 copy per 10⁵ non-target RNAs/DNAs, from 1 copy per 10⁵ non-target RNAs/DNAs to 1 copy per 10 non-target RNAs/DNAs, from 1 copy per 10⁵ non-target RNAs/DNAs to 1 copy per 10² non-target RNAs/DNAs, from 1 copy per 10⁵ non-target RNAs/DNAs to 1 copy per 10³ non-target RNAs/DNAs, or from 1 copy per 10⁵ non-target RNAs/DNAs to 1 copy per 10⁴ non-target RNAs/DNAs).

In some embodiments, a method of the present disclosure can detect a target RNA/DNA present in a sample, where the target RNA/DNA is present at from one copy per 10⁷ non-target RNAs/DNAs to one copy per 100 non-target RNAs/DNAs (e.g., from 1 copy per 10⁷ non-target RNAs/DNAs to 1 copy per 10² non-target RNAs/DNAs, from 1 copy per 10⁷ non-target RNAs/DNAs to 1 copy per 10³ non-target RNAs/DNAs, from 1 copy per 10⁷ non-target RNAs/DNAs to 1 copy per 10⁴ non-target RNAs/DNAs, from 1 copy per 10⁷ non-target RNAs/DNAs to 1 copy per 10⁵ non-target RNAs/DNAs, from 1 copy per 10⁷ non-target RNAs/DNAs to 1 copy per 10⁶ non-target RNAs/DNAs, from 1 copy per 10⁶ non-target RNAs/DNAs to 1 copy per 100 non-target RNAs/DNAs, from 1 copy per 10⁶ non-target RNAs/DNAs to 1 copy per 10² non-target RNAs/DNAs, from 1 copy per 10⁶ non-target RNAs/DNAs to 1 copy per 10³ non-target RNAs/DNAs, from 1 copy per 10⁶ non-target RNAs/DNAs to 1 copy per 10⁴ non-target RNAs/DNAs, from 1 copy per 10⁶ non-target RNAs/DNAs to 1 copy per 10⁵ non-target RNAs/DNAs, from 1 copy per 10⁵ non-target RNAs/DNAs to 1 copy per 100 non-target RNAs/DNAs, from 1 copy per 10⁵ non-target RNAs/DNAs to 1 copy per 10² non-target RNAs/DNAs, from 1 copy per 10⁵ non-target RNAs/DNAs to 1 copy per 10³ non-target RNAs/DNAs, or from 1 copy per 10⁵ non-target RNAs/DNAs to 1 copy per 10⁴ non-target RNAs/DNAs).

In some embodiments, the threshold of detection, for a subject method of detecting a target RNA/DNA in a sample, is 10 nM or less. The term "threshold of detection" is used herein to describe the minimal amount of target RNA/DNA that must be present in a sample in order for detection to occur. Thus, as an illustrative example, when a threshold of detection is 10 nM, then a signal can be detected when a target RNA/DNA is present in the sample at a concentration of 10 nM or more. In some embodiments, a method of the present disclosure has a threshold of detection of 5 nM or less. In some embodiments, a method of the present disclosure has a threshold of detection of 1 nM or less. In some embodiments, a method of the present disclosure has a threshold of detection of 0.5 nM or less. In some embodiments, a method of the present disclosure has a threshold of detection of 0.1 nM or less. In some embodiments, a method of the present disclosure has a threshold of detection of 0.05 nM or less. In some embodiments, a method of the present disclosure has a threshold of detection of 0.01 nM or less. In some embodiments, a method of the present disclosure has a threshold of detection of 0.005 nM or less. In some embodiments, a method of the present disclosure has a threshold of detection of 0.001 nM or less. In some embodiments, a method of the present disclosure has a threshold of detection of 0.0005 nM or less. In some embodiments, a method of the present disclosure has a threshold of detection of 0.0001 nM or less. In some embodiments, a method of the present disclosure has a threshold of detection of 0.00005 nM or less. In some embodiments, a method of the present disclosure has a threshold of detection of 0.00001 nM or less. In some embodiments, a method of the present disclosure has a threshold of detection of 10 pM or less. In some embodiments, a method of the present disclosure has a threshold of detection of 1 pM or less. In some embodiments, a method of the present disclosure has a threshold of detection of 500 fM or less. In some embodiments, a method of the present disclosure has a threshold of detection of 250 fM or less. In some embodiments, a method of the present disclosure has a threshold of detection of 100 fM or less. In some embodiments, a method of the present disclosure has a threshold of detection of 50 fM or less. In some embodiments, a method of the present disclosure has a threshold of detection of 500 aM (attomolar) or less. In some embodiments, a method of the present disclosure has a threshold of detection of 250 aM or less. In some embodiments, a method of the present disclosure has a threshold of detection of 100 aM or less. In some embodiments, a method of the present disclosure has a threshold of detection of 50 aM or less. In some embodiments, a method of the present disclosure has a threshold of detection of 10 aM or less. In some embodiments, a method of the present disclosure has a threshold of detection of 1 aM or less.

In some embodiments, the threshold of detection (for detecting the target RNA and/or DNA in a subject method), is in a range of from 500 fM to 1 nM (e.g., from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM) (where the concentration refers to the threshold concentration of target RNA/DNA at which the target RNA/DNA can be detected). In some embodiments, a method of the present disclosure has a threshold of detection in a range of from 800 fM to 100 pM. In some embodiments, a method of the present disclosure has a threshold of detection in a range of from 1 pM to 10 pM. In some embodiments, a method of the present disclosure has a threshold of detection in a range of from 10 fM to 500 fM, e.g., from 10 fM to 50 fM, from 50 fM to 100 fM, from 100 fM to 250 fM, or from 250 fM to 500 fM.

In some embodiments, the minimum concentration at which a target RNA/DNA can be detected in a sample is in a range of from 500 fM to 1 nM (e.g., from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM). In some embodiments, the minimum concentration at which a target RNA/DNA can be detected in a sample is in a range of from 800 fM to 100 pM. In some embodiments, the minimum concentration at which a target RNA/DNA can be detected in a sample is in a range of from 1 pM to 10 pM.

In some embodiments, the threshold of detection (for detecting the target RNA./DNA in a subject method), is in a range of from 1 aM to 1 nM (e.g., from 1 aM to 500 pM, from 1 aM to 200 pM, from 1 aM to 100 pM, from 1 aM to 10 pM, from 1 aM to 1 pM, from 100 aM to 1 nM, from 100 aM to 500 pM, from 100 aM to 200 pM, from 100 aM to 100 pM, from 100 aM to 10 pM, from 100 aM to 1 pM, from 250 aM to 1 nM, from 250 aM to 500 pM, from 250 aM to 200 pM, from 250 aM to 100 pM, from 250 aM to 10 pM, from 250 aM to 1 pM, from 500 aM to 1 nM, from 500 aM to 500 pM, from 500 aM to 200 pM, from 500 aM to 100 pM, from 500 aM to 10 pM, from 500 aM to 1 pM, from 750 aM to 1 nM, from 750 aM to 500 pM, from 750 aM to 200 pM, from 750 aM to 100 pM, from 750 aM to 10 pM, from 750 aM to 1 pM, from 1 fM to 1 nM, from 1 fM to 500 pM, from 1 fM to 200 pM, from 1 fM to 100 pM, from 1 fM to 10 pM, from 1 fM to 1 pM, from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM) (where the concentration refers to the threshold concentration of target RNA/DNA at which the target RNA/DNA can be detected). In some embodiments, a method of the present disclosure has a threshold of detection in a range of from 1 aM to 800 aM. In some embodiments, a method of the present disclosure has a threshold of detection in a range of from 50 aM to 1 pM. In some embodiments, a method of the present disclosure has a threshold of detection in a range of from 50 aM to 500 fM.

In some embodiments, the minimum concentration at which a target RNA/DNA can be detected in a sample is in a range of from 1 aM to 1 nM (e.g., from 1 aM to 500 pM, from 1 aM to 200 pM, from 1 aM to 100 pM, from 1 aM to 10 pM, from 1 aM to 1 pM, from 100 aM to 1 nM, from 100 aM to 500 pM, from 100 aM to 200 pM, from 100 aM to 100 pM, from 100 aM to 10 pM, from 100 aM to 1 pM, from 250 aM to 1 nM, from 250 aM to 500 pM, from 250 aM to 200 pM, from 250 aM to 100 pM, from 250 aM to 10 pM, from 250 aM to 1 pM, from 500 aM to 1 nM, from 500 aM to 500 pM, from 500 aM to 200 pM, from 500 aM to 100 pM, from 500 aM to 10 pM, from 500 aM to 1 pM, from 750 aM to 1 nM, from 750 aM to 500 pM, from 750 aM to 200 pM, from 750 aM to 100 pM, from 750 aM to 10 pM, from 750 aM to 1 pM, from 1 fM to 1 nM, from 1 fM to 500 pM, from 1 fM to 200 pM, from 1 fM to 100 pM, from 1 fM to 10 pM, from 1 fM to 1 pM, from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM). In some embodiments, the minimum concentration at which a target RNA/DNA can be detected in a sample is in a range of from 1 aM to 500 pM. In some embodiments, the minimum concentration at which a target RNA/DNA can be detected in a sample is in a range of from 100 aM to 500 pM.

In some embodiments, a disclosed composition or method exhibits an attomolar (aM) sensitivity of detection. In some embodiments, a disclosed composition or method exhibits a femtomolar (fM) sensitivity of detection. In some embodiments, a disclosed composition or method exhibits a picomolar (pM) sensitivity of detection. In some embodiments, a disclosed composition or method exhibits a nanomolar (nM) sensitivity of detection.

A disclosed sample includes sample nucleic acids (e.g., a plurality of sample nucleic acids). The term "plurality" is used herein to mean two or more. Thus, in some embodiments, a sample includes two or more (e.g., 3 or more, 5 or more, 10 or more, 20 or more, 50 or more, 100 or more, 500 or more, 1,000 or more, or 5,000 or more) sample nucleic acids (e.g., RNAs). A disclosed method can be used as a very sensitive way to detect a target nucleic acid present in a sample (e.g., in a complex mixture of nucleic acids such as RNAs). In some embodiments the sample includes 5 or more DNAs (e.g., 10 or more, 20 or more, 50 or more, 100 or more, 500 or more, 1,000 or more, or 5,000 or more RNAs) that differ from one another in sequence. In some embodiments, the sample includes 10 or more, 20 or more, 50 or more, 100 or more, 500 or more, 10³ or more, 5×10³ or more, 10⁴ or more, 5×10⁴ or more, 10⁵ or more, 5×10⁵ or more, 10⁶ or more 5×10⁶ or more, or 10⁷ or more, DNAs. In some embodiments, the sample comprises from 10 to 20, from 20 to 50, from 50 to 100, from 100 to 500, from 500 to 10³, from 10³ to 5×10³, from 5×10³ to 10⁴, from 10⁴ to 5×10⁴, from 5×10⁴ to 10⁵, from 10⁵ to 5×10⁵, from 5×10⁵ to 10⁶, from 10⁶ to 5×10⁶, or from 5×10⁶ to 10⁷, or more than 10⁷, DNAs. In some embodiments, the sample comprises from 5 to 10⁷ RNAs (e.g., that differ from one another in sequence)(e.g., from 5 to 10⁶, from 5 to 10⁵, from 5 to 50,000, from 5 to 30,000, from 10 to 10⁶, from 10 to 10⁵, from 10 to 50,000, from 10 to 30,000, from 20 to 10⁶, from 20 to 10⁵, from 20 to 50,000, or from 20 to 30,000 DNAs). In some embodiments the sample includes 20 or more RNAs that differ from one another in sequence. In some embodiments, the sample includes RNAs from a cell lysate (e.g., a eukaryotic cell lysate, a mammalian cell lysate, a human cell lysate, a prokaryotic cell lysate, a plant cell lysate, and the like). For example, in some embodiments, the sample includes DNA from a cell such as a eukaryotic cell, e.g., a mammalian cell such as a human cell.

The term "sample" is used here shall be given its ordinary meaning and shall include any sample that includes RNA and/or DNA (e.g., in order to determine whether a target DNA and/or target RNA is present among a population of RNAs and/or DNAs). The sample can be derived from any source, e.g., the sample can be a synthetic combination of purified DNAs and/or RNAs; the sample can be a cell lysate, an DNA/RNA-enriched cell lysate, or DNAs/RNAs isolated and/or purified from a cell lysate. The sample can be from a patient (e.g., for the purpose of diagnosis). The sample can be from permeabilized cells. The sample can be from crosslinked cells. The sample can be in tissue sections. The sample can be from tissues prepared by crosslinking followed by delipidation and adjustment to make a uniform refractive index.

Suitable samples include but are not limited to saliva, blood, serum, plasma, urine, aspirate, and biopsy samples. A sample can be from a patient and encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., RNAs. The term "sample" encompasses biological samples such as a clinical sample such as blood, plasma, serum, aspirate, cerebral spinal fluid (CSF), and also includes tissue obtained by surgical resection, tissue obtained by biopsy, cells in culture, cell supernatants, cell lysates, tissue samples, organs, bone marrow, and the like. A "biological sample" includes biological fluids derived therefrom (e.g., cancerous cell, infected cell, etc.), e.g., a sample comprising RNAs that is obtained from such cells (e.g., a cell lysate or other cell extract comprising RNAs).

In some embodiments, the source of the sample is a (or is suspected of being a) diseased cell, fluid, tissue, or organ. In some embodiments, the source of the sample is a normal (non-diseased) cell, fluid, tissue, or organ. In some embodiments, the source of the sample is a (or is suspected of being a pathogen-infected cell, tissue, or organ. For example, the source of a sample can be an individual who may or may not be infected-and the sample could be any biological sample (e.g., blood, saliva, biopsy, plasma, serum, bronchoalveolar lavage, sputum, a fecal sample, cerebrospinal fluid, a fine needle aspirate, a swab sample (e.g., a buccal swab, a cervical swab, a nasal swab), interstitial fluid, synovial fluid, nasal discharge, tears, buffy coat, a mucous membrane sample, an epithelial cell sample (e.g., epithelial cell scraping), etc.) collected from the individual. In some embodiments, the sample is a cell-free liquid sample. In some embodiments, the sample is a liquid sample that can comprise cells. Pathogens include viruses, fungi, helminths, protozoa, malarial parasites, *Plasmodium* parasites, *Toxoplasma* parasites, *Schistosoma* parasites, and the like. "Helminths" include roundworms, heartworms, and phytophagous nematodes (Nematoda), flukes (Tematoda), Acanthocephala, and tapeworms (Cestoda). Protozoan infections include infections from Giardia spp., *Trichomonas* spp., African trypanosomiasis, amoebic dysentery, babesiosis, balantidial dysentery, Chaga's disease, coccidiosis, malaria and toxoplasmosis. Examples of pathogens such as parasitic/protozoan pathogens include, but are not limited to: *Plasmodium falciparum, Plasmodium vivax, Trypanosoma cruzi* and *Toxoplasma gondii.* Fungal pathogens include, but are not limited to: *Cryptococcus neoformans, Histoplasma capsulatum, Coccidioides immitis, Blastomyces dermatitidis, Chlamydia trachomatis,* and *Candida albicans.* Pathogenic viruses include, e.g, immunodeficiency virus (e.g., HIV); influenza virus; dengue; West Nile virus; herpes virus; yellow fever virus; Hepatitis Virus C; Hepatitis Virus A; Hepatitis Virus B; papillomavirus; and the like. Pathogenic viruses can include DNA viruses such as: a papovavirus (e.g., human papillomavirus (HPV), polyomavirus); a hepadnavirus (e.g., Hepatitis B Virus (HBV)); a herpesvirus (e.g., herpes simplex virus (HSV), varicella zoster virus (VZV), epstein-barr virus (EBV), cytomegalovirus (CMV), herpes lymphotropic virus, *Pityriasis Rosea,* kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like. Pathogens can include, e.g., DNAviruses [e.g.: a papovavirus (e.g., human papillomavirus (HPV), polyomavirus); a hepadnavirus (e.g., Hepatitis B Virus (HBV)); a herpesvirus (e.g., herpes simplex virus (HSV), varicella zoster virus (VZV), epstein-barr virus (EBV), cytomegalovirus (CMV), herpes lymphotropic virus, *Pityriasis Rosea,* kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like], *Mycobacterium tuberculosis, Streptococcus agalactiae,* methicillin-resistant *Staphylococcus aureus, Legionella pneumophila, Streptococcus pyogenes, Escherichia coli, Neisseria gonorrhoeae, Neisseria meningitidis, Pneumococcus, Cryptococcus neoformans, Histoplasma capsulatum, Hemophilus influenzae B, Treponema pallidum,* Lyme disease spirochetes, *Pseudomonas aeruginosa, Mycobacterium leprae, Brucella abortus,* rabies virus, influenza virus, cytomegalovirus, herpes simplex virus I, herpes simplex virus II, human serum parvo-like virus, respiratory syncytial virus, varicella-zoster virus, hepatitis B virus, hepatitis C virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, Sendai virus, feline leukemia virus, Reovirus, polio virus, simian virus 40, mouse mammary tumor virus, dengue virus, rubella virus, West Nile virus, *Plasmodium falciparum, Plasmodium vivax, Toxoplasma gondii, Trypanosoma rangeli, Trypanosoma cruzi, Trypanosoma rhodesiense, Trypanosoma brucei, Schistosoma mansoni, Schistosoma japonicum, Babesia bovis, Eimeria tenella, Onchocerca volvulus, Leishmania tropica, Mycobacterium tuberculosis, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium and M. pneumoniae.* Pathogenic viruses can comprise one or more of SARS-CoV-2, Influenza A, Influenza B, and/or Influenza C.

The sample can be a biological sample, for example a clinical sample. In some embodiments, the sample is taken from a biological source, such as vagina, urethra, penis, anus, throat, cervix, fermentation broths, cell cultures, and the like. The sample can comprise, for example, fluid and cells from stool samples. The biological sample can be used (i) directly as obtained from the subject or source, or (ii) following a pre-treatment to modify the character of the sample. Thus, the test sample can be pre-treated prior to use, for example, by disrupting cells or viral particles, preparing liquids from solid materials, diluting viscous fluids, filtering liquids, concentrating liquids, inactivating interfering components, adding reagents, purifying nucleic acids, and the like. Accordingly, a "biological sample" as used herein includes nucleic acids (DNA, RNA or total nucleic acids) extracted from a clinical or biological specimen. Sample preparation can also include using a solution that contains buffers, salts, detergents, and/or the like which are used to prepare the sample for analysis. In some embodiments, the sample is processed before molecular testing. In some embodiments, the sample is analyzed directly, and is not pre-processed prior to testing. The sample can be, for example, a stool sample. In some embodiments, the sample is a stool sample from a patient with clinical symptoms of acute gastroenteritis.

In some embodiments, a sample to be tested is processed prior to performing the methods disclosed herein. For example, in some embodiments, the sample can be isolated, concentrated, or subjected to various other processing steps prior to performing the methods disclosed herein. For example, in some embodiments, the sample can be processed to isolate nucleic acids from the sample prior to contacting the sample with the oligonucleotides, as disclosed herein. In some embodiments, the methods disclosed herein are performed on the sample without culturing the sample *in vitro.* In some embodiments, the methods disclosed herein are performed on the sample without isolating nucleic acids from the sample prior to contacting the sample with oligonucleotides as disclosed herein.

A sample can comprise one or more nucleic acids (e.g., a plurality of nucleic acids). The term "plurality" as used herein can refer two or more. Thus, in some embodiments, a sample includes two or more (e.g., 3 or more, 5 or more, 10 or more, 20 or more, 50 or more, 100 or more, 500 or more, 1,000 or more, or 5,000 or more) nucleic acids (e.g., gDNA, mRNA). A disclosed method can be used as a very sensitive way to detect a target nucleic acid present in a sample (e.g., in a complex mixture of nucleic acids such as gDNAs). In some embodiments, the sample includes 5 or more nucleic acids (e.g., 10 or more, 20 or more, 50 or more, 100 or more, 500 or more, 1,000 or more, or 5,000 or more nucleic acids) that differ from one another in sequence. In some embodiments, the sample includes 10 or more, 20 or more, 50 or more, 100 or more, 500 or more, 10³ or more, 5 x 10³ or more, 10⁴ or more, 5 x 10⁴ or more, 10⁵ or more, 5 x 10⁵ or more, 10⁶ or more 5 x 10⁶ or more, or 10⁷ or more, nucleic acids.

In some embodiments, the sample comprises from 10 to 20, from 20 to 50, from 50 to 100, from 100 to 500, from 500 to 10³, from 10³ to 5 x 10³, from 5 x 10³ to 10⁴, from 10⁴ to 5 x 10⁴, from 5 x 10⁴ to 10⁵, from 10⁵ to 5 x 10⁵, from 5 x 10⁵ to 10⁶, from 10⁶ to 5 x 10⁶, or from 5 x 10⁶ to 10⁷, or more than 10⁷, nucleic acids. In some embodiments, the sample comprises from 5 to 10⁷ nucleic acids (e.g., that differ from one another in sequence)(e.g., from 5 to 10⁶, from 5 to 10⁵, from 5 to 50,000, from 5 to 30,000, from 10 to 10⁶, from 10 to 10⁵, from 10 to 50,000, from 10 to 30,000, from 20 to 10⁶, from 20 to 10⁵, from 20 to 50,000, or from 20 to 30,000 nucleic acids, or a number or a range between any two of these values). In some embodiments, the sample includes 20 or more nucleic acids that differ from one another in sequence.

A sample can be any sample that includes nucleic acid (e.g., in order to determine whether a target nucleic acid is present among a population of nucleic acids). The sample can be derived from any source, e.g., the sample can be a synthetic combination of purified nucleic acids; the sample can be a cell lysate, an DNA-enriched cell lysate, or nucleic acids isolated and/or purified from a cell lysate. The sample can be from a patient (e.g., for the purpose of diagnosis). The sample can be from permeabilized cells. The sample can be from crosslinked cells. The sample can be in tissue sections. The sample can be from tissues prepared by crosslinking followed by delipidation and adjustment to make a uniform refractive index.

A sample can include a target nucleic acid and a plurality of non-target nucleic acids. In some embodiments, the target nucleic acid is present in the sample at one copy per 10 non-target nucleic acids, one copy per 20 non-target nucleic acids, one copy per 25 non-target nucleic acids, one copy per 50 non-target nucleic acids, one copy per 100 non-target nucleic acids, one copy per 500 non-target nucleic acids, one copy per 10³ non-target nucleic acids, one copy per 5 x 10³ non-target nucleic acids, one copy per 10⁴ non-target nucleic acids, one copy per 5 x 10⁴ non-target nucleic acids, one copy per 10⁵ non-target nucleic acids, one copy per 5 x 10⁵ non-target nucleic acids, one copy per 10⁶ non-target nucleic acids, less than one copy per 10⁶ non-target nucleic acids, or a number or a range between any two of these values. In some embodiments, the target nucleic acid is present in the sample at from one copy per 10 non-target nucleic acids to 1 copy per 20 non-target nucleic acids, from 1 copy per 20 non-target nucleic acids to 1 copy per 50 non target nucleic acids, from 1 copy per 50 non-target nucleic acids to 1 copy per 100 non-target nucleic acids, from 1 copy per 100 non-target nucleic acids to 1 copy per 500 non-target nucleic acids, from 1 copy per 500 non target nucleic acids to 1 copy per 10³ non-target nucleic acids, from 1 copy per 10³ non-target nucleic acids to 1 copy per 5 x 10³ non-target nucleic acids, from 1 copy per 5 x 10³ non-target nucleic acids to 1 copy per 10⁴ non target nucleic acids, from 1 copy per 10⁴ non-target nucleic acids to 1 copy per 10⁵ non-target nucleic acids, from 1 copy per 10⁵ non-target nucleic acids to 1 copy per 10⁶ non-target nucleic acids, or from 1 copy per 10⁶ non target nucleic acids to 1 copy per 10⁷ non-target nucleic acids, or a number or a range between any two of these values.

Suitable samples include but are not limited to saliva, blood, serum, plasma, urine, aspirate, and biopsy samples. Thus, the term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids. The term "sample" encompasses biological samples such as a clinical sample such as blood, plasma, serum, aspirate, cerebral spinal fluid (CSF), and also includes tissue obtained by surgical resection, tissue obtained by biopsy, cells in culture, cell supernatants, cell lysates, tissue samples, organs, bone marrow, and the like. A "biological sample" includes biological fluids derived therefrom (e.g., cancerous cell, infected cell, etc.), e.g., a sample comprising nucleic acids that is obtained from such cells (e.g., a cell lysate or other cell extract comprising nucleic acids).

Appropriate samples for use in the methods disclosed herein include any conventional biological sample obtained from an organism or a part thereof, such as a plant, animal, bacteria, and the like. In particular embodiments, the biological sample is obtained from an animal subject, such as a human subject. A biological sample is any solid or fluid sample obtained from, excreted by or secreted by any living organism, including, without limitation, single celled organisms, such as bacteria, yeast, protozoans, and amoebas among others, multicellular organisms (such as plants or animals, including samples from a healthy or apparently healthy human subject or a human patient affected by a condition or disease to be diagnosed or investigated, such as an infection with a pathogenic microorganism, such as a pathogenic bacteria or virus). For example, a biological sample can be a biological fluid obtained from, for example, blood, plasma, serum, urine, stool, sputum, mucous, lymph fluid, synovial fluid, bile, ascites, pleural effusion, seroma, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease, such as rheumatoid arthritis, osteoarthritis, gout or septic arthritis), or a swab of skin or mucosal membrane surface.

A sample can also be a sample obtained from any organ or tissue (including a biopsy or autopsy specimen, such as a tumor biopsy) or can include a cell (whether a primary cell or cultured cell) or medium conditioned by any cell, tissue or organ. Exemplary samples include, without limitation, cells, cell lysates, blood smears, cytocentrifuge preparations, cytology smears, bodily fluids (e.g., blood, plasma, serum, saliva, sputum, urine, bronchoalveolar lavage, semen, etc.), tissue biopsies (e.g., tumor biopsies), fine-needle aspirates, and/or tissue sections (e.g., cryostat tissue sections and/or paraffin-embedded tissue sections). In other examples, the sample includes circulating tumor cells (which can be identified by cell surface markers). In particular examples, samples are used directly (e.g., fresh or frozen), or can be manipulated prior to use, for example, by fixation (e.g., using formalin) and/or embedding in wax (such as formalin-fixed paraffin-embedded (FFPE) tissue samples). It will be appreciated that any method of obtaining tissue from a subject can be utilized, and that the selection of the method used will depend upon various factors such as the type of tissue, age of the subject, or procedures available to the practitioner. Standard techniques for acquisition of such samples are available in the art.

A sample can be an environmental sample, such as water, soil, or a surface such as industrial or medical surface.

Owing to the increased sensitivity of the embodiments disclosed herein, in certain example embodiments, the assays and methods may be run on crude samples or samples where the target molecules to be detected are not further fractionated or purified from the sample.

Cells can be lysed to liberate the target molecules (e.g., target dsDNA). Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9,10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

### Kits

The kit described herein can comprise: a plurality of protein complexes. In some embodiments, each of the plurality of protein complexes comprises a transposome and a programmable DNA binding unit capable of specifically binding to a binding site on a target double-stranded DNA (dsDNA). In some embodiments, the transposome comprises a transposase, a first adaptor and a second adaptor. In some embodiments, the binding site for each of the plurality of protein complexes is different from each other. In some embodiments, the kit comprises: at least one component providing real-time detection activity for a nucleic acid amplification product. The real-time detection activity can be provided by a molecular beacon. The dried composition can comprise a reverse transcriptase and/or a reverse transcription primer.

Kits can comprise, for example, one or more polymerases and one or more primers, and optionally one or more reverse transcriptases and/or reverse transcription primers, as described herein. Where one target is amplified, a pair of primers (forward and reverse) can be included in the kit. Where multiple target sequences are amplified, a plurality of primer pairs can be included in the kit. A kit can include a control polynucleotide, and where multiple target sequences are amplified, a plurality of control polynucleotides can be included in the kit.

Kits can also comprise one or more of the components in any number of separate vessels, chambers, containers, packets, tubes, vials, microtiter plates and the like, or the components can be combined in various combinations in such containers. Components of the kit can, for example, be present in one or more containers. In some embodiments, all of the components are provided in one container. In some embodiments, the enzymes (e.g., polymerase(s) and/or reverse transcriptase(s)) can be provided in a separate container from the primers. The components can, for example, be lyophilized, heat dried, freeze dried, or in a stable buffer. In some embodiments, polymerase(s) and/or reverse transcriptase(s) are in lyophilized form or heat dried form in a single container, and the primers are either lyophilized, heat dried, freeze dried, or in buffer, in a different container. In some embodiments, polymerase(s) and/or reverse transcriptase(s), and the primers are, in lyophilized form or heat dried form, in a single container.

Kits can further comprise, for example, dNTPs used in the reaction, or modified nucleotides, vessels, cuvettes or other containers used for the reaction, or a vial of water or buffer for re-hydrating lyophilized or heat-dried components. The buffer used can, for example, be appropriate for both polymerase and primer annealing activity.

Kits can also comprise instructions for performing one or more methods described herein and/or a description of one or more components described herein. Instructions and/or descriptions can be in printed form and can be included in a kit insert. A kit also can include a written description of an internet location that provides such instructions or descriptions.

Kits can further comprise reagents used for detection methods, for example, reagents used for FRET, lateral flow devices, dipsticks, fluorescent dye, colloidal gold particles, latex particles, a molecular beacon, or polystyrene beads.

FIGS. 1, 3, 4, 5A-5F and 7A-7H of the present disclosure were created with BioRender.com.

### EXAMPLES

Some aspects of the embodiments discussed above are disclosed in further detail in the following examples, which are not in any way intended to limit the scope of the present disclosure.

### Example 1

### Design and validation of fusion proteins and guideRNAs (sgRNAs)

Four constructs for generation of fusion proteins were designed: dCAS9-Fl26-Tn5, dCAS9-xTen-Tn5, Tn5-Fl26-dCas9, Tn5-xTen-dCas9 (*See,* e.g., FIG. 8-FIG. 10). These constructs have either the dCas9 or the Tn5 sequence at the N-terminal end of the fusion protein separated by a Fl26 linker or xTen linker. Plasmid design was based on, in some embodiments: "Chen, S.P. & Wang, H.H. (2019). An Engineered Cas-Transposon System for Programmable and Site-Directed DNA Transposition. The CRISPR Journal. Vol 2, Number 6. DOI: 10.1089/crispr.2019.0030 and Picelli S., Bjorklund, A.K., Reinius, B., Sgasser, S., Wingerb, G., & Sandbert, R. (2014)"; and "Tn5 transposase and tagmentation procedures for massively scaled sequencing projects. Genome Research. 24:2033-2040. ISSN 1088-9051/14."

### sgRNA design

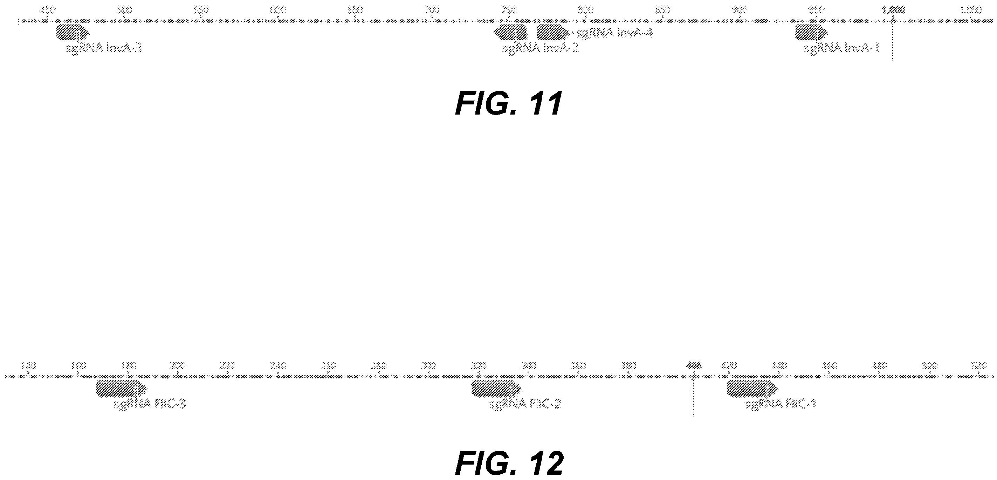

sgRNAs targeting the InvA and FliC genes of Salmonella Enterica were designed. Sequence from S. enterica strain ATCC 13311 was used. sgRNAs were designed using tools from integrated DNA technologies (IDT) (Table 1). Relative positions of the sgRNAs for InvA and FliC genes are shown in FIG. 11 and FIG. 12, respectively.

**TABLE 1: S. ENTERICA sgRNAS**

| **Sample Name** | **Tube label** | **[µM]** | **Species** | **Gene** | **Sequence** | **SEQ ID NO** |
|---|---|---|---|---|---|---|
| CD.Cas9.RNXS0617.AA | Sg1 | 10 | Salmonella enterica | InvA | GCTATTTTGACCATTTC AAT | 10 |
| CD.Cas9.RNXS0617.AB | Sg2 | 10 | Salmonella enterica | InvA | CGGAGGACAAATCCAT ACCA | 11 |
| CD.Cas9.RNXS0617.AC | Sg3 | 10 | Salmonella enterica | InvA | CAGTTTATCGTTATTAC CAA | 12 |
| CD.Cas9.RNXS0617.AD | Sg4 | 10 | Salmonella enterica | InvA | ACTTATACCATGCTGAC CAT | 13 |
| CD.Cas9.RZZJ8230.AA | Sg5 | 10 | Salmonella enterica | FliC | GGACAACACCCTGACC ATCC | 14 |
| CD.Cas9.RZZ7J8230.AC | Sg6 | 10 | Salmonella enterica | FliC | GTCTGACCTCGACTCC ATCC | 15 |
| CD.Cas9.RZZJ8230.AD | Sg7 | 10 | Salmonella enterica | FliC | GAACATCAAAGGTCTG ACTC | 16 |
| AC to AA 79 bp from PAM to 5' sgRNA. AD to AC 127 bp from PAM to 5' sgRNA. | | | | | | |

264 bp, 8 bp, 148 bp, 292 bp, 458 bp and 195 bp fragments were expected for InvA. About 130 bp and 82 bp and 232 bp fragments were expected for FliC.

### Validation of Salmonella Enterica sgRNAs

To validate the specificity of the sgRNAs, genomic samples were cut with Cas9. Adaptors were ligated to the DNA cut by Cas9, and PCR-amplified fragments were visualized by bioanalyzer.

**TABLE 2: BIOANALYZER ANALYSIS OF sgRNA ACTIVITY**

| **Cas 9 Expected Fragments [bp]** | **Universal Adaptor size [bp]** | **Barcode Adaptor size [bp]** | **Bioanalyzer expected size [bp]** | **Bioanalyzer Expected size low range [bp]** | **Bioanalyzer Expected size low range [bp]2** | **Actual [bp]** |
|---|---|---|---|---|---|---|
| 8 | 57 | 63 | 128 | 115.2 | 140.8 | 125 |
| 82 | 57 | 63 | 202 | 181.8 | 222.2 | 225 |
| 130 | 57 | 63 | 250 | 225 | 275 | 266 |
| 148 | 57 | 63 | 268 | 241.2 | 294.8 | 272 |
| 195 | 57 | 63 | 315 | 283.5 | 346.5 | 296 |
| 232 | 57 | 63 | 352 | 316.8 | 387.2 | 323 |
| 264 | 57 | 63 | 384 | 345.6 | 422.4 | 382 |
| 292 | 57 | 63 | 412 | 370.8 | 453.2 | 425 |
| 458 | 57 | 63 | 578 | 520.2 | 635.8 | |

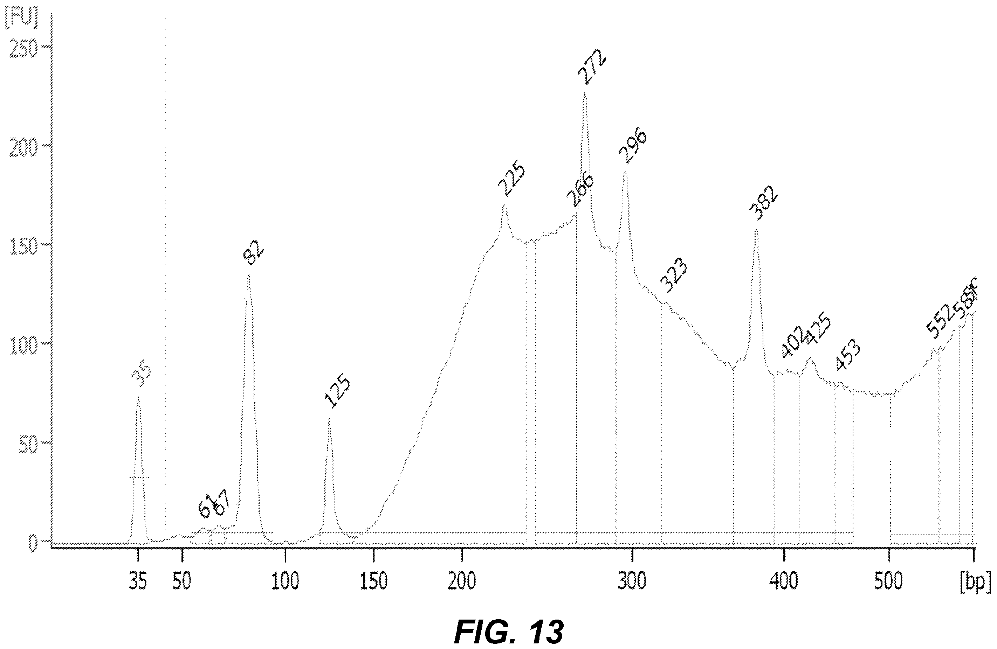

FIG. 13 and Table 2 show that cuts in the gDNA were specific to the expected size (compare "Bioanalyzer expected size [bp]" column to "Actual [bp]" column in Table 2), therefore demonstrating that the guide RNAs for Salmonella enterica are functional.

Next, sgRNAs targeting human genes EXT1, BCL9, HOXA13, HOXD11, and OLIG2 were designed for 10 total sgRNAs (Table 3A-Table 3C). sgRNAs were designed using GenScript's Tools.

**TABLE 3A: HUMAN sgRNA TARGETS**

| **Gene** | **Cut size [bp]** | **Adapter size [bp]** | **Total Fragment Size [bp]** | **gRNA Design** | **Tumor Type** |
|---|---|---|---|---|---|
| EXT1 | 255 | 34 | 323 | 1,4 | Exostoses, osteosarcoma |
| BCL9 | 66 | 34 | 134 | 2, 4 | B-cell acute lymphocytic leukaemia |
| HOXA13 | 445 | 34 | 513 | 1,4 | acute myelogenous leukaemia |
| HOXD11 | 225 | 34 | 293 | 1,3 | acute myelogenous leukaemia |
| OLIG2 | 114 | 34 | 182 | 1,2 | T-cell acute lymphoblastic leukaemia |

**TABLE 3B: HUMAN sgRNA TARGETS**

| **Gene** | **On Target Score 1** | **On Target Score 2** | **Overall Score 1** | **Overall Score 2** | **On Target AVG** | **Overall Score AVG** | **On Target Score 1** |
|---|---|---|---|---|---|---|---|
| EXT1 | 85.69 | 84.4 | 82 | 58 | 85.045 | 70 | 85.69 |
| BCL9 | 88.35 | 80.6 | 76 | 46 | 84.475 | 61 | 88.35 |
| HOXA13 | 91.27 | 73.1 | 77 | 60 | 82.185 | 68.5 | 91.27 |
| HOXD11 | 88.1 | 74.58 | 75 | 66 | 81.34 | 70.5 | 88.1 |
| OLIG2 | 64.69 | 96.08 | 65 | 57 | 80.385 | 61 | 64.69 |

**TABLE 3C: HUMAN sgRNA TARGETS**

| **Name** | **Input Sequence** | **SEQ ID NO:** |
|---|---|---|
| EXT1-1 | ATATCACGTCCATAACGGGG | 17 |
| EXT1-4 | CACTTGGCCTGACTACACCG | 18 |
| BCL9-2 | GGGTTGGCATCGGAACCACG | 19 |
| BCL9-4 | GATGCCCTCTCCAAATGCCG | 20 |
| HOXA13-1 | GTAGCCATAGGGCAGCGCCG | 21 |
| HOXA13-4 | TTTCTCTACGACAACGGCGG | 22 |
| HOXD11-1 | GGGCTTCGACCAGTTCTACG | 23 |
| HOXD 11-3 | GGGCTACGCTCCCTACTACG | 24 |
| OLIG2-1 | ACTGGTGAGCGAGATCTACG | 25 |
| OLIG2-2 | GCACGCCGCACATCACCCCG | 26 |

sgRNAs were also designed to target Chlamydia trachomatis gene polymorphic membrane protein A (pmp A) (Table 4). 5 sgRNAs in total were designed using tools from IDT.

**TABLE 4: C. TRACHOMATIS sgRNA TARGETS**

| Design ID | Gene Symbol | Position | Strand | Sequence | SEQ ID NO | PAM | On-Target Score | Fragment Size [bp] |
|---|---|---|---|---|---|---|---|---|
| CD.Cas9.CVSJ 0588.AF | pmpA | 45 | + | GAAATTAATG GTTTAAGCTT | 4 | TGG | 76 | 161 |
| CD.Cas9.CVSJ 0588.AA | pmpA | 206 | - | AGGTGAGCAA GATTTCCATT | 5 | TGG | 100 | 169 |
| CD.Cas9.CVSJ 0588.AC | pmpA | 375 | - | TCAAGGACAT ATTCTCCTGT | 6 | TGG | 85 | 48 |
| CD.Cas9.CVSJ 0588.AJ | pmpA | 423 | - | AATGTGCTCCA TAAGGAATT | 7 | AGG | 71 | 140 |
| CD.Cas9.CVSJ 0588.AG | pmpA | 563 | - | AACTTTCTTCT TCTGAGGAG | 8 | TGG | 75 | 260 |
| CD.Cas9.CVSJ 0588.AB | pmpA | 823 | + | AAGATCACAC CTATGGGAAA | 9 | TGG | 100 | |

### Validation of Transposase Tn5

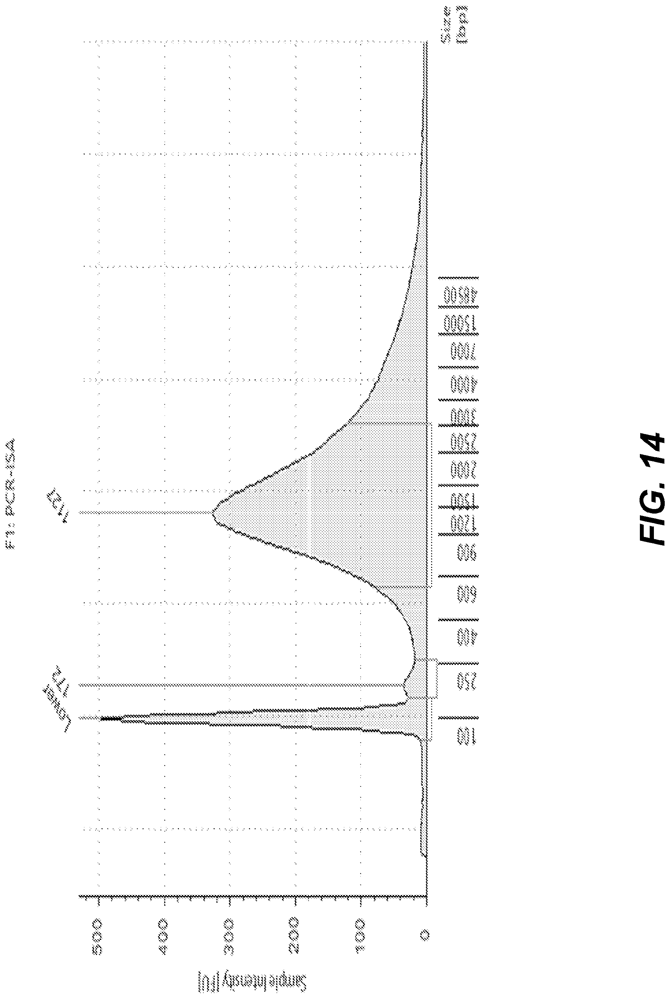

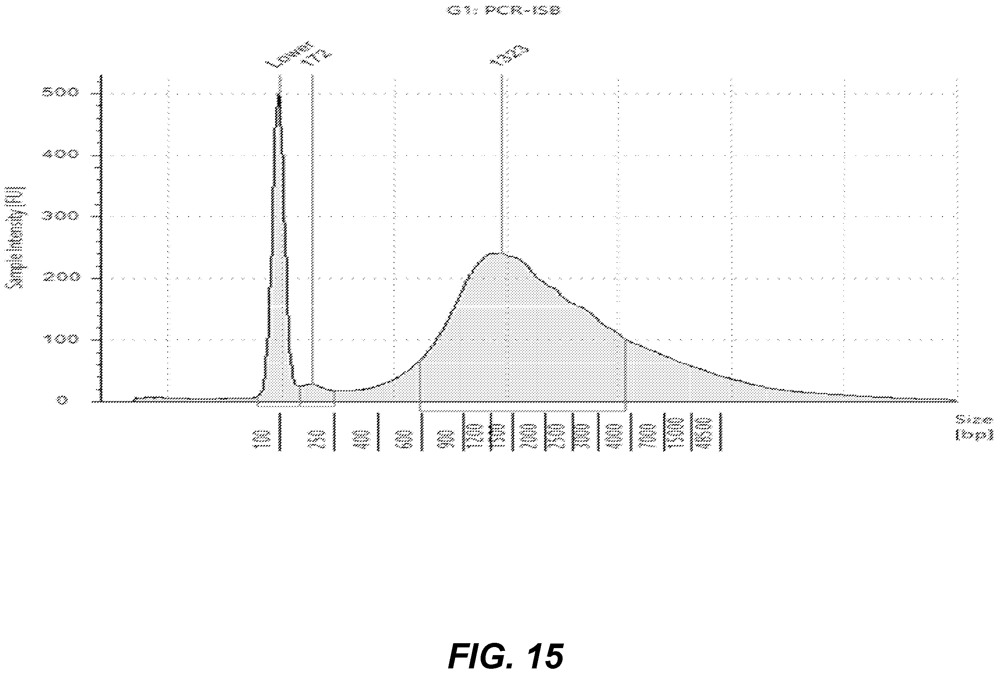

FIG. 14-FIG. 15 show that Tn5 can ligate designed Adaptor A (5'-TCGTCGGCAGCGTCAGATGTGTATAAGAGACAG-3', SEQ ID NO: 27) and Adaptor B (5'-GTCTCGTGGGCTCGGAGATGTGTATAAGAGACAG-3', SEQ ID NO: 28) to DNA fragments for PCR amplification, demonstrating functionality. First, Tn5 was used to cut and paste gDNA from *S. enterica* with custom adaptors. Labeled fragments were then amplified by PCR. Data in FIG. 14- FIG. 15 show that the Tn5 transposase was loaded with custom adaptors.

### Validation of Fusion Proteins

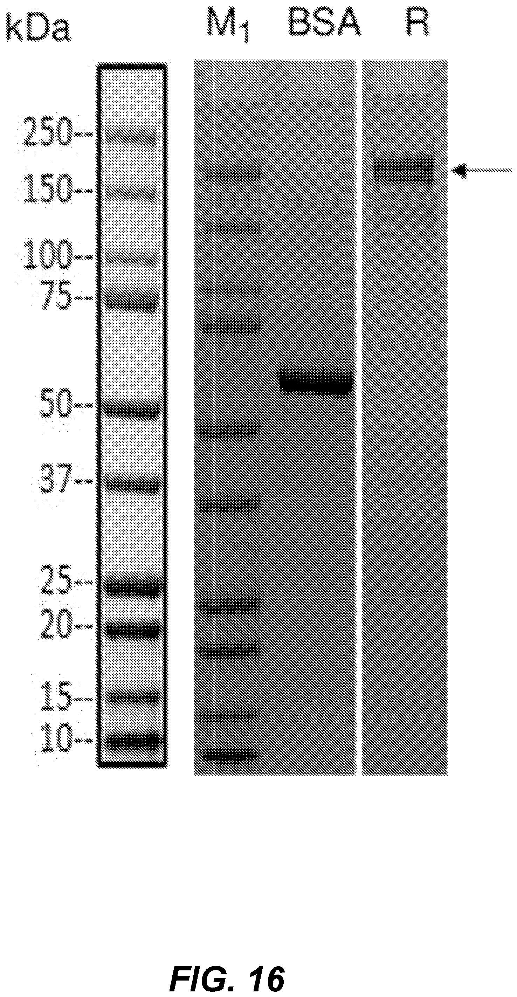

dCAS9-Fl26-Tn5, dCAS9-xTen-Tn5, Tn5-F126-dCas9, Tn5-xTen-dCas9 were recombinantly expressed and then purified. In some embodiments, the recombinant protein was isolated using a self-cleaving moiety (intein) on a chitin column. Purified fusion proteins were analyzed for predicted size and purity on SDS-PAGE gels (FIG. 16-FIG. 21).

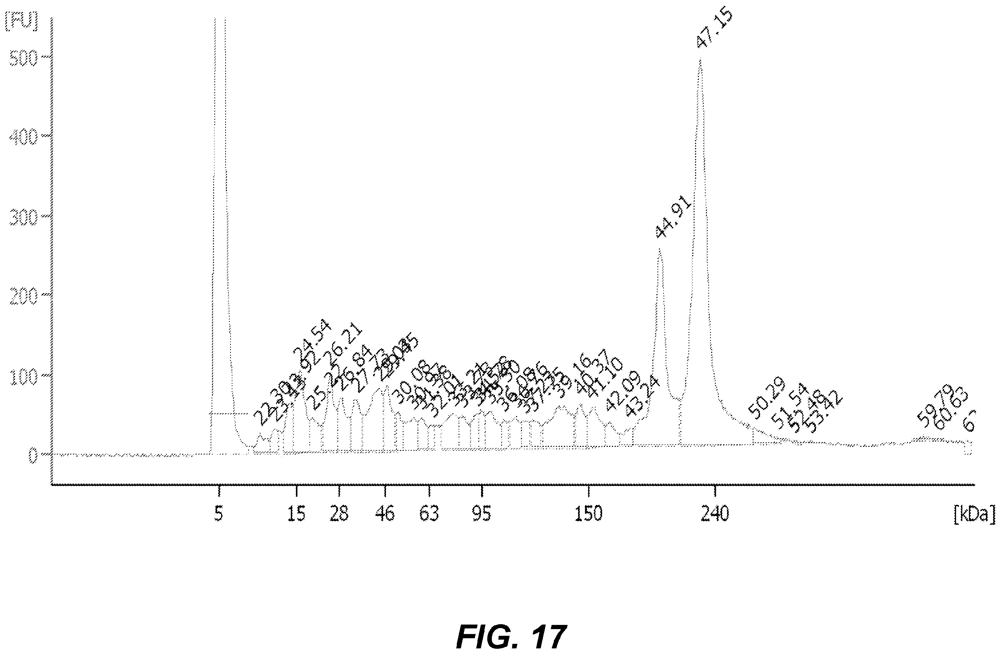

SDS-PAGE analysis of dCAS9-F126-Tn5 is shown in FIG. 16. The sample was observed to have a purity: > 80%. In some embodiments, the fusion protein may also comprise intein domain. Bioanalyzer analysis in FIG. 17 shows a portion of the protein made (peak in 44.91) is the correct size (contains no intein).

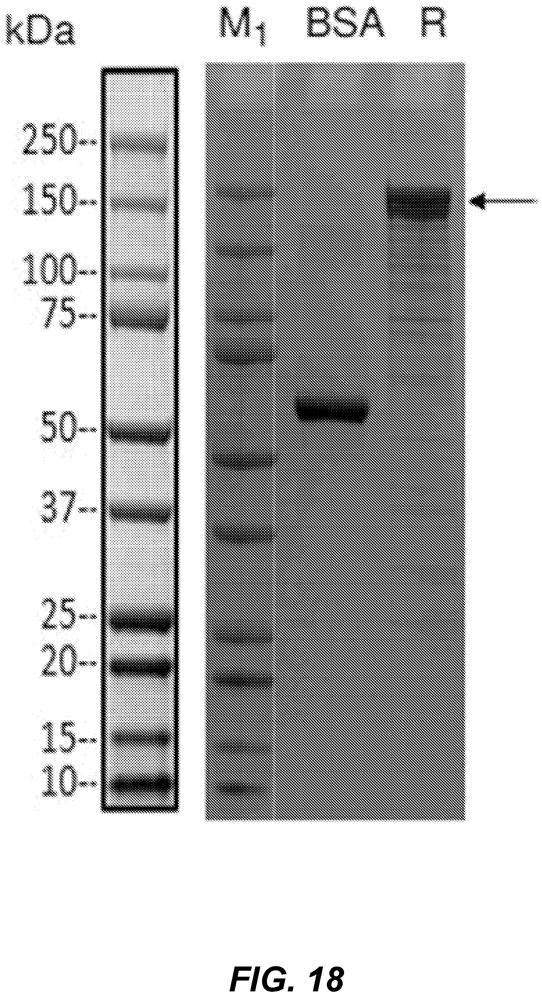

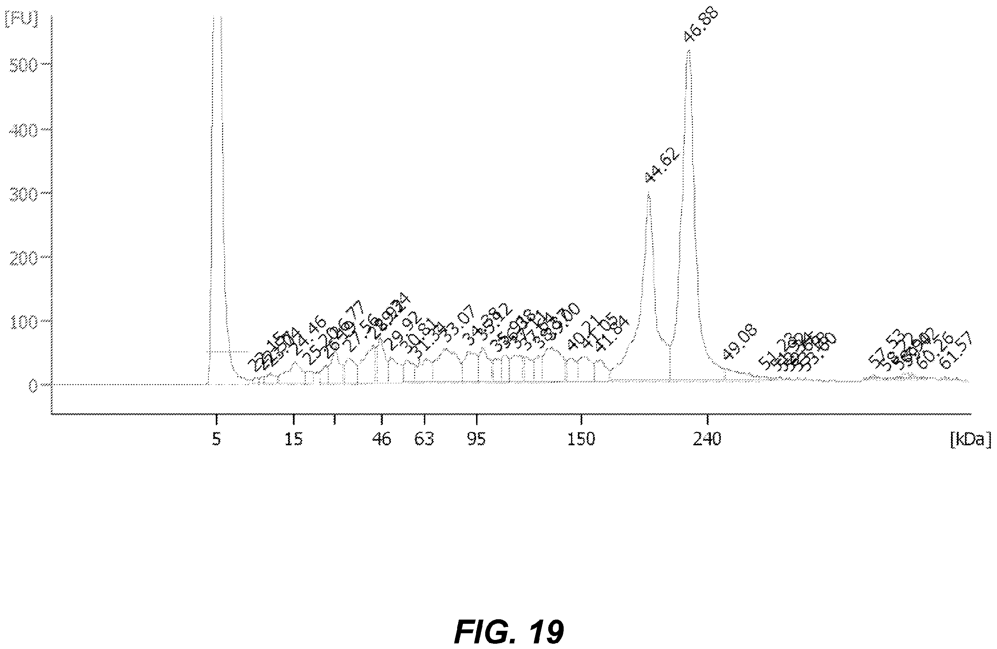

SDS-PAGE analysis of dCAS9-xTen-Tn5 is shown in FIG. 18. The sample was observed to have a purity: > 70%. In some embodiments, the fusion protein may also comprise intein domain, resulting in a larger than expected size. Bioanalyzer analysis in FIG. 19 shows that a portion of the protein made (peak in 44.62) is the correct size (contains no intein).

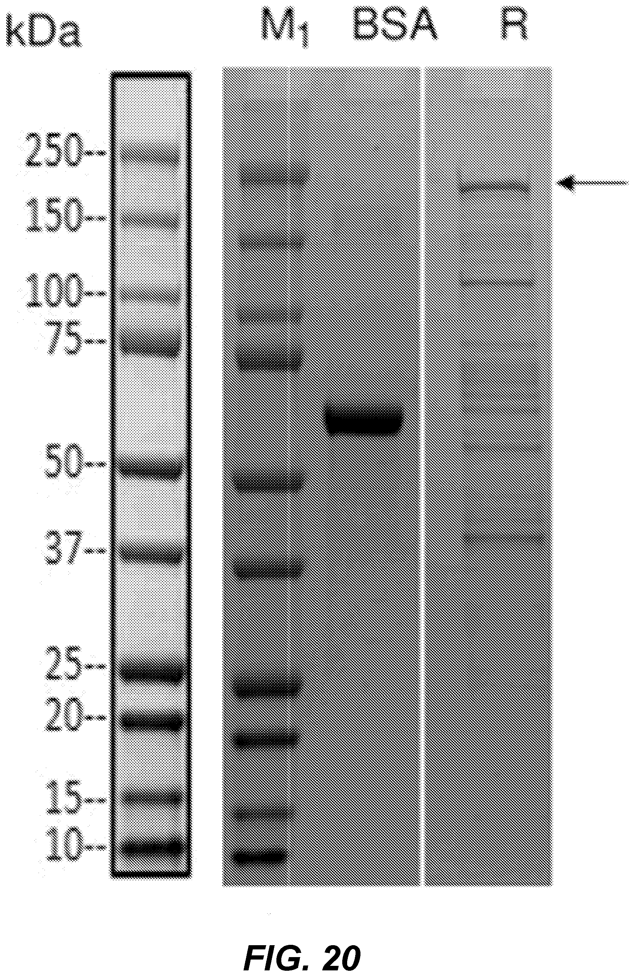

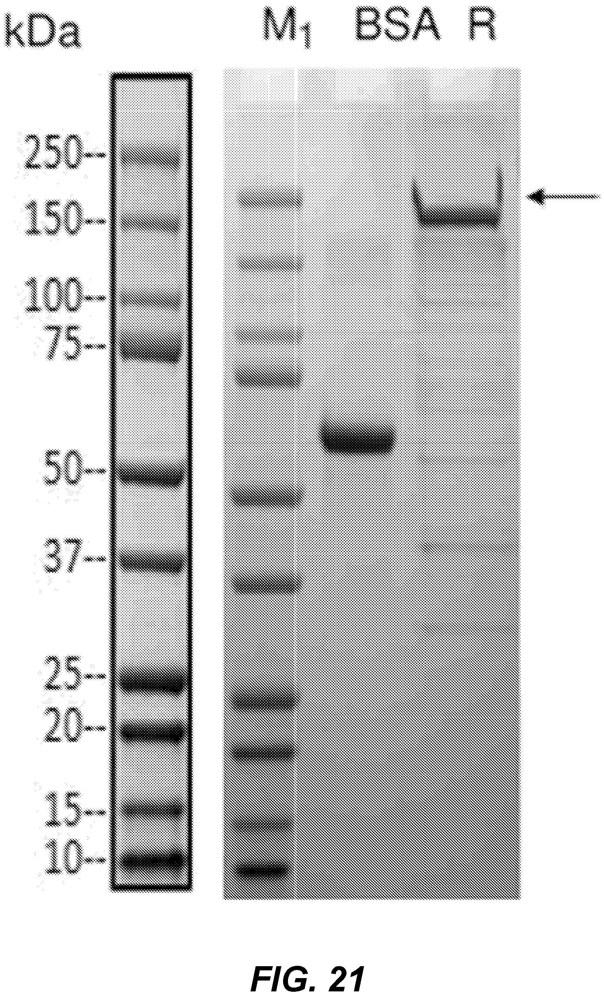

FIG. 20 depicts SDS-PAGE analysis of recombinantly expressed and purified Tn5-Fl26-dCas9. FIG. 21 depicts SDS-PAGE analysis of recombinantly expressed and purified Tn5-xTen-dCas9. The sample was observed to have a purity: > 65%.

### Testing Fusion Proteins for Functionality

dCas9-Fl26-Tn5 and dCas9-xTen-Tn5 were tested for functionality. The protocol was a follows: (1) Load sgRNA and Adaptors into fusion proteins (used Human sgRNAs unless otherwise indicated), (2) Guided Tagmentation, (3) Clean-up, (4) PCR amplification, (5) quality control (QC), and (6) results analysis.

### Load sgRNA and Adaptors into fusion proteins

Fusion protein was loaded at a ratio of 1:1:2 (1 molecule dCas9-Tn5 to 1 of sgRNA to 2 of adaptor. Mixture was incubated at 24°C for 30 minutes.

### Guided Tagmentation

100 mM dCas9-Tn5 (6.02 e10 molecules) and 500 ng human gDNA (1.52 e5 molecules) were combined for a 1 to 3.95e5 ratio of gDNA to dCas9-Tn5. Mixture was incubated for: 37°C for 60 mins and 55°C for 60 mins to generate tagged fragments. Several incubation methods were tried, and in some embodiments dCas9 can be functional in the range of 25°C to 42°C and Tn5 can be functional in the range of 37°C to 60°C. PCR amplification program is shown in Table 5.

**TABLE 5: PCR AMPLIFICATION**

| **Temp** | **Time** | |
|---|---|---|
| 95 | 30 seconds | |
| 72 | 3 minutes | |
| 95 | 10 seconds | X 35 cycles |
| 64 | 30 seconds | |
| 72 | 30 seconds | |
| 72 | 5 minutes | |
| 4 | Forever | |

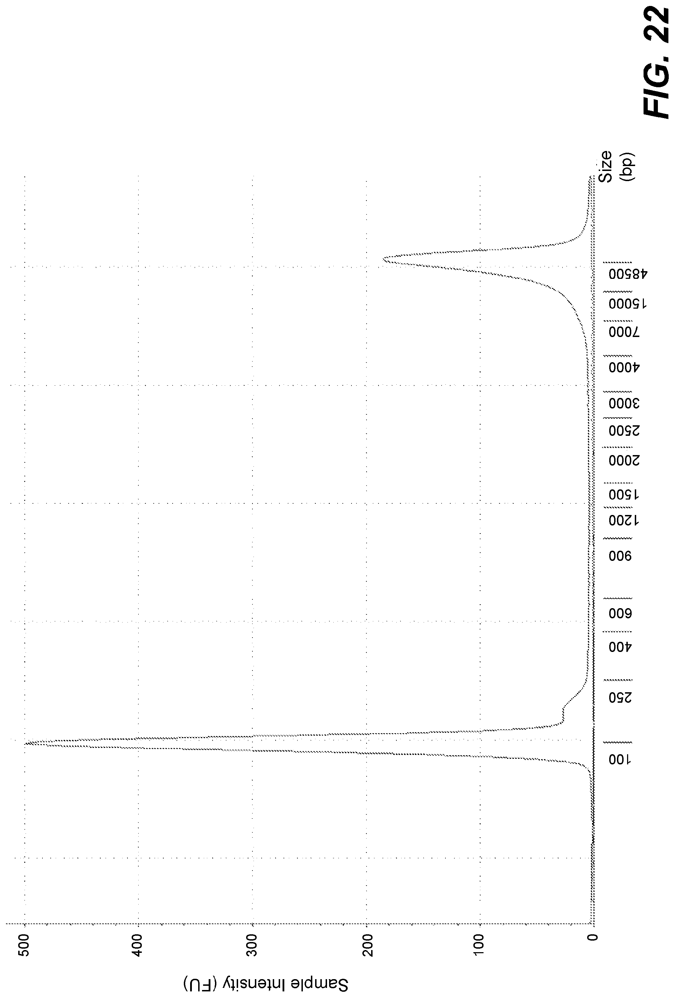

FIG. 22 depicts data related to Cas9 only control reactions. Visible line shows tape station analysis of DNA after Cas9 digestion. Analysis of sample after PCR amplification reaction shows no signal. This data indicated that the Cas9 by itself is unable to add the adaptors to the 5' or 3' end of DNA fragments.

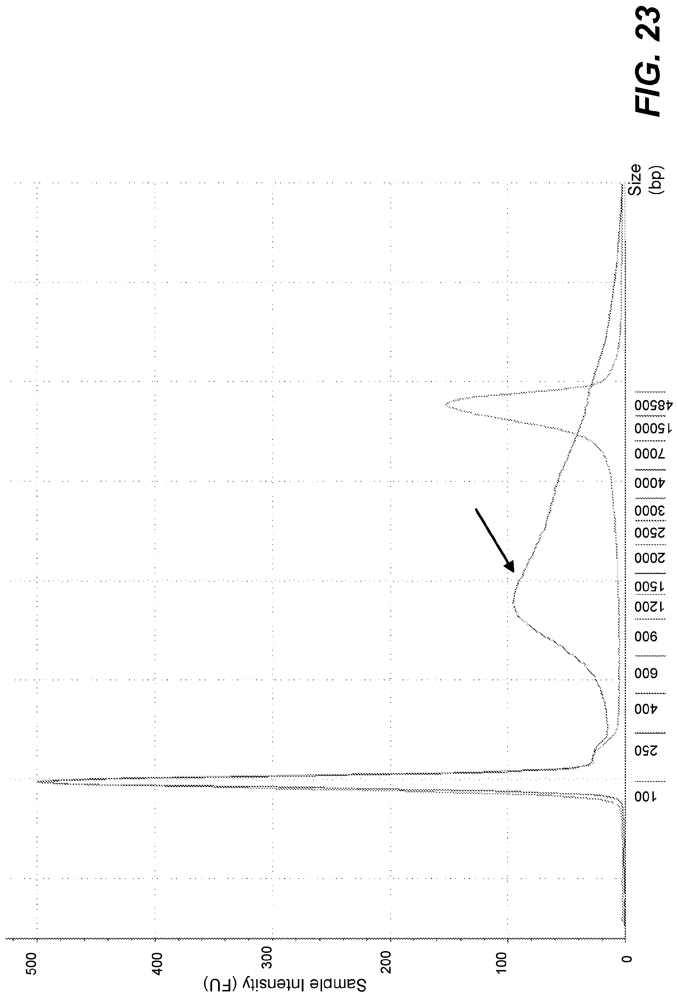

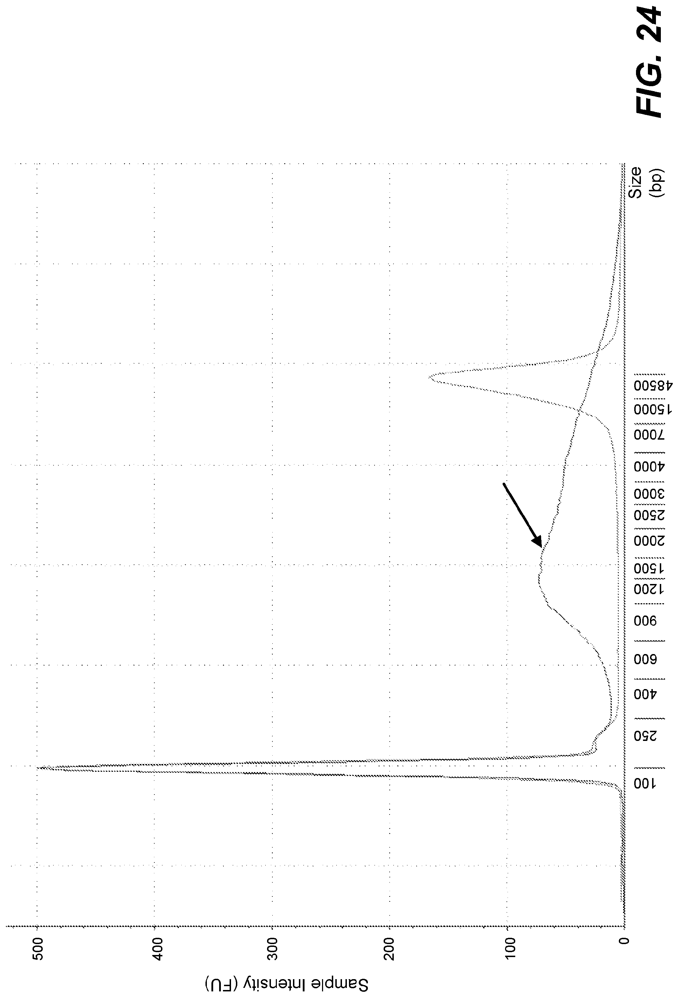

FIG. 23-FIG. 24 show results of PCR amplification following digestion and ligation of adaptors with dCas9-Fl26-Tn5 or dCas9-xTen-Tn5, respectively. Arrows in graphs point to signal from samples following PCR. PCR amplification was detected, which is only possible if both fusion proteins (dCas9-Fl26-Tn5 and dCas9-xTen-Tn5) can perform transposition (e.g., adding adaptors (Adaptor B) to the 5' end and 3'end of a DNA molecules).

### Results

Results show that the Tn5 was able to add the custom adaptors to the Human gDNA. The Cas9 only control showed that the process needs the Tn5 to amplify. These results showed functionality of the Tn5 fused to dCas9.

### Fusion Protein to DNA Ratio Test

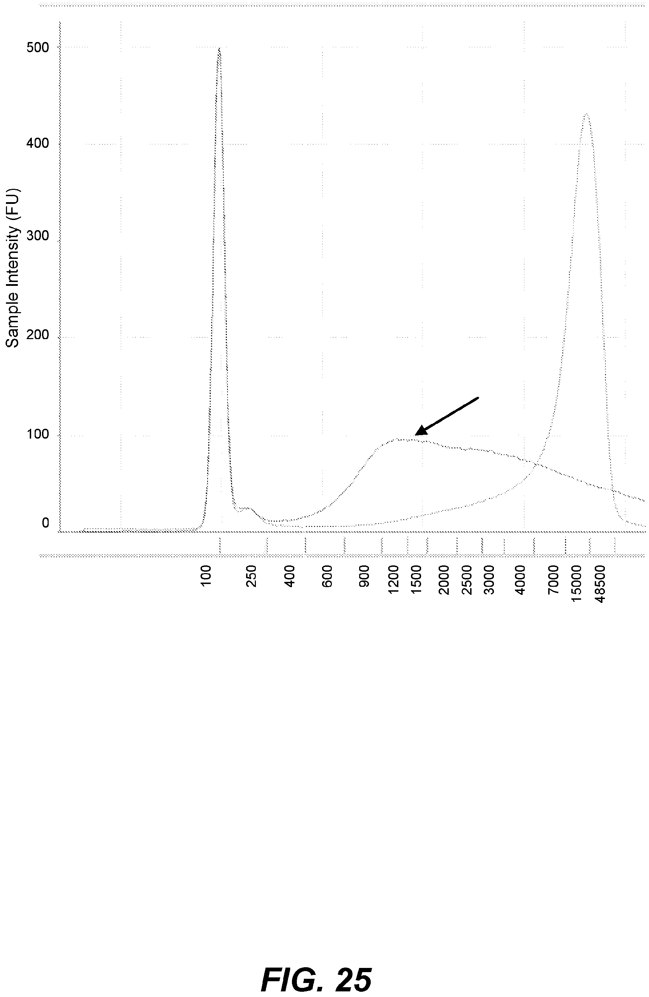

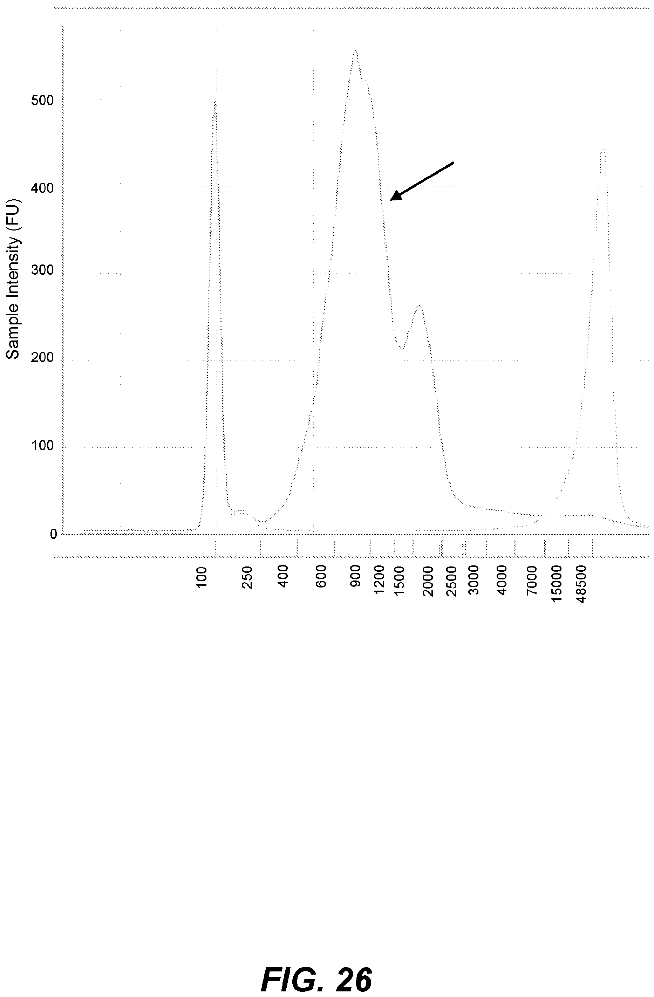

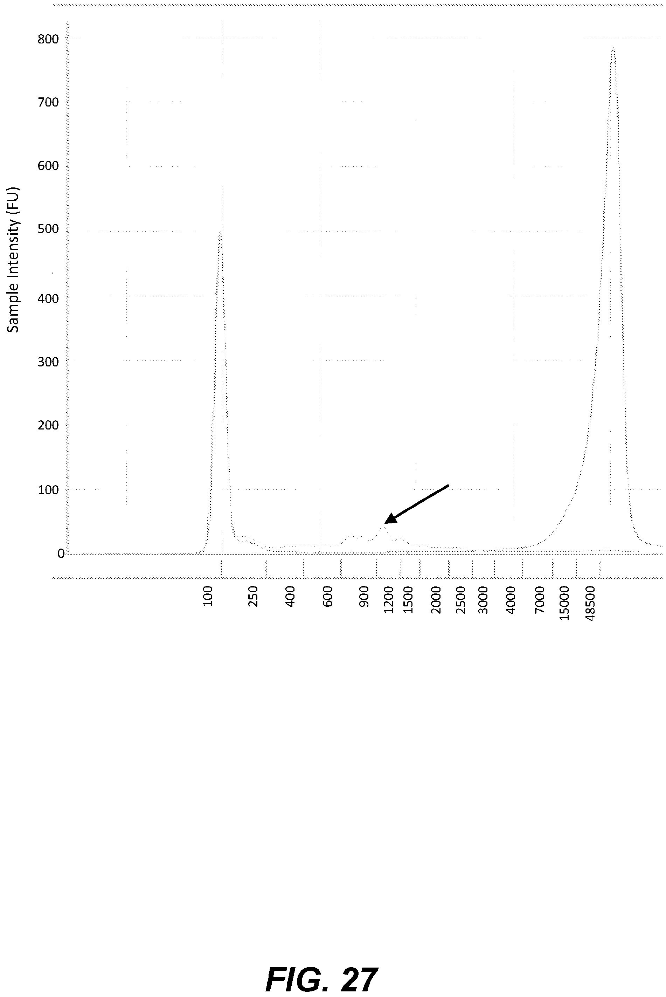

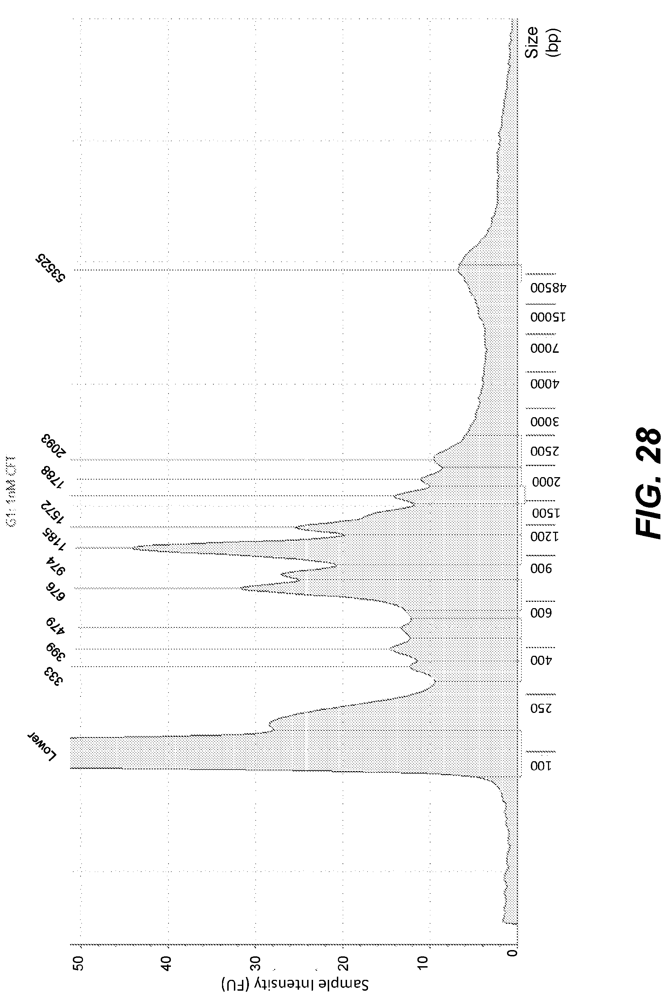

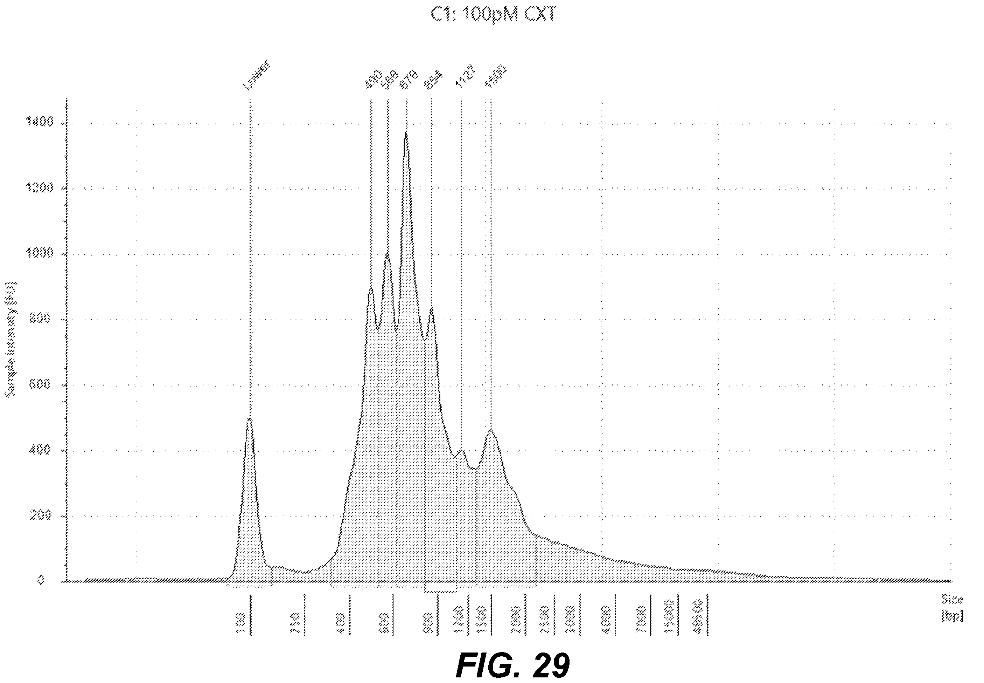

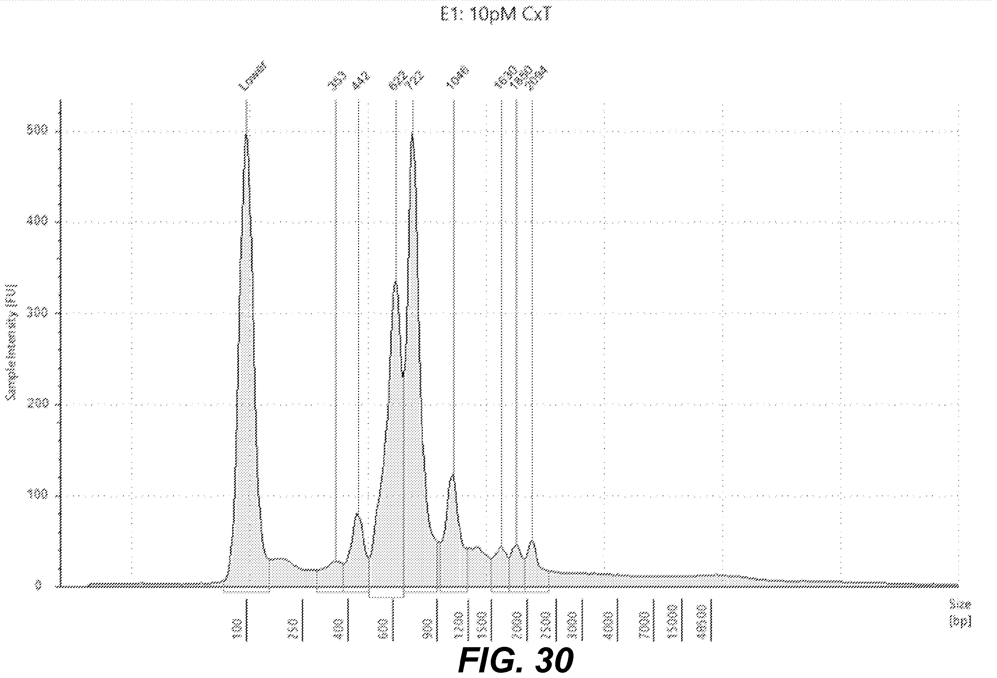

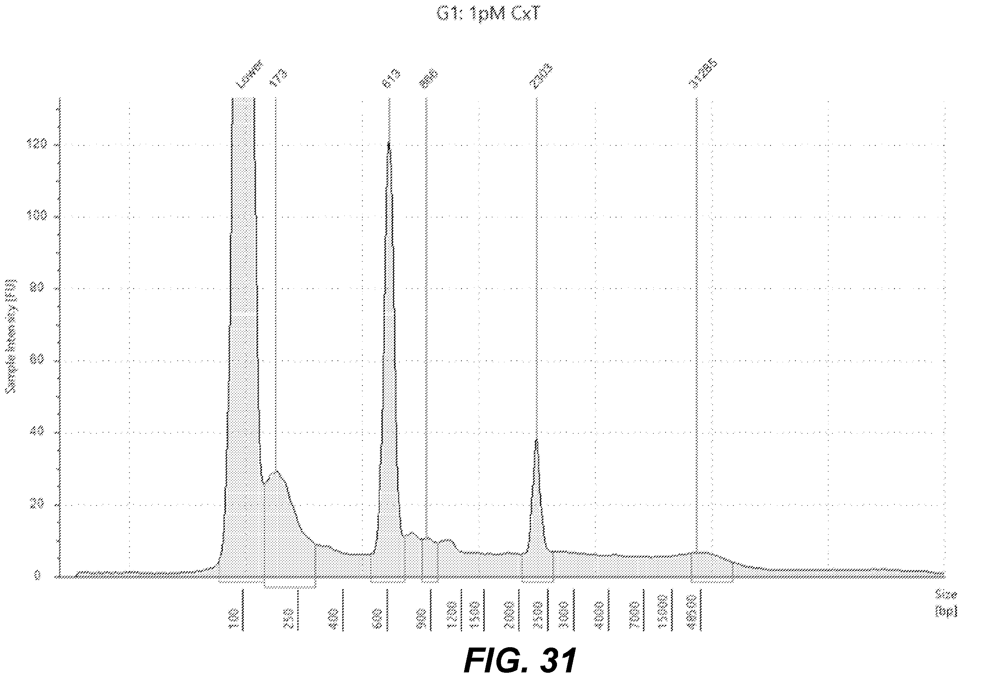

Next, effects of lowering the gDNA to Cas9-Tn5 ratio was tested. The DNA concentration was kept constant while lowering Cas-Tn fusion protein concentration: 100 nM (194,071 molecules of dCas9-Tn5 to 1 genomic copy of DNA), 1 nM (1,940 : 1), 100 pM (194: 1), 10 pM (19.4 : 1), 1 pM (1.94: 1). Results are shown in FIG. 25-FIG. 31. FIG. 25 depicts results of PCR amplification following guided tagmentation reactions using 194,071:1 ratio of dCas9-Tn5, and shows a broad peak following PCR, indicating non-specific tagmentation. Reducing the amount of dCas9-Tn5 (FIG. 26-FIG. 31) results in production of detectable peaks from the PCR reaction, indicating that decreasing the ratio of fusion protein to DNA adds specificity to tagmentation.

### Results

Results show that the Tn5 was able to add the custom adaptors to the Human gDNA. The Cas9-only control showed that the process needs the Tn5 to amplify DNA. The Tn5 was shown to be functional and there was evidence of guided transposition. Therefore, there is evidence for the fusion protein comprising both dCas9 and Tn5 activities.

### Fusion protein and sgRNAs on S. enterica

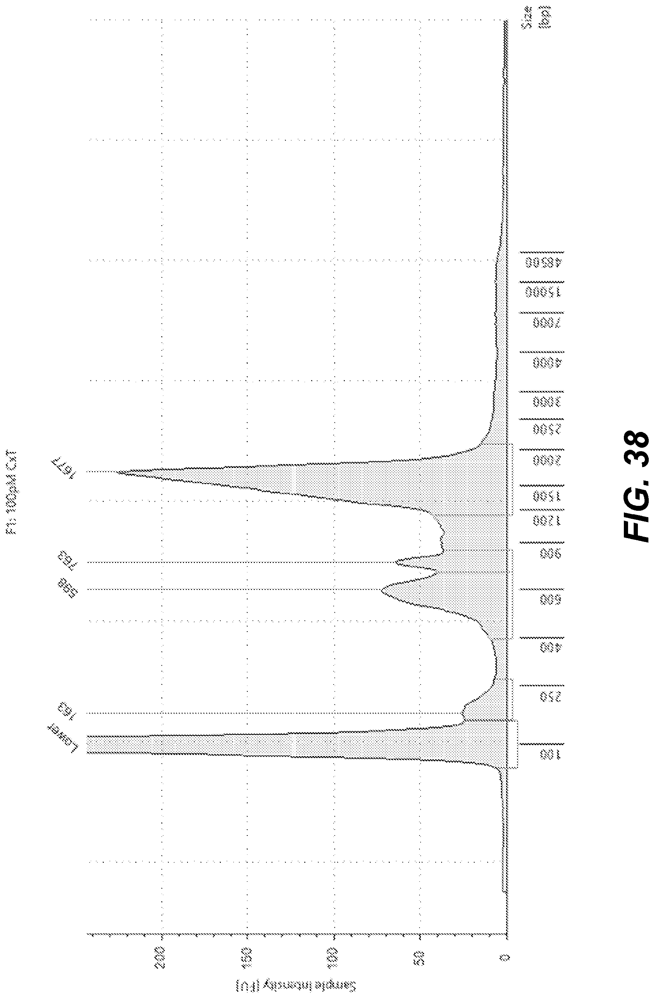

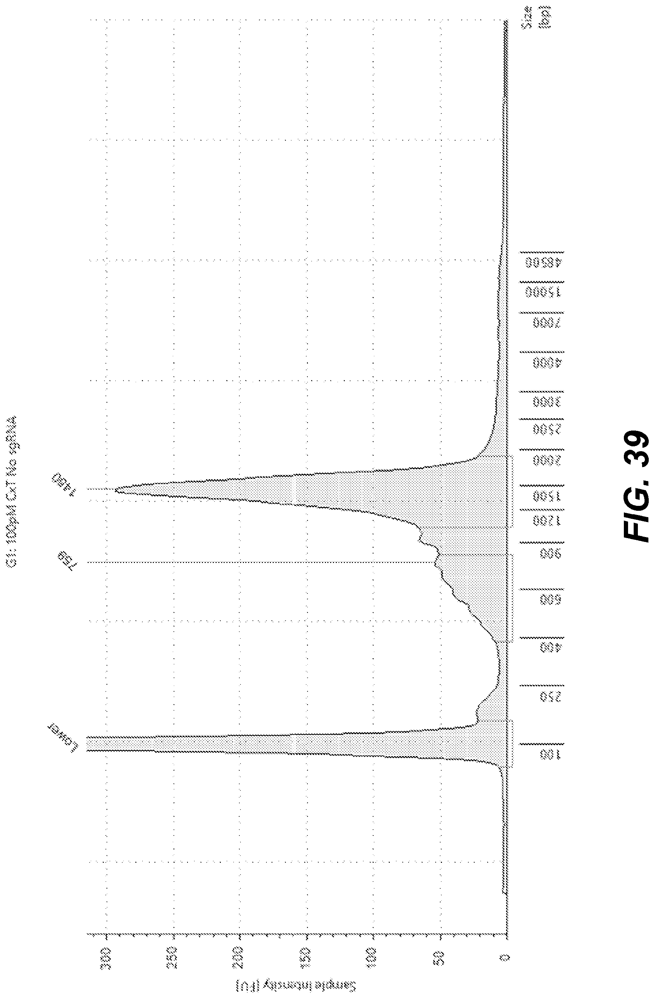

FIG. 38-FIG. 39 depict guided tagmentation using *S. enterica* sgRNA on dCas9-xTen-Tn5. This data shows that the addition of sgRNA adds specificity. FIG. 39 shows that guided tagmentation with no sgRNA is random. FIG. 38 shows that adding sgRNA confers specificity.

### Example 2

### Sample library preparation

### Guided Tagmentation Libraries

Described herein are methods and compositions for generating libraries for sequencing on the Illumina NextSeq.

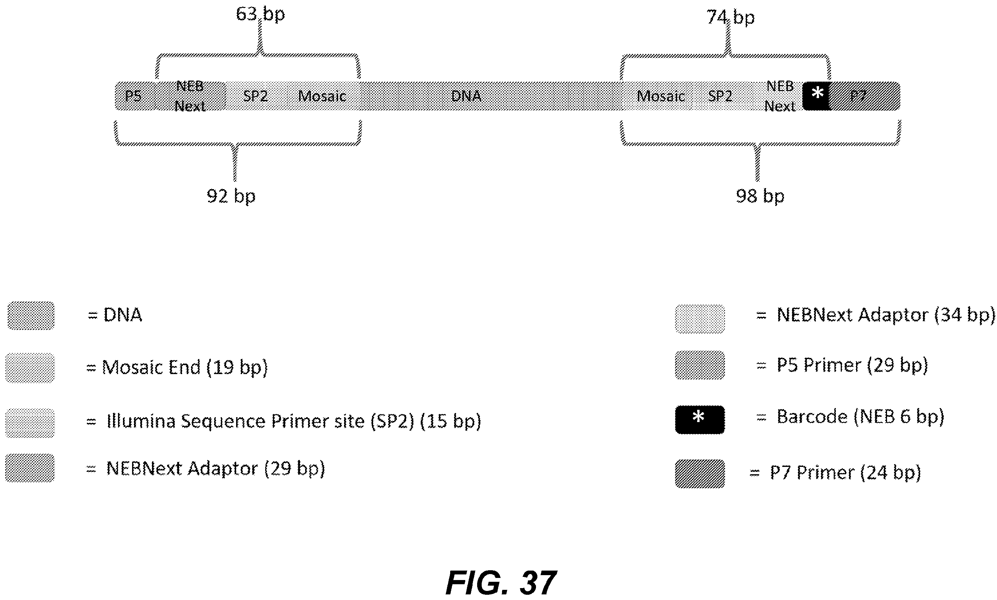

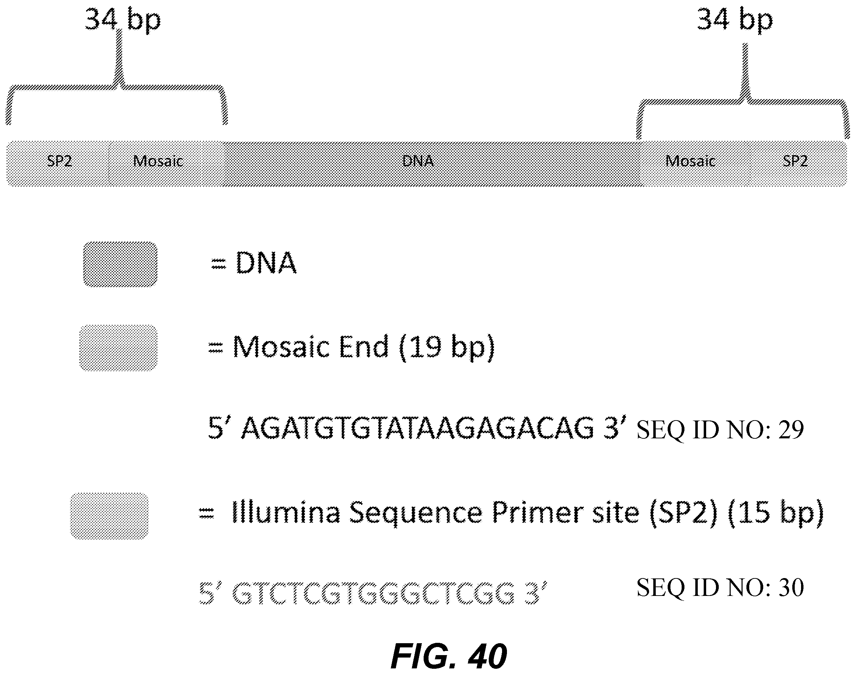

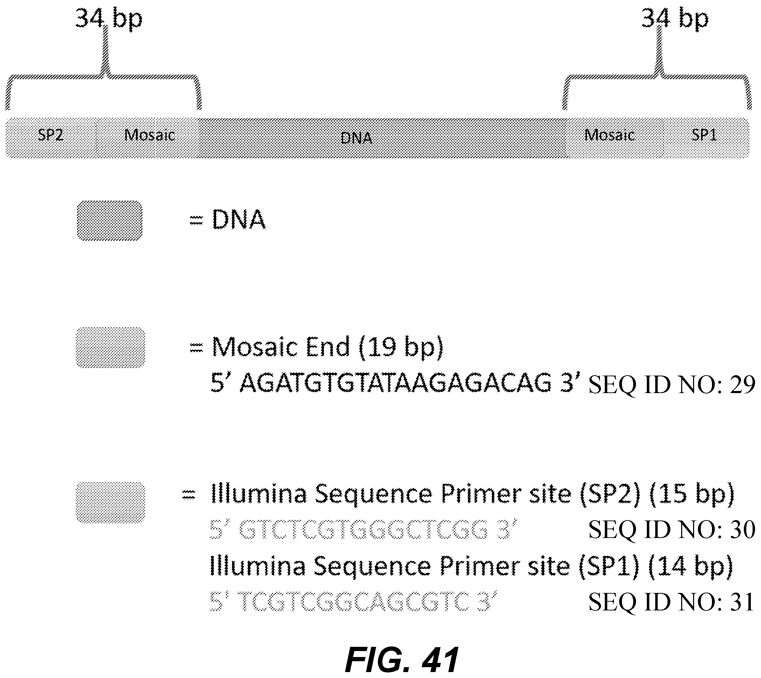

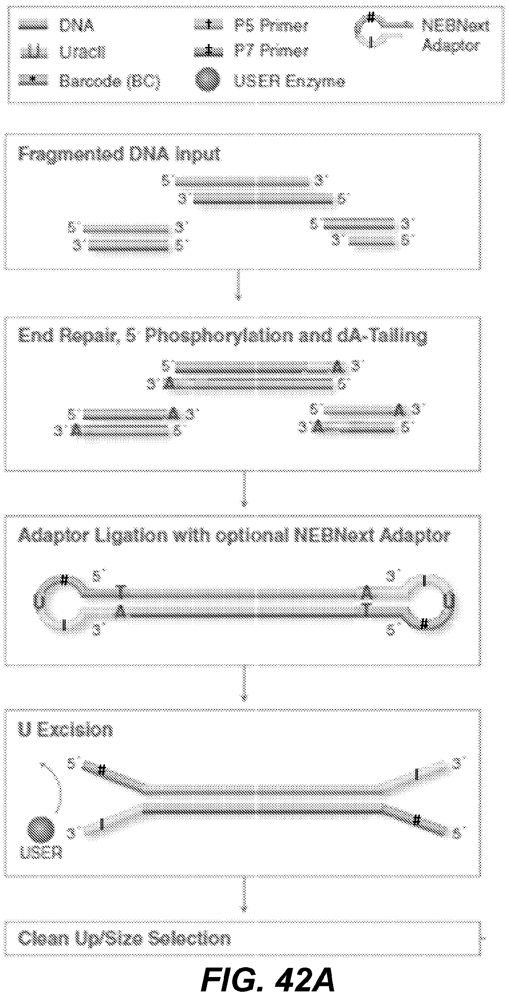

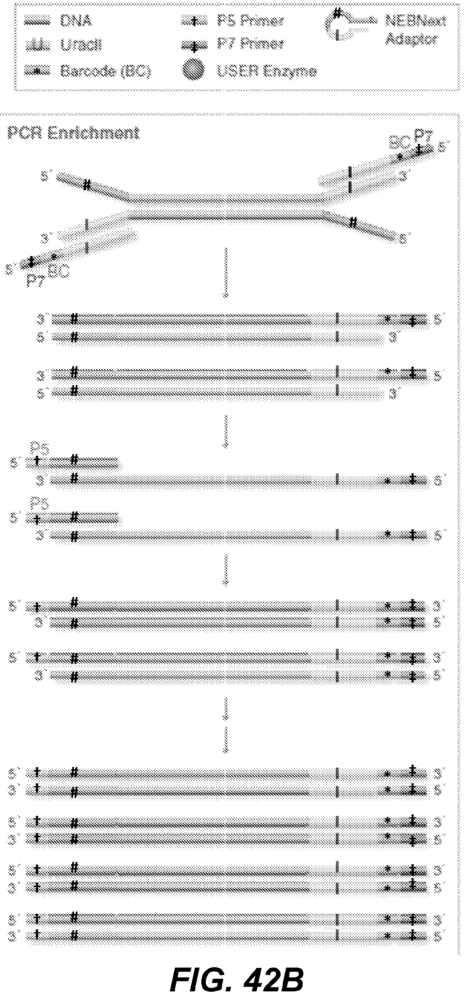

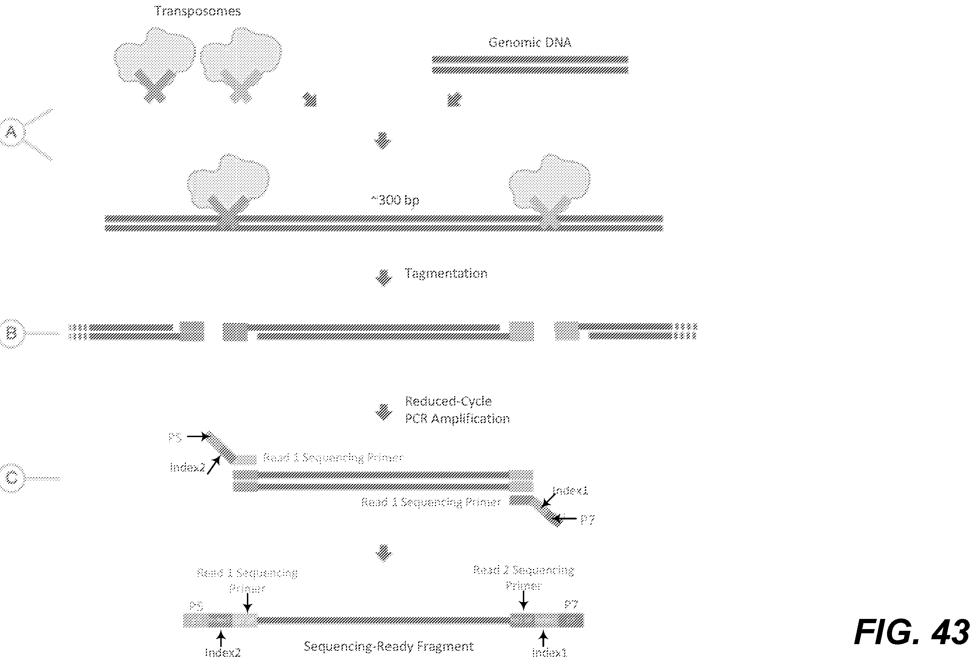

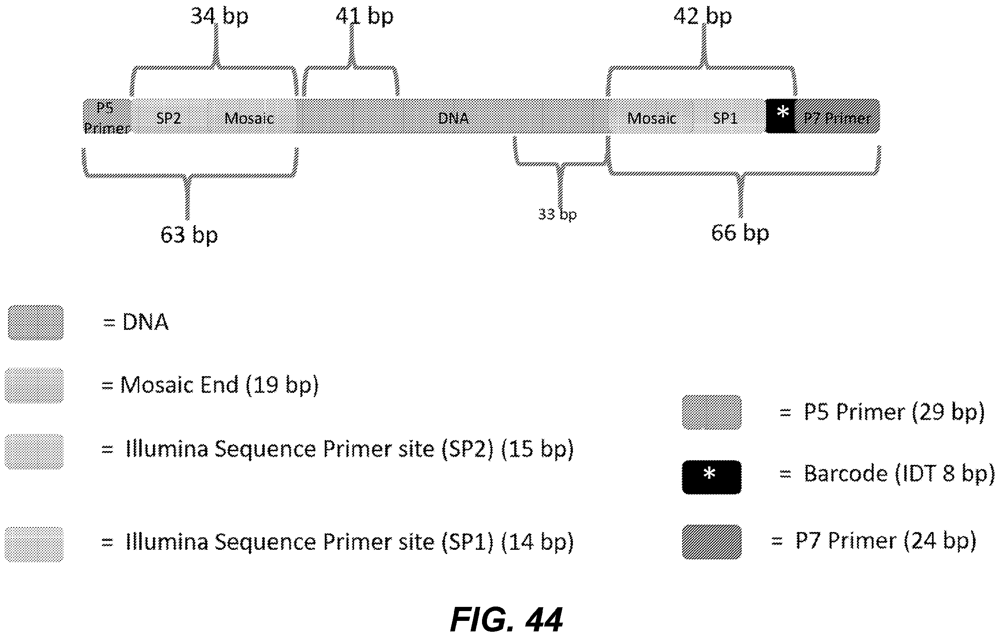

Three libraries were made with ligation-based method (FIG. 37, FIG. 40, FIG. 42A-FIG. 42B), in which NEBNext sequencing adaptors were added following the tagmentation step using a single adaptor (e.g., Adaptor B, using either Tn5 alone or dCas9-Tn5 fusion) and two libraries were made with guided tagmentation based method (FIG. 41, FIG. 43-FIG. 44), in which the sequences required for NGS are included in the guided tagmentation step on Adaptors A and B. All libraries were prepared using Human sgRNA. dCas9-Fl26-Tn5 fusion protein was used for guided tagmentation. In these experiments, DNA was incubated with dCas-Tn5 under long or short incubation protocols. For the short protocol, reactions were incubated for 30 minutes at 30°C and then for 30 minutes at 37°C. For the long protocol, reactions were incubated at 30°C for 30 minutes, followed by 38°C for 60 minutes and then 55°C for 60 minutes.

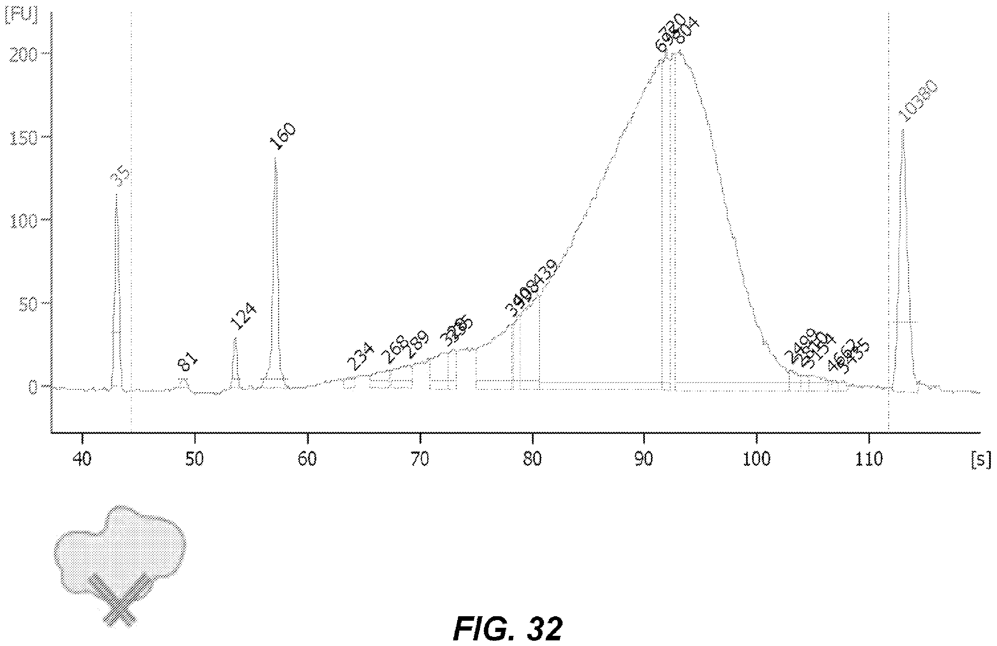

FIG. 32 shows Highly Multiplexed Single Primer DNA Amplification using Tn5 only. Bioanalyzer analysis indicates Nonspecific DNA amplification by PCR showing that DNA can be amplified using only 1 primer (Adaptor B).

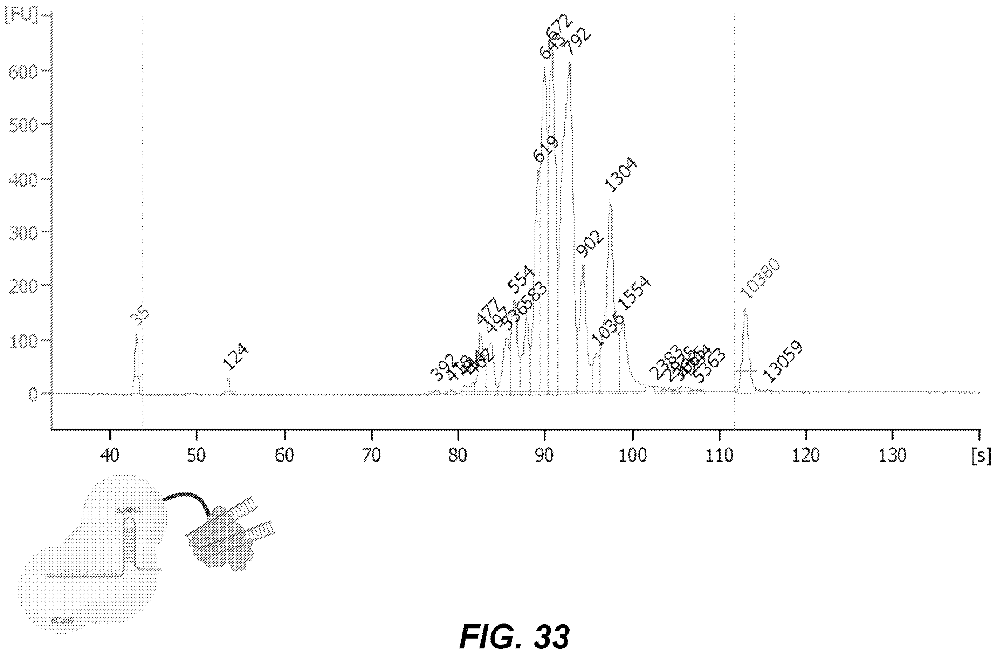

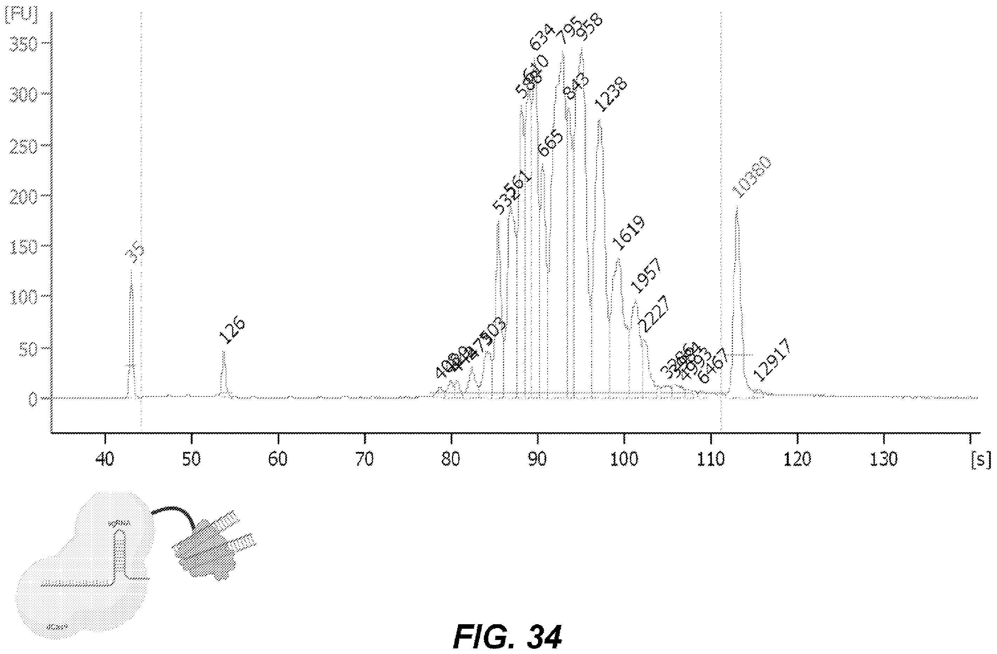

Evidence to support Highly Multiplexed Single Primer DNA Amplification using dCas9-Tn fusion protein is shown in FIG. 33 (short incubation protocol) and FIG. 34 (longer incubation protocol). Bioanalyzer analysis of PCR amplification showed simultaneous specific amplification of several DNA fragments using only 1 primer (Adaptor B).

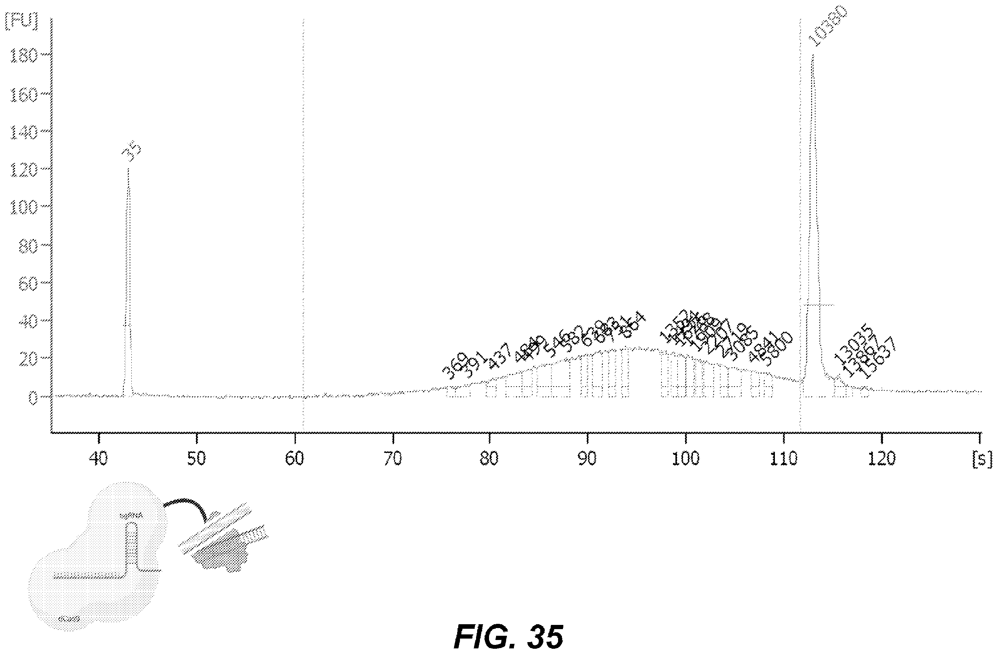

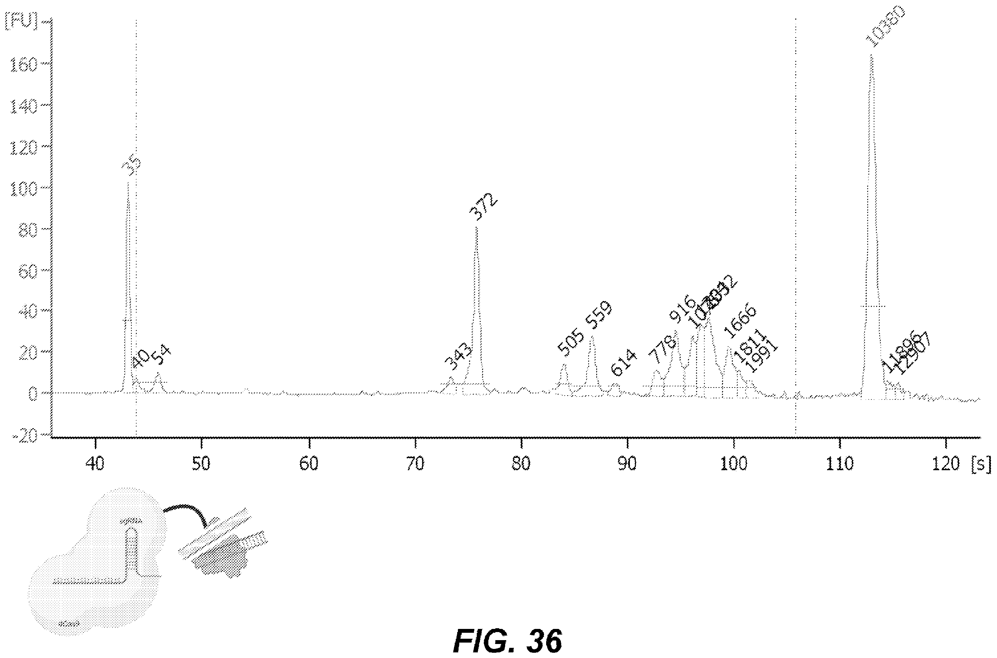

Evidence to support Customized Loci-Specific Sequencing Library Preparation is shown in FIG. 35 (longer incubation protocol) and FIG. 36 (shorter incubation protocol). Bioanalyzer analysis shows that a Sequencing Library can be created. Adding adaptors A and B, which are required for sequencing in the Illumina platform, shows that a Sequencing Library can be created using guided tagmentation.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.,* bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting.

## Claims

1. A composition, comprising a plurality of protein complexes, wherein each of the plurality of protein complexes comprises a transposome and a programmable DNA binding unit capable of specifically binding to a binding site on a target double-stranded DNA (dsDNA), wherein the transposome comprises a transposase, a first adaptor and a second adaptor, wherein the first adaptor is a sequencing adaptor and wherein the second adaptor is a sequencing adaptor, and wherein the binding site for each of the plurality of protein complexes is different from each other.

2. The composition of claim 1,
(i) wherein at least two of the plurality of protein complexes comprise the same transposome;
(ii) wherein all of the plurality of protein complexes comprise the same transposome; and/or
(iii) wherein all of the plurality of protein complexes comprise the same transposase.

3. The composition of any one of claims 1-2,
(i) wherein the first adaptor and the second adaptor in the same transposome are the same;
(ii) wherein the first adaptor, the second adaptor, or both, in different transposome are different;
(iii) wherein the first adaptor, the second adaptor, or both, is a dsDNA or an RNA/DNA duplex; and/or
(iv) wherein the adaptor is about 3-200 base pairs in length; and/or
(v) wherein the sequencing adaptor comprises P5 or P7 primer sequences.

4. The composition of any one of claims 1-3,
(i) wherein the binding sites of at least two of the plurality of protein complexes are on the same target dsDNA, optionally wherein
(a) the binding sites of at least two of the plurality of protein complexes are about 1-50000 nucleotides apart on the same target dsDNA,
(b) the distance between the binding sites of a pair of the plurality of protein complexes is substantially the same as the distance between the binding sites of another pair of the plurality of protein complexes, and/or
(c) the distance between the binding sites of a pair of the plurality of protein complexes is different from the distance between the binding sites of another pair of the plurality of protein complexes; and/or
(ii) wherein the binding sites of at least two of the plurality of protein complexes are on the different strand of a target dsDNA.

5. The composition of any one of claims 1-4,
(i) wherein at least two of the plurality of protein complexes are capable of specifically binding to different target dsDNA, and/or
(ii) wherein the plurality of protein complex are capable of specifically binding to about 2-5000 target dsDNA.

6. The composition of any one of claims 1-5, wherein the transposase is (i) Tn5 transposase, Tn7 transposase, mariner Tc1-like transposase, Himar1C9 transposase, or Sleeping Beauty transposase; and/or (ii) a hyperactive transposase.

7. The composition of any one of claims 1-6, wherein the programmable DNA binding unit comprises:
(1) a nuclease-deficient CRISPR associated protein (dCAS protein) and a guide RNA (gRNA) capable of specifically binding to the binding site of the target dsDNA, optionally wherein
(i) the transposome is associated with the programmable DNA binding unit via a linker connecting the transposase and the dCAS protein, further optionally wherein the linker comprises a peptide linker, a chemical linker, or both,
(ii) the transposase is present in a fusion protein comprising the dCAS protein, and/or
(iii) the dCAS protein is dCAS9, dCAS12, dCAS13, dCAS14, or SpRY dCAS, further optionally wherein the dCAS13 protein is dCAS13a, dCAS13b, dCAS13c, or dCAS13d; or
(2) a protein component capable of specifically binding to the binding site on the target dsDNA, wherein the protein component comprises an endonuclease-deficient zinc finger nuclease (ZFN), an endonuclease-deficient transcription activator-like effector nuclease (TALEN), Argonaute protein, an endonuclease-deficient meganuclease, a recombinase, or a combination thereof, optionally wherein
(i) the transposome is associated with the programmable DNA binding unit via a linker connecting the transposase and the protein component,
further optionally wherein the linker comprises a peptide linker, a chemical linker, or both, further optionally wherein the peptide linker:
(a) comprises multiple glycine, serine, threonine, alanine, lysine, glutamine, or a combination thereof, further optionally wherein the peptide linker comprises a GS linker, and/or
(b) is a XTEN linker; and/or
(ii) the protein component is present in a fusion protein comprising the transposase.

8. A reaction mixture, comprising
the composition of any one of claims 1-7; and
sample nucleic acids suspected of comprising one or more target dsDNA.

9. The reaction mixture of claim 8,
(i) wherein the reaction mixture further comprises a DNA polymerase, dNTPs, or a combination thereof,
(ii) wherein the adaptor is covalently attached to the target dsDNA or a fragment thereof,
(iii) wherein the reaction mixture comprises a plurality of dsDNA fragments each comprising the first adaptor and the second adaptor of one of the plurality of protein complexes at each terminus respectively,
(iv) wherein the sample nucleic acids comprises eukaryotic DNA, bacterial DNA, viral DNA, fungal DNA, protozoa DNA, or a combination thereof,
(v) wherein the target dsDNA is genomic DNA, mitochondrial DNA, plasmid DNA, or a combination thereof, and/or
(vi) wherein the sample nucleic acids are from a biological sample, a clinical sample, an environmental sample, or a combination thereof, optionally wherein the biological sample comprises stool, sputum, peripheral blood, plasma, serum, lymph nodes, respiratory tissue, exudates, bodily fluid, or a combination thereof.

10. A method for tagging nucleic acids, comprising:
contacting the composition of any one of claims 1-7 with a sample suspected of comprising a plurality of target double-stranded DNA (dsDNA) to form a reaction mixture; and
incubating the reaction mixture to generate a plurality of dsDNA fragments each comprising the first adaptor and the second adaptor of one of the plurality of protein complexes at each terminus respectively.

11. A method for generating a sequencing library, comprising:
contacting the composition of any one of claims 1-7 with a sample suspected of comprising a plurality of target double-stranded DNA (dsDNA) to form a reaction mixture;
incubating the reaction mixture to generate a plurality of dsDNA fragments each comprising the first adaptor and the second adaptor of one of the plurality of protein complexes at each terminus respectively; and
amplifying the plurality of dsDNA fragments with primers capable of binding to the adaptors at the termini of the dsDNA fragments to generate a sequencing library, optionally wherein each of the primers is about 5-80 nucleotides in length.

12. The method of claim 11, wherein amplifying the plurality of dsDNA fragments with the primer is carried out using polymerase chain reaction (PCR), optionally wherein the PCR is:
(i) loop-mediated isothermal Amplification (LAMP), helicase-dependent Amplification (HDA), recombinase polymerase amplification (RPA), strand displacement amplification (SDA), nucleic acid sequence-based amplification (NASBA), transcription mediated amplification (TMA), nicking enzyme amplification reaction (NEAR), rolling circle amplification (RCA), multiple displacement amplification (MDA), Ramification (RAM), circular helicase dependent amplification (cHDA), single primer isothermal amplification (SPIA), signal mediated amplification of RNA technology (SMART), self-sustained sequence replication (3SR), genome exponential amplification reaction (GEAR), or isothermal multiple displacement amplification (IMDA), and/or
(ii) real-time PCR or quantitative real-time PCR (QRT-PCR).

13. The method of any one of claims 10-12,
(i) wherein the sample
(a) comprises eukaryotic DNA, bacterial DNA, viral DNA, fungal DNA, protozoa DNA, or a combination thereof, and/or
(b) is, or is derived from, a biological sample, a clinical sample, an environmental sample, or a combination thereof;
(ii) wherein the method further comprises generating the plurality of target dsDNA from a plurality of target RNA with a reverse transcriptase; and/or
(iii) wherein the plurality of target dsDNA comprises:
(a) genomic DNA, mitochondrial DNA, plasmid DNA, or a combination thereof,
(b) DNA from at least 2 different organisms,
(c) DNA from at least 2 different genes, and/or
(d) target dsDNA generated from target RNA with a reverse transcriptase.

14. The method of any one of claims 10-13, wherein the plurality of target dsDNA comprises a genetic signature of interest, optionally wherein the genetic signature of interest
(i) comprises one or more mutations of interest, further optionally wherein the one or more mutations of interest comprise:
(a) point mutation, inversion, deletion, insertion, translocation, duplication, copy number variation, or a combination thereof, and/or
(b) nucleotide substitution, deletion, insertion, or a combination thereof;
(ii) is indicative of pathogen identification, antibiotic resistance or antibiotic susceptibility of the organism from which the target dsDNA is derived;
(iii) is indicative of cancer status of the organism from which the target dsDNA is derived; and/or
(iv) is indicative of the status of a genetic disease of the organism from which the target dsDNA is derived, further optionally wherein the genetic disease is:
(a) single-gene disorder, and/or
(b) cystic fibrosis, Huntington's disease, sickle cell anemia, hemophilia, Duchenne muscular dystrophy, Thalassemia, Fragile X syndrome, familial hypercholesterolemia, polycystic kidney disease, neurofibromatosis type I, hereditary spherocytosis, Marfan syndrome, Tay-Sachs disease, phenylketonuria, mucopolysaccharidoses, lysosomal acid lipase deficiency, glycogen storage diseases, galactosemia, or hemochromatosis.

15. The method of any one of claims 10-14,
(i) wherein contacting the plurality of target dsDNA with the plurality of protein complex pairs is carried out at about 25°C to about 80°C;
(ii) wherein incubating the reaction mixture comprises incubating the reaction mixture at about 37°C to about 55°C;
(iii) wherein the plurality of protein complex pairs and the plurality of target dsDNA are present in the reaction mixture at a molecular ratio of about 2:1 to about 2,000:1, optionally at a molecular ratio of about 2:1 to about 200:1; and/or
(iv) wherein the method further comprises labeling one or both ends of one or more of the plurality of dsDNA fragments, optionally wherein the method comprises labeling the two ends of one or more of the plurality of dsDNA fragments differently, further optionally wherein
(a) the labeling comprises labeling with anionic labels, cationic labels, neutral labels, electrochemical labels, protein labels, fluorescent labels, magnetic labels, or a combination thereof, and/or
(b) the method further comprises enriching the labeled dsDNA fragments, capturing the labeled dsDNA fragments, isolating the labeled dsDNA fragments, and/or visualizing the labeled dsDNA fragments.

## Patentansprüche

1. Zusammensetzung, umfassend eine Vielzahl von Proteinkomplexen, wobei jeder der Vielzahl von Proteinkomplexen ein Transposom und eine programmierbare DNA-Bindungseinheit umfasst, die in der Lage ist, spezifisch an eine Bindungsstelle auf einer Ziel-Doppelstrang-DNA (dsDNA) zu binden, wobei das Transposom eine Transposase, einen ersten Adapter und einen zweiten Adapter umfasst, wobei der erste Adapter ein Sequenzieradapter ist und wobei der zweite Adapter ein Sequenzieradapter ist, und wobei die Bindungsstelle für jeden aus der Vielzahl von Proteinkomplexen voneinander verschieden ist.

2. Zusammensetzung nach Anspruch 1,
(i) wobei mindestens zwei aus der Vielzahl von Proteinkomplexen das gleiche Transposom umfassen;
(ii) wobei alle aus der Vielzahl von Proteinkomplexen das gleiche Transposom umfassen; und/oder
(iii) wobei alle aus der Vielzahl von Proteinkomplexen die gleiche Transposase umfassen.

3. Zusammensetzung nach einem der Ansprüche 1-2,
(i) wobei der erste Adapter und der zweite Adapter in demselben Transposom gleich sind;
(ii) wobei der erste Adapter, der zweite Adapter oder beide in verschiedenen Transposomen unterschiedlich sind;
(iii) wobei es sich bei dem ersten Adapter, dem zweiten Adapter, oder beiden, um eine dsDNA oder einen RNA/DNA-Duplex handelt; und/oder
(iv) wobei der Adapter etwa 3-200 Basenpaare lang ist; und/oder
(v) wobei der Sequenzieradapter P5- oder P7-Primersequenzen umfasst.

4. Zusammensetzung nach einem der Ansprüche 1-3,
(i) wobei sich die Bindungsstellen von mindestens zwei aus der Vielzahl von Proteinkomplexen auf derselben Ziel-dsDNA befinden, gegebenenfalls wobei
(a) die Bindungsstellen von mindestens zwei aus der Vielzahl von Proteinkomplexen etwa 1-50000 Nukleotide auf derselben Ziel-dsDNA voneinander entfernt liegen,
(b) der Abstand zwischen den Bindungsstellen eines Paars der Vielzahl von Proteinkomplexen im Wesentlichen gleich dem Abstand zwischen den Bindungsstellen eines anderen Paars der Vielzahl von Proteinkomplexen ist, und/oder
(c) der Abstand zwischen den Bindungsstellen eines Paars der Vielzahl von Proteinkomplexen sich von dem Abstand zwischen den Bindungsstellen eines anderen Paars der Vielzahl von Proteinkomplexen unterscheidet; und/oder
(ii) wobei sich die Bindungsstellen von mindestens zwei aus der Vielzahl von Proteinkomplexen auf dem unterschiedlichen Strang einer Ziel-dsDNA befinden.

5. Zusammensetzung nach einem der Ansprüche 1-4,
(i) wobei mindestens zwei aus der Vielzahl von Proteinkomplexen in der Lage sind, spezifisch an unterschiedliche Ziel-dsDNA zu binden, und/oder
(ii) wobei die Vielzahl von Proteinkomplexen in der Lage ist, spezifisch an etwa 2-5000 Ziel-dsDNAs zu binden.

6. Zusammensetzung nach einem der Ansprüche 1-5, wobei die Transposase (i) Tn5-Transposase, Tn7-Transposase, Mariner-Tc1-ähnliche Transposase, Himar1C9-Transposase oder Sleeping-Beauty-Transposase; und/oder (ii) eine hyperaktive Transposase ist.

7. Zusammensetzung nach einem der Ansprüche 1-6, wobei die programmierbare DNA-Bindungseinheit umfasst:
(1) ein Nuklease-defizientes CRISPR-assoziiertes Protein (dCAS-Protein) und eine Guide-RNA (gRNA), die in der Lage ist, spezifisch an die Bindungsstelle der Ziel-dsDNA zu binden, wobei optional
(i) das Transposom mit der programmierbaren DNA-Bindungseinheit über einen Linker assoziiert ist, der die Transposase und das dCAS-Protein verbindet, wobei der Linker ferner optional einen Peptidlinker, einen chemischen Linker oder beides umfasst,
(ii) die Transposase in einem Fusionsprotein vorhanden ist, das das dCAS-Protein umfasst, und/oder
(iii) das dCAS-Protein dCAS9, dCAS12, dCAS13, dCAS14 oder SpRY-dCAS ist, ferner optional wobei das dCAS13-Protein dCAS13a, dCAS13b, dCAS13c oder dCAS13d ist; oder
(2) eine Proteinkomponente, die in der Lage ist, spezifisch an die Bindungsstelle auf der Ziel-dsDNA zu binden, wobei die Proteinkomponente eine Endonuklease-defiziente Zinkfingernuklease (ZFN), eine Endonuklease-defiziente Transkriptionsaktivator-ähnliche-Effektornuklease (TALEN), Argonaute-Protein, eine Endonuklease-defiziente Meganuklease, eine Rekombinase oder eine Kombination davon umfasst, optional wobei
(i) das Transposom über einen Linker, der die Transposase und die Proteinkomponente verbindet, mit der programmierbaren DNA-Bindungseinheit assoziiert ist, ferner optional wobei der Linker einen Peptidlinker, einen chemischen Linker oder beides umfasst, ferner optional wobei der Peptidlinker:
(a) mehrere Glycin, Serin, Threonin, Alanin, Lysin, Glutamin oder eine Kombination davon umfasst, ferner optional wobei der Peptidlinker einen GS-Linker umfasst, und/oder
(b) ein XTEN-Linker ist; und/oder
(ii) die Proteinkomponente in einem Fusionsprotein vorhanden ist, das die Transposase umfasst.

8. Reaktionsmischung, umfassend
die Zusammensetzung nach einem der Ansprüche 1-7; und Probennukleinsäuren, von denen vermutet wird, dass sie eine oder mehrere Ziel-dsDNAs umfassen.

9. Reaktionsmischung nach Anspruch 8,
(i) wobei die Reaktionsmischung ferner eine DNA-Polymerase, dNTPs oder eine Kombination davon umfasst,
(ii) wobei der Adapter kovalent an die Ziel-dsDNA oder ein Fragment davon angeheftet ist,
(iii) wobei die Reaktionsmischung eine Vielzahl an dsDNA-Fragmenten umfasst, die jeweils den ersten Adapter und den zweiten Adapter von je einem aus der Vielzahl von Proteinkomplexen an jedem Terminus umfassen,
(iv) wobei die Probennukleinsäuren eukaryotische DNA, bakterielle DNA, virale DNA, Pilz-DNA, Protozoen-DNA oder eine Kombination davon umfassen,
(v) wobei die Ziel-dsDNA genomische DNA, mitochondriale DNA, Plasmid-DNA oder eine Kombination davon ist, und/oder
(vi) wobei die Probennukleinsäuren aus einer biologischen Probe, einer klinischen Probe, einer Umweltprobe oder einer Kombination davon stammen, wobei die biologische Probe gegebenenfalls Stuhl, Sputum, peripheres Blut, Plasma, Serum, Lymphknoten, Atemwegsgewebe, Exsudate, Körperflüssigkeit oder eine Kombination davon umfasst.

10. Verfahren zum Taggen von Nukleinsäuren, umfassend:
Inkontaktbringen der Zusammensetzung nach einem der Ansprüche 1-7 mit einer Probe, von der vermutet wird, dass sie eine Vielzahl von Ziel-Doppelstrang-DNA (dsDNA) umfasst, um eine Reaktionsmischung zu bilden; und
Inkubieren der Reaktionsmischung, um eine Vielzahl von dsDNA-Fragmenten zu erzeugen, die jeweils den ersten Adapter und den zweiten Adapter von je einem aus der Vielzahl von Proteinkomplexen an jedem Terminus umfassen.

11. Verfahren zum Erzeugen einer Sequenzierungsbibliothek, das Folgendes umfasst:
Inkontaktbringen der Zusammensetzung nach einem der Ansprüche 1-7 mit einer Probe, von der vermutet wird, dass sie eine Vielzahl von Ziel-Doppelstrang-DNA (dsDNA) umfasst, um eine Reaktionsmischung zu bilden;
Inkubieren der Reaktionsmischung, um eine Vielzahl von dsDNA-Fragmenten zu erzeugen, die jeweils den ersten Adapter und den zweiten Adapter von je einem aus der Vielzahl von Proteinkomplexen an jedem Terminus umfassen; und
Amplifizieren der Vielzahl von dsDNA-Fragmenten mit Primern, die in der Lage sind, an die Adaptoren an den Termini der dsDNA-Fragmente zu binden, um eine Sequenzierungsbibliothek zu erzeugen, wobei optional jeder der Primer etwa 5-80 Nukleotide lang ist.

12. Verfahren nach Anspruch 11, wobei das Amplifizieren der Vielzahl von dsDNA-Fragmenten mit dem Primer unter Verwendung einer Polymerasekettenreaktion (PCR) durchgeführt wird, wobei es sich bei der PCR optional handelt um:
(i) Loop-vermittelte isotherme Amplifikation (LAMP), Helikase-abhängige Amplifikation (HDA), Rekombinase-Polymerase-Amplifikation (RPA), Strangverdrängungsamplifikation (SDA), Nukleinsäuresequenz-basierte Amplifikation (NASBA), transkriptionsvermittelte Amplifikation (TMA), Nicking-Enzym-Amplifikationsreaktion (NEAR), Rolling-Circle-Amplifikation (RCA), multiple Verdrängungsamplifikation (MDA), Ramifikation (RAM), zirkuläre Helikase-abhängige Amplifikation (cHDA), isotherme Einzelprimer-Amplifikation (SPIA), signalvermittelte Amplifikation der RNA-Technologie (SMART), selbst-tragende Sequenzreplikation (3SR), exponentielle Genomamplifikationsreaktion (GEAR) oder isotherme multiple Verdrängungsamplifikation (IMDA), und/oder
(ii) Echtzeit-PCR oder quantitative Echtzeit-PCR (QRT-PCR) .

13. Verfahren nach einem der Ansprüche 10-12,
(i) wobei die Probe
(a) eukaryotische DNA, bakterielle DNA, virale DNA, Pilz-DNA, Protozoen-DNA oder eine Kombination davon umfasst, und/oder
(b) eine biologische Probe, eine klinische Probe, eine Umweltprobe oder eine Kombination davon ist oder davon abgeleitet ist;
(ii) wobei das Verfahren ferner das Erzeugen der Vielzahl von Ziel-dsDNA aus einer Vielzahl von Ziel-RNA mit einer reversen Transkriptase umfasst; und/oder
(iii) wobei die Vielzahl von Ziel-dsDNA Folgendes umfasst:
(a) genomische DNA, mitochondriale DNA, Plasmid-DNA oder eine Kombination davon,
(b) DNA aus mindestens 2 verschiedenen Organismen,
(c) DNA aus mindestens 2 verschiedenen Genen und/oder
(d) Ziel-dsDNA, die aus Ziel-RNA mit einer reversen Transkriptase erzeugt wird.

14. Verfahren nach einem der Ansprüche 10-13, wobei die Vielzahl von Ziel-dsDNA eine genetische Signatur von Interesse umfasst, optional wobei die genetische Signatur von Interesse
(i) eine oder mehrere interessierende Mutationen umfasst, wobei ferner optional die eine oder die mehreren interessierenden Mutationen Folgendes umfassen:
(a) Punktmutation, Inversion, Deletion, Insertion, Translokation, Duplikation, Variation der Kopienzahl oder eine Kombination davon, und/oder
(b) Nukleotidsubstitution, -deletion, -insertion oder eine Kombination davon;
(ii) indikativ für Pathogenidentifizierung, Antibiotikaresistenz oder Antibiotikaempfänglichkeit bei dem Organismus ist, aus dem die Ziel-dsDNA stammt;
(iii) indikativ für den Krebsstatus des Organismus ist, aus dem die Ziel-dsDNA stammt; und/oder
(iv) indikativ für den Status einer genetischen Erkrankung bei dem Organismus ist, aus dem die Ziel-dsDNA stammt, wobei es sich bei der genetischen Erkrankung ferner optional handelt um:
(a) Einzel-Gen-Störung und/oder
(b) zystische Fibrose, Huntington-Krankheit, Sichelzellenanämie, Hämophilie, Duchenne-Muskeldystrophie, Thalassämie, fragiles X-Syndrom, familiäre Hypercholesterinämie, polyzystische Nierenerkrankung, Neurofibromatose Typ I, hereditäre Sphärozytose, Marfan-Syndrom, Tay-Sachs-Krankheit, Phenylketonurie, Mucopolysaccharidosen, Lysosomalesaure-Lipase-Defizienz, Glykogenspeicherkrankheiten, Galactosämie oder Hämochromatose.

15. Verfahren nach einem der Ansprüche 10-14,
(i) wobei das Inkontaktbringen der Vielzahl von Ziel-dsDNAs mit der Vielzahl von Proteinkomplexpaaren bei etwa 25 °C bis etwa 80 °C durchgeführt wird;
(ii) wobei das Inkubieren der Reaktionsmischung das Inkubieren der Reaktionsmischung bei etwa 37 °C bis etwa 55 °C umfasst;
(iii) wobei die Vielzahl von Proteinkomplexpaaren und die Vielzahl von Ziel-dsDNA in der Reaktionsmischung in einem Molekülverhältnis von etwa 2:1 bis etwa 2.000:1, gegebenenfalls in einem Molekülverhältnis von etwa 2:1 bis etwa 200:1, vorhanden sind; und/oder
(iv) wobei das Verfahren ferner das Markieren eines oder beider Enden eines oder mehrerer der Vielzahl von dsDNA-Fragmenten umfasst, wobei das Verfahren optional unterschiedliches Markieren der zwei Enden eines oder mehrerer der Vielzahl von dsDNA-Fragmenten umfasst, wobei ferner optional
(a) das Markieren das Markieren mit anionischen Markierungen, kationischen Markierungen, neutralen Markierungen, elektrochemischen Markierungen, Proteinmarkierungen, fluoreszierenden Markierungen, magnetischen Markierungen oder eine Kombination davon umfasst, und/oder
(b) das Verfahren ferner Anreichern der markierten dsDNA-Fragmente, Einfangen der markierten dsDNA-Fragmente, Isolieren der markierten dsDNA-Fragmente und/oder Visualisieren der markierten dsDNA-Fragmente umfasst.

## Revendications

1. Composition, comprenant une pluralité de complexes protéiques, dans laquelle chacun de la pluralité de complexes protéiques comprend un transposome et une unité de liaison à l'ADN programmable capable de se lier spécifiquement à un site de liaison sur un ADN double brin (ADNdb) cible, dans laquelle le transposome comprend une transposase, un premier adaptateur et un second adaptateur, dans laquelle le premier adaptateur est un adaptateur de séquençage et dans laquelle le second adaptateur est un adaptateur de séquençage, et dans laquelle le site de liaison pour chacun de la pluralité de complexes protéiques est différent les uns des autres.

2. Composition selon la revendication 1,
(i) dans laquelle au moins deux de la pluralité de complexes protéiques comprennent le même transposome ;
(ii) dans laquelle tous de la pluralité de complexes protéiques comprennent le même transposome ; et/ou
(iii) dans laquelle tous de la pluralité de complexes protéiques comprennent la même transposase.

3. Composition selon l'une quelconque des revendications 1 ou 2,
(i) dans laquelle le premier adaptateur et le deuxième adaptateur dans le même transposome sont les mêmes ;
(ii) dans laquelle le premier adaptateur, le second adaptateur, ou les deux, dans un transposome différent sont différents ;
(iii) dans laquelle le premier adaptateur, le deuxième adaptateur, ou les deux, est un ADNdb ou un duplex ARN/ADN ; et/ou
(iv) dans laquelle l'adaptateur a une longueur d'environ 3 à 200 paires de bases ; et/ou
(v) dans laquelle l'adaptateur de séquençage comprend des séquences d'amorces P5 ou P7.

4. Composition selon l'une quelconque des revendications 1 à 3,
(i) les sites de liaison d'au moins deux de la pluralité de complexes protéiques sont sur le même ADNdb cible, éventuellement dans laquelle
(a) les sites de liaison d'au moins deux de la pluralité de complexes protéiques sont distants d'environ 1 à 50 000 nucléotides sur le même ADNdb cible,
(b) la distance entre les sites de liaison d'une paire de la pluralité de complexes protéiques est sensiblement la même que la distance entre les sites de liaison d'une autre paire de la pluralité de complexes protéiques, et/ou
(c) la distance entre les sites de liaison d'une paire de la pluralité de complexes protéiques est différente de la distance entre les sites de liaison d'une autre paire de la pluralité de complexes protéiques ; et/ou
(ii) dans laquelle les sites de liaison d'au moins deux de la pluralité de complexes protéiques sont sur le brin différent d'un ADNdb cible.

5. Composition selon l'une quelconque des revendications 1 à 4,
(i) dans laquelle au moins deux de la pluralité de complexes protéiques sont capables de se lier spécifiquement à un ADNdb cible différent, et/ou
(ii) dans laquelle la pluralité de complexe protéique sont capables de se lier spécifiquement à environ 2 à 5 000 ADNdb cibles.

6. Composition selon l'une quelconque des revendications 1 à 5, dans laquelle la transposase est (i) la transposase Tn5, la transposase Tn7, la transposase de type mariner Tc1, la transposase Himar1C9 ou la transposase Sleeping Beauty ; et/ou (ii) une transposase hyperactive.

7. Composition selon l'une quelconque des revendications 1 à 6, dans laquelle l'unité de liaison d'ADN programmable comprend :
(1) une protéine associée à CRISPR déficiente en nucléase (protéine dCAS) et un ARN guide (ARNg) capable de se lier spécifiquement au site de liaison de l'ADNdb cible, éventuellement dans laquelle
(i) le transposome est associé à l'unité de liaison d'ADN programmable par l'intermédiaire d'un lieur reliant la transposase et la protéine dCAS, et éventuellement dans laquelle le lieur comprend un lieur peptidique, un lieur chimique, ou les deux,
(ii) la transposase est présente dans une protéine de fusion comprenant la protéine dCAS, et/ou
(iii) la protéine dCAS est dCAS9, dCAS12, dCAS13, dCAS14, ou SpRY dCAS, en outre éventuellement dans laquelle la protéine dCAS13 est dCAS13a, dCAS13b, dCAS13c ou dCAS13d ; ou
(2) un composant protéique capable de se lier spécifiquement au site de liaison sur l'ADNdb cible, dans laquelle le composant protéique comprend une nucléase à doigt de zinc (ZFN) déficiente en endonucléase, une nucléase effectrice de type activateur de transcription (TALEN) déficiente en endonucléase, une protéine Argonaute, une méganucléase déficiente en endonucléase, une recombinase, ou une combinaison correspondante, éventuellement dans laquelle
(i) le transposome est associé à l'unité de liaison d'ADN programmable par l'intermédiaire d'un lieur reliant la transposase et le composant protéique, en outre éventuellement dans laquelle le lieur comprend un lieur peptidique, un lieur chimique, ou les deux, en outre éventuellement dans laquelle le lieur peptidique :
(a) comprend plusieurs glycine, sérine, thréonine, alanine, lysine, glutamine, ou une combinaison correspondante, éventuellement en outre, dans laquelle le lieur peptidique comprend un lieur GS, et/ou
(b) est un lieur XTEN ; et/ou
(ii) le composant protéique est présent dans une protéine de fusion comprenant la transposase.

8. Mélange réactionnel, comprenant
la composition selon l'une quelconque des revendications 1 à 7 ; et
des acides nucléiques échantillons suspectés de comprendre un ou plusieurs ADNdb cibles.

9. Mélange réactionnel selon la revendication 8,
(i) dans lequel le mélange réactionnel comprend en outre une ADN polymérase, des dNTP, ou une combinaison correspondante,
(ii) dans lequel l'adaptateur est fixé de façon covalente à l'ADNdb cible ou un fragment correspondant,
(iii) dans lequel le mélange réactionnel comprend une pluralité de fragments d'ADNdb comprenant chacun le premier adaptateur et le deuxième adaptateur d'un de la pluralité de complexes protéiques à chaque extrémité respectivement,
(iv) dans lequel les acides nucléiques échantillons comprennent de l'ADN eucaryote, de l'ADN bactérien, de l'ADN viral, de l'ADN fongique, de l'ADN protozoaire, ou une combinaison correspondante,
(v) dans lequel l'ADNdb cible est un ADN génomique, un ADN mitochondrial, un ADN plasmidique, ou une combinaison correspondante, et/ou
(vi) dans lequel les acides nucléiques échantillons proviennent d'un échantillon biologique, un échantillon clinique, un échantillon environnemental ou une combinaison correspondante, éventuellement dans lequel l'échantillon biologique comprend des selles, des expectorations, du sang périphérique, du plasma, du sérum, des ganglions lymphatiques, des tissus respiratoires, des exsudats, un fluide corporel ou une combinaison correspondante.

10. Procédé d'étiquetage d'acides nucléiques, comprenant :
la mise en contact de la composition selon l'une quelconque des revendications 1 à 7 avec un échantillon suspecté de comprendre une pluralité d'ADN doubles brins (ADNdb cibles) pour former un mélange réactionnel ; et
l'incubation du mélange réactionnel pour générer une pluralité de fragments d'ADNdb comprenant chacun le premier adaptateur et le second adaptateur d'un complexe protéique parmi la pluralité de complexes protéiques à chaque extrémité respectivement.

11. Procédé de génération d'une bibliothèque de séquençage, comprenant :
la mise en contact de la composition selon l'une quelconque des revendications 1 à 7 avec un échantillon suspecté de comprendre une pluralité d'ADN doubles brins (ADNdb) cibles pour former un mélange réactionnel ;
l'incubation du mélange réactionnel pour générer une pluralité de fragments d'ADNdb comprenant chacun le premier adaptateur et le second adaptateur d'un parmi la pluralité de complexes protéiques à chaque extrémité respectivement ; et
l'amplification de la pluralité de fragments d'ADNdb avec des amorces capables de se lier aux adaptateurs aux extrémités des fragments d'ADNdb pour générer une bibliothèque de séquençage, éventuellement dans lequel chacune des amorces a une longueur d'environ 5 à 80 nucléotides.

12. Procédé selon la revendication 11, dans lequel l'amplification de la pluralité de fragments d'ADNdb avec l'amorce est effectuée en utilisant une réaction de polymérase en chaîne (PCR), éventuellement dans lequel la PCR est :
(i) amplification isotherme à médiation par boucle (LAMP), amplification dépendante de l'hélicase (HDA), amplification par recombinase polymérase (RPA), amplification par déplacement de brin (SDA), amplification basée sur une séquence d'acide nucléique (NASBA), amplification médiée par transcription (TMA), réaction d'amplification par enzyme de coupure (NEAR), amplification par cercle roulant (RCA), amplification par déplacement multiple (MDA), amplification par ramification (RAM), amplification dépendante de l'hélicase circulaire (cHDA), amplification isotherme à amorce unique (SPIA), technologie d'amplification de l'ARN médiée par signal (SMART), réplication de séquence auto-entretenue (3SR), réaction d'amplification exponentielle du génome (GEAR), ou amplification isotherme par déplacement multiple (IMDA), et/ou
(ii) PCR en temps réel ou PCR quantitative en temps réel (QRT-PCR).

13. Procédé selon l'une quelconque des revendications 10 à 12,
(i) dans lequel l'échantillon
(a) comprend de l'ADN eucaryote, de l'ADN bactérien, de l'ADN viral, de l'ADN fongique, de l'ADN protozoaire, ou une combinaison correspondante, et/ou
(b) est, ou est dérivé de, un échantillon biologique, un échantillon clinique, un échantillon environnemental ou une combinaison correspondante ;
(ii) dans lequel le procédé comprend en outre la génération de la pluralité d'ADNdb cibles à partir d'une pluralité d'ARN cibles avec une transcriptase inverse ; et/ou
(iii) dans lequel la pluralité d'ADNdb cibles comprend :
(a) de l'ADN génomique, de l'ADN mitochondrial, de l'ADN plasmidique, ou une combinaison correspondante,
(b) de l'ADN provenant d'au moins 2 organismes différents,
(c) de l'ADN provenant d'au moins 2 gènes différents, et/ou
(d) un ADNdb cible généré à partir d'un ARN cible avec une transcriptase inverse.

14. Procédé selon l'une quelconque des revendications 10 à 13, dans lequel la pluralité d'ADNdb cibles comprend une signature génétique d'intérêt, éventuellement dans lequel la signature génétique d'intérêt
(i) comprend une ou plusieurs mutations souhaitées, éventuellement en outre dans lequel les une ou plusieurs mutations souhaitées comprennent :
(a) une inversion, délétion, insertion, translocation, duplication, variation du nombre de copies, mutation ponctuelle, ou une combinaison correspondante, et/ou
(b) une substitution, délétion, insertion de nucléotides, ou une combinaison correspondante ;
(ii) est indicative de l'identification d'agents pathogènes, d'une résistance à un antibiotique ou d'une sensibilité à un antibiotique de l'organisme dont provient l'ADNdb cible ;
(iii) est indicative du statut cancéreux de l'organisme dont l'ADNdb cible est issu ; et/ou
(iv) est indicative du statut d'une maladie génétique de l'organisme dont l'ADNdb cible est issu, en outre éventuellement dans lequel la maladie génétique est :
(a) un trouble monogénique, et/ou
(b) une fibrose kystique, la maladie de Huntington, l'anémie falciforme, l'hémophilie, la dystrophie musculaire de Duchenne, la thalassémie, le syndrome de l'X fragile, l'hypercholestérolémie familiale, la polykystose rénale, la neurofibromatose de type I, la sphérocytose héréditaire, le syndrome de Marfan, la maladie de Tay-Sachs, la phénylcétonurie, les mucopolysaccharidoses, le déficit en lipase acide lysosomale, les glycogénoses, la galactosémie ou l'hémochromatose.

15. Procédé selon l'une quelconque des revendications 10 à 14,
(i) dans lequel la mise en contact de la pluralité d'ADNdb cibles avec la pluralité de paires de complexes protéiques est réalisée à une température d'environ 25 °C à environ 80 °C ;
(ii) dans lequel l'incubation du mélange réactionnel comprend l'incubation du mélange réactionnel à une température d'environ 37 °C à environ 55 °C ;
(iii) dans lequel la pluralité de paires de complexes protéiques et la pluralité d'ADNdb cibles sont présents dans le mélange réactionnel à un rapport moléculaire d'environ 2:1 à environ 2 000:1, éventuellement à un rapport moléculaire d'environ 2:1 à environ 200:1 ; et/ou
(iv) dans lequel le procédé comprend en outre le marquage d'une ou des deux extrémités d'un ou plusieurs de la pluralité de fragments d'ADNdb, éventuellement dans lequel le procédé comprend le marquage des deux extrémités d'un ou plusieurs de la pluralité de fragments d'ADNdb différemment, en outre éventuellement dans lequel
(a) le marquage comprend un marquage avec des marqueurs anioniques, des marqueurs cationiques, des marqueurs neutres, des marqueurs électrochimiques, des marqueurs protéiques, des marqueurs fluorescents, des marqueurs magnétiques ou une combinaison correspondante, et/ou
(b) le procédé comprend en outre l'enrichissement des fragments d'ADNdb marqués, la capture des fragments d'ADNdb marqués, l'isolement des fragments d'ADNdb marqués et/ou la visualisation des fragments d'ADNdb marqués.
